# EUROPÄISCHE PATENTSCHRIFT

(11) **EP 3 877 373 B1**
(45) Veröffentlichungstag und Bekanntmachung des Hinweises auf die Patenterteilung: **11.01.2023**
(21) Anmeldenummer: 19800961.5
(22) Anmeldetag: 04.11.2019
(51) Int. Cl.: C07D 279/02, C07D 417/04, C07D 291/08, C07D 285/36, C07D 409/14, C07D 403/10, C07D 251/22, C07D 333/76, C07D 239/28, C07D 239/42, C07D 403/12, C07D 209/10, C07F 9/6584, H01L 51/00, H01L 51/50

(54) **5,6-DIPHENYL-5,6-DIHYDRO-DIBENZ[C,E][1,2]AZAPHOSPHORIN- UND 6-PHENYL-6H-DIBENZO[C,E][1,2]THIAZIN-5,5-DIOXID-DERIVATE UND ÄHNLICHE VERBINDUNGEN ALS ORGANISCHE ELEKTROLUMINESZENZMATERIALIEN FÜR OLEDS**
5,6-DIPHENYL-5,6-DIHYDRO-DIBENZ[C,E][1,2]AZAPHOSPHORINE AND 6-PHENYL-6H-DIBENZO[C,E][1,2]THIAZINE-5,5-DIOXIDE DERIVATIVES AND RELATED COMPOUNDS AS ORGANIC ELECTROLUMINESCENT MATERIALS FOR OLEDS
DÉRIVÉS DE 5,6-DIPHENYL-5,6-DIHYDRO-DIBENZ[C,E][1,2]AZAPHOSPHORINE ET DE 6-PHÉNYL-6H-DIBENZO[C,E][1,2]THIAZINE-5,5-DIOXIDE ET COMPOSÉS SIMILAIRES EN TANT QUE MATÉRIAUX ÉLECTROLUMINESCENTS POUR OLEDS

(30) Priorität: 06.11.2018 EP 18204542
(43) Veröffentlichungstag der Anmeldung: 15.09.2021
(73) Patentinhaber: Merck Patent GmbH, 64293 Darmstadt (DE)
(72) Erfinder: PARHAM, Amir, 60486 Frankfurt am Main (DE); ENGELHART, Jens, 64285 Darmstadt (DE); EICKHOFF, Christian, 68259 Mannheim (DE); EHRENREICH, Christian, 64285 Darmstadt (DE); KROEBER, Jonas, 60311 Frankfurt am Main (DE)
(86) Internationale Anmeldenummer: PCT/EP2019/080039
(87) Internationale Veröffentlichungsnummer: WO 2020/094542

(56) Entgegenhaltungen:
- WO-A1-2011/137951
- WO-A1-2013/012681
- RAJENDER NALLAGONDA ET AL: "Cobalt-Catalyzed Diastereoselective [4+2] Annulation of Phosphinamides with Heterobicyclic Alkenes at Room Temperature", ADVANCED SYNTHESIS & CATALYSIS, Bd. 360, Nr. 2, 19. Oktober 2017 (2017-10-19), - 17. Januar 2018 (2018-01-17), Seiten 255-260, XP55650727, DE ISSN: 1615-4150, DOI: 10.1002/adsc.201701162
- YUAN-HUI CHEN ET AL: "Efficient synthesis of cyclic P-stereogenic phosphinamides from acyclic chiral precursors via radical oxidative intramolecular aryl C-H phosphinamidation", CHEMICAL COMMUNICATIONS, Bd. 53, Nr. 43, Januar 2017 (2017-01), Seiten 5826-5829, XP55650731, UK ISSN: 1359-7345, DOI: 10.1039/C7CC02263H
- JING GUAN ET AL: "Pd II -Catalyzed Mild C?H ortho Arylation and Intramolecular Amination Oriented by a Phosphinamide Group", CHEMISTRY - A EUROPEAN JOURNAL, Bd. 20, Nr. 12, 17. März 2014 (2014-03-17) , Seiten 3301-3305, XP55650738, DE ISSN: 0947-6539, DOI: 10.1002/chem.201303056
- LIU ET AL: "Synthesis of carbazoles and dibenzofurans via cross-coupling of o-iodoanilines and o-iodophenols with silylaryl triflates and subsequent Pd-catalyzed cyclization", TETRAHEDRON, ELSEVIER SCIENCE PUBLISHERS, AMSTERDAM, NL, Bd. 63, Nr. 2, 5. Dezember 2006 (2006-12-05), Seiten 347-355, XP005802258, ISSN: 0040-4020, DOI: 10.1016/J.TET.2006.10.071
- DIETER HELLWINKEL ET AL: "Heterocyclic syntheses via carbanionically induced rearrangement reactions", TETRAHEDRON, Bd. 39, Nr. 12, Januar 1983 (1983-01), Seiten 2073-2084, XP055379507, AMSTERDAM, NL ISSN: 0040-4020, DOI: 10.1016/S0040-4020(01)91925-8
- DEWAR P S ET AL: "PERSULPHATE OXIDATION. PART IX. OXIDATION OF BIPHENYL-2- SULPHONAMIDES AND OMICRON-PHENOXYBENZENESULPHONAMIDES", JOURNAL OF THE CHEMICAL SOCIETY, PERKIN TRANSACTIONS 1, ROYAL SOCIETY OF CHEMISTRY, GB, Nr. 19, Januar 1972 (1972-01), Seiten 2862-2865, XP000602158, ISSN: 0300-922X, DOI: 10.1039/P19720002862

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

In organischen Elektrolumineszenzvorrichtungen (OLEDs) werden als emittierende Materialien häufig phosphoreszierende metallorganische Komplexe eingesetzt. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung.

Aufgabe der vorliegenden Erfindung ist die Bereitstellung von Verbindungen, welche sich für den Einsatz in einer OLED eignen, insbesondere als Matrixmaterial für phosphoreszierende Emitter, aber auch als Elektronentransportmaterialien bzw. Lochblockiermaterialien und je nach Substitutionsmuster auch als Lochtransport- bzw. Lochinjektions- bzw. Elektronenblockiermaterialien. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen und sich gut für die Verwendung in OLEDs eignen. Dabei weisen die OLEDs insbesondere eine lange Lebensdauer, eine hohe Effizienz und eine geringe Betriebsspannung auf. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Aus WO 2011/137951 sind cyclische Phosphinoxid- und Sulfonderivate bekannt, in welchen das Phosphor- bzw. Schwefelatom an ein Stickstoffatom gebunden ist, welches Teil eines cyclischen Systems, wie beispielsweise Carbazol ist. Aus WO 2012/121398 sind Phosphinoxidderivate bekannt, in welchen das Phosphoratom an ein Stickstoffatom gebunden ist, wobei die beiden weiteren Substituenten am Phosphoratom mit den beiden weiteren Substituenten am Stickstoffatom jeweils einen Ring bilden. Verbindungen gemäß der vorliegenden Erfindung sind nicht offenbart. WO 2013/012681 A1, RAJENDER NALLAGONDA ET AL (ADVANCED SYNTHESIS & CATALYSIS, Bd. 360, Nr. 2, 19. Oktober 2017 (2017-10-19), - 17. Januar 2018 (2018-01-17), Seiten 255-260), YUAN-HUI CHEN ET AL (CHEMICAL COMMUNICATIONS, Bd. 53, Nr. 43, Januar 2017 (2017-01), Seiten 5826-5829), JING GUAN ET AL (CHEMISTRY - A EUROPEAN JOURNAL, Bd. 20, Nr. 12, 17. März 2014 (2014-03-17), Seiten 3301-3305), LIU ET AL (TETRAHEDRON, ELSEVIER SCIENCE PUBLISHERS, AMSTERDAM, NL, Bd. 63, Nr. 2, 5. Dezember 2006 (2006-12-05), Seiten 347-355), DIETER HELLWINKEL ET AL (TETRAHEDRON, Bd. 39, Nr. 12, Januar 1983 (1983-01), Seiten 2073-2084), und DEWAR P S ET AL (JOURNAL OF THE CHEMICAL SOCIETY, PERKIN TRANSACTIONS 1, ROYAL SOCIETY OF CHEMISTRY, GB, Nr. 19, Januar 1972 (1972-01), Seiten 2862-2865) offenbaren Verbinungen, die durch Maßgaben aus dem Anspruchsumfang ausgeschlossen wurden. Diese Dokumente betreffen andere technische Gebiete.

Gegenstand der vorliegenden Erfindung ist eine Verbindung gemäß Formel (1), 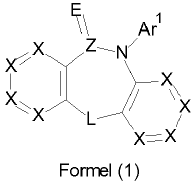 wobei für die verwendeten Symbole gilt:
- Z: ist bei jedem Auftreten gleich oder verschieden PAr² oder S(=O);
- E: ist bei jedem Auftreten gleich oder verschieden O oder S, wenn das Symbol Z, an das dieses E bindet, für PAr² steht, und O, wenn das Symbol Z, an das dieses E bindet, für S(=O) steht;
- L: ist ausgewählt aus der Gruppe bestehend aus einer Einfachbindung, NAr², O, S, S(=O)₂, P(=O)Ar² , -X=X- oder -C(=O)-NAr²-;
- X: steht bei jedem Auftreten gleich oder verschieden für CR oder N, wobei maximal zwei Gruppen X pro Cyclus für N stehen, oder zwei benachbarte X stehen für eine Gruppe der folgenden Formel (2), (3) oder (4), 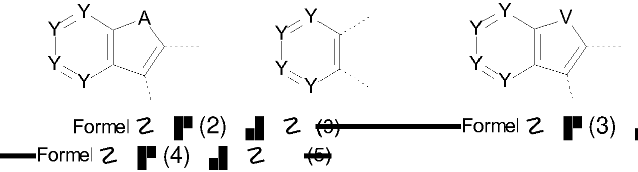 wobei die gestrichelten Bindungen die Verknüpfung dieser Gruppe in der Formel (1) kennzeichnen;
- Y: ist gleich oder verschieden bei jedem Auftreten CR oder N, wobei maximal zwei Gruppen Y pro Cyclus für N stehen;
- Ar¹, Ar²: ist bei jedem Auftreten gleich oder verschieden ein aromatisches oder heteroaromatisches Ringsystem mit 5 bis 40 aromatischen Ringatomen, welches mit einem oder mehreren Resten R substituiert sein kann, wobei Ar¹ und Ar² nicht miteinander verknüpft sind, mit der Maßgabe, dass die Substituenten R, die an Ar¹ binden, nicht für Fluor stehen;
- A: ist bei jedem Auftreten gleich oder verschieden NAr², O, S oder CR2;
- V: ist bei jedem Auftreten gleich oder verschieden -NAr²-Z(=E)-;
- R: ist bei jedem Auftreten gleich oder verschieden H, D, F, Cl, Br, I, N(Ar³)₂, OAr³, SAr³, CN, NO₂, NAr³R¹, N(R¹)₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 40 aromatischen Ringatomen, das jeweils durch einen oder mehrere Reste R¹ substituiert sein kann; dabei können zwei Reste R auch miteinander ein aliphatisches oder heteroaliphatisches Ringsystem bilden;
- Ar³: ist bei jedem Auftreten gleich oder verschieden ein aromatisches oder heteroaromatisches Ringsystem mit 5 bis 30 aromatischen Ringatomen, das durch einen oder mehrere Reste R¹ substituiert sein kann;
- R¹: ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch Si(R²)₂, C=0, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein aliphatisches Ringsystem bilden;
- R²: ist bei jedem Auftreten gleich oder verschieden H, D, F, CN oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
dabei sind die folgenden Verbindungen von der Erfindung ausgeschlossen:

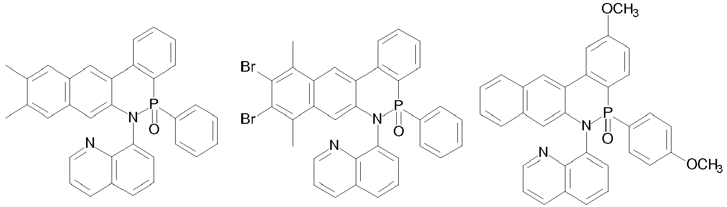

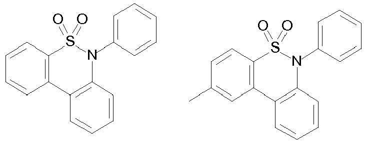

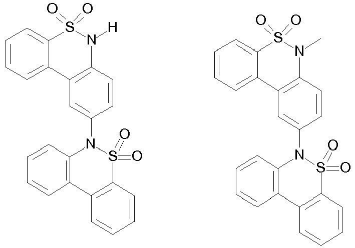

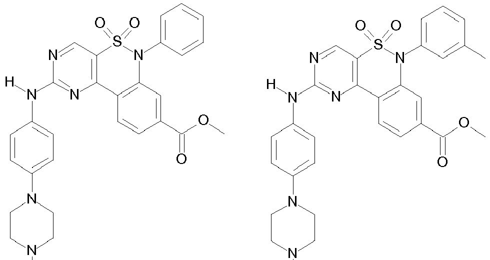

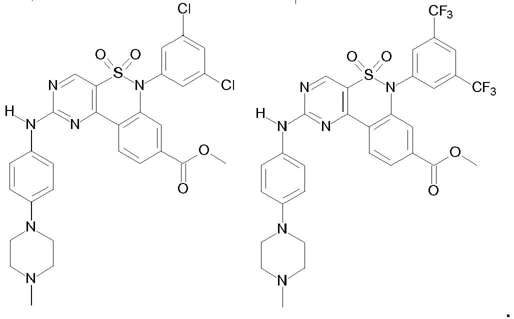

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. Ebenso sollen hierunter Systeme verstanden werden, in denen zwei oder mehr Aryl- bzw. Heteroarylgruppen direkt miteinander verknüpft sind, wie z. B. Biphenyl, Terphenyl, Bipyridin oder Phenylpyridin. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugte aromatische bzw. heteroaromatische Ringsysteme sind einfache Aryl- bzw. Heteroarylgruppen sowie Gruppen, in denen zwei oder mehr Aryl- bzw. Heteroarylgruppen direkt miteinander verknüpft sind, beispielsweise Biphenyl oder Bipyridin, sowie Fluoren oder Spirobifluoren.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe OR¹ mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe SR¹ mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH2-Gruppen durch die oben genannten Gruppen ersetzt sein können; weiterhin können auch ein oder mehrere H-Atome durch D, F, Cl, Br, I, CN oder NO₂, bevorzugt F, Cl oder CN, besonders bevorzugt F oder CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter der Formulierung, dass zwei oder mehr Reste miteinander einen aliphatischen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht:

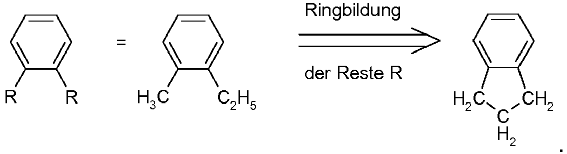

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

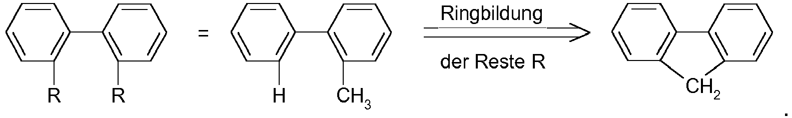

In einer Ausführungsform der Erfindung enthält die Verbindung der Formel (1) keine Gruppe der Formel (2), (3) oder (4), so dass es sich um eine Verbindung der folgenden Formel (1-1) handelt, 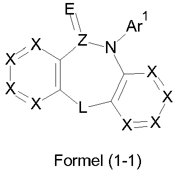 wobei X bei jedem Auftreten gleich oder verschieden für CR oder N steht, wobei maximal zwei Gruppen X pro Cyclus für N stehen, und die weiteren Symbole die oben genannten Bedeutungen aufweisen.

Wenn die Verbindung eine Gruppe der Formel (2), (3) oder (4) enthält, ergeben sich je nach Position der Bindung unterschiedliche Isomere. Diese sind für Verbindungen der Formel (1) und die Gruppe der Formel (2) nachfolgend exemplarisch durch die Formeln (5) bis (16) dargestellt, 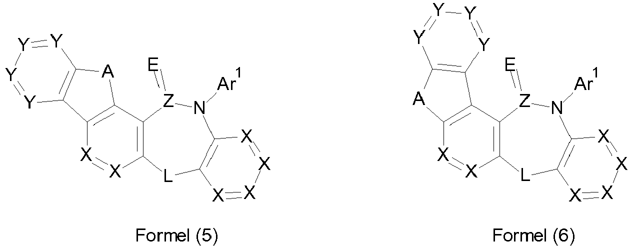 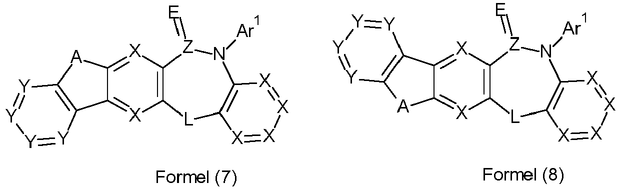 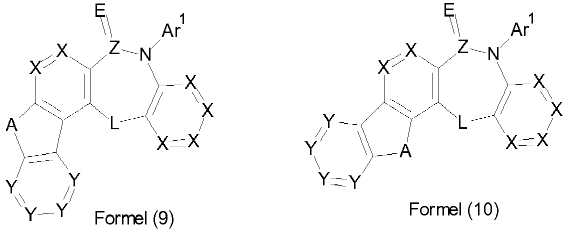 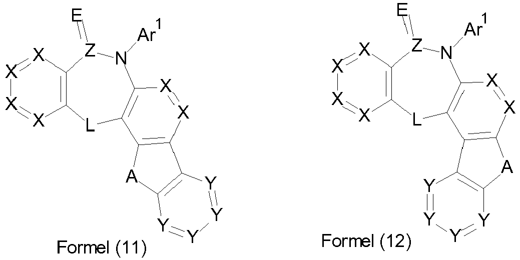 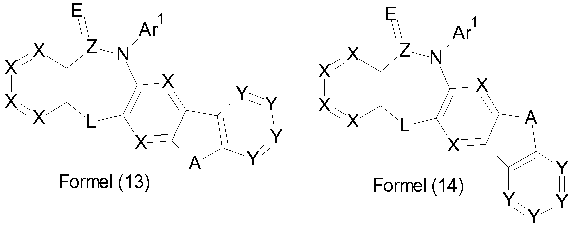 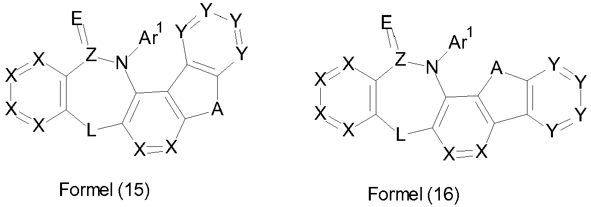 wobei X die oben genannten Bedeutungen aufweist und bevorzugt bei jedem Auftreten gleich oder verschieden für CR oder N steht, wobei maximal zwei Gruppen X pro Cyclus für N stehen, und die weiteren Symbole die oben genannten Bedeutungen aufweisen.

Entsprechende Strukturen ergeben sich analog für die ankondensierten Gruppen der Formeln (3) bzw. (4).

In einer bevorzugten Ausführungsform der Erfindung steht in der Gruppe der Formel (2), (3) oder (4) maximal ein Symbol Y für N, und die anderen Symole Y stehen gleich oder verschieden für CR. Besonders bevorzugt stehen alle Symbole Y in den Formeln (2), (3) und (4) für CR, so dass es sich bevorzugt um eine Gruppe der Formel (2a), (3a) bzw. (4a) handelt, 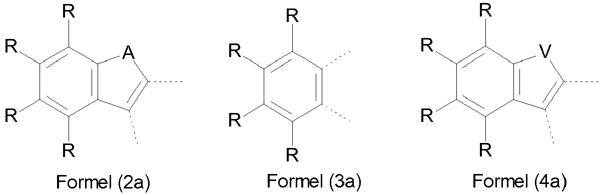 wobei die verwendeten Symbole die oben genannten Bedeutungen aufweisen.

In einer bevorzugten Ausführungsform der Formel (2) bzw. (2a) bzw. der Formeln (5) bis (16) und den nachfolgenden Formeln steht A bei jedem Auftreten gleich oder verschieden für NAr².

In einer bevorzugten Ausführungsform der Erfindung enthält die Verbindung der Formel (1) insgesamt keine, eine oder zwei Gruppen der Formel (2), (3) oder (4), wobei bevorzugt maximal eine Gruppe der Formel (2), (3) oder (4) pro Cyclus gebunden ist. Besonders bevorzugt enthält die Verbindung der Formel (1) keine oder eine Gruppe der Formel (2), (3) oder (4). Die restlichen Symbole X, die nicht für eine Gruppe der Formel (2), (3) oder (4) stehen, stehen gleich oder verschieden bei jedem Auftreten für CR oder N. Ganz besonders bevorzugt enthält die Verbindung der Formel (1) keine Gruppe der Formel (2), (3) oder (4), und die Symbole X stehen gleich oder verschieden für CR oder N.

In einer weiteren bevorzugten Ausführungsform der Erfindung steht in Formel (1) maximal ein Symbol X pro Cyclus für N und besonders bevorzugt insgesamt maximal ein Symbol X für N. Ganz besonders bevorzugt stehen die Symbole X, die nicht für eine Gruppe der Formel (2), (3) oder (4) stehen, gleich oder verschieden bei jedem Auftreten für CR. In einer besonders bevorzugten Ausführungsform der Erfindung stehen die Symbole Y gleich oder verschieden bei jedem Auftreten für CR, so dass es sich bevorzugt um eine Gruppe der Formel (2a), (3a) oder (4a) handelt, und die Symbole X, die nicht für eine Gruppe der Formel (2a), (3a) oder (4a) stehen, stehen gleich oder verschieden bei jedem Auftreten für CR.

Bevorzugte Ausführungsformen der Formel (1) sind somit die Strukturen der folgenden Formeln (1a) und (5a) bis (16a), 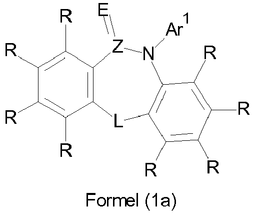 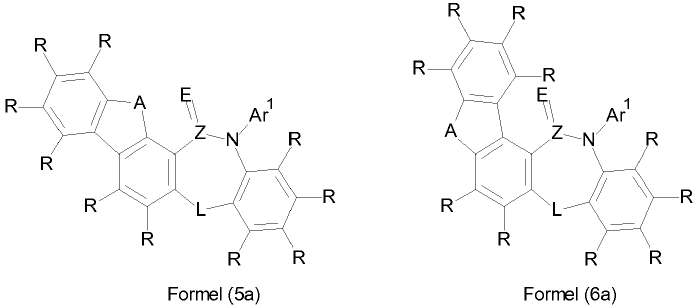 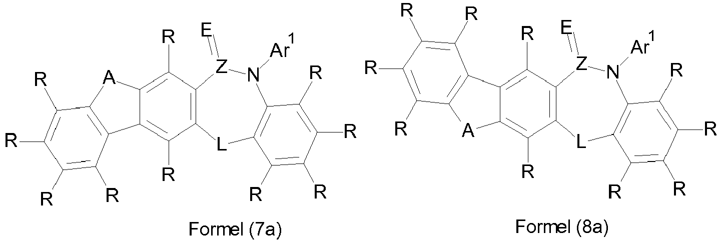 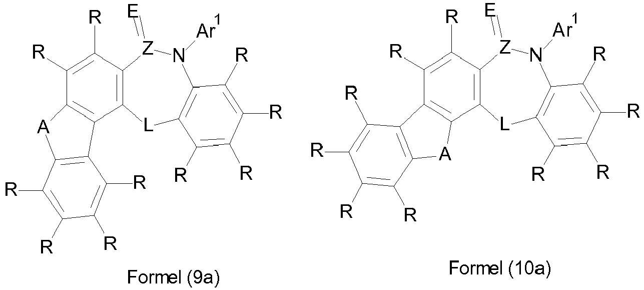 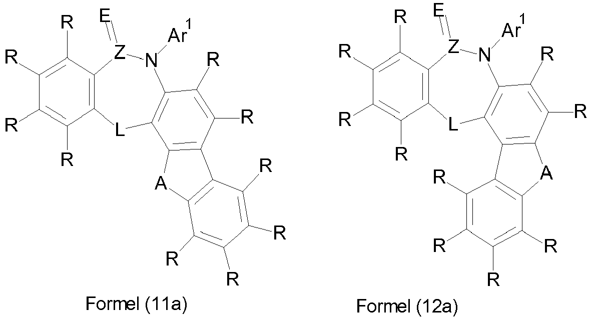 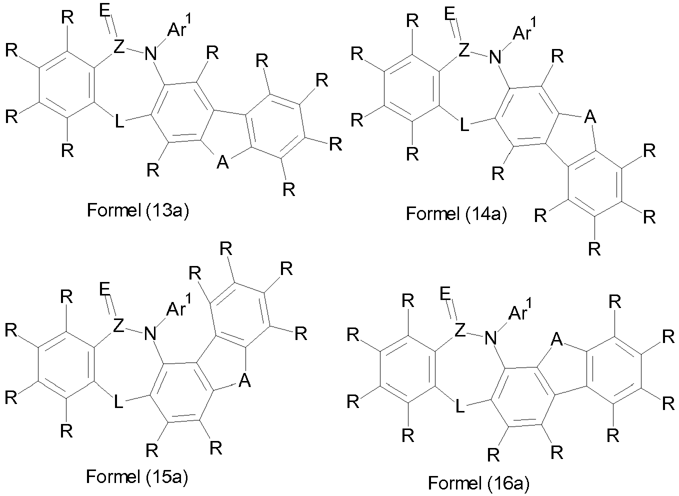 wobei die verwendeten Symbole die oben genannten Bedeutungen aufweisen. Dabei steht A bevorzugt für NAr².

In Formel (1) sowie den oben aufgeführten bevorzugten Ausführungsformen der Erfindung steht E bevorzugt für O.

In einer Ausführungsform der Erfindung steht L für eine Einfachbindung, ein Rest R, der benachbart zu L gebunden ist, steht für P(=O)R¹ oder S(=O)₂R¹ und der andere Rest R, der benachbart zu L gebunden ist, steht für N(Ar³)R¹, wobei diese beiden Reste R miteinander einen Ring bilden. Dadurch ergeben sich die Strukturen der folgenden Formeln (17) und (18) bzw. die bevorzugten Ausführungsformen (17a) und (18a), 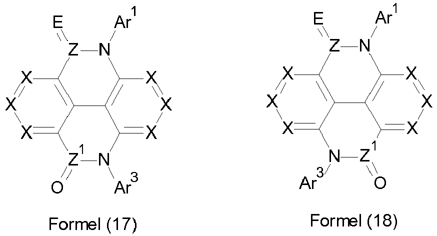 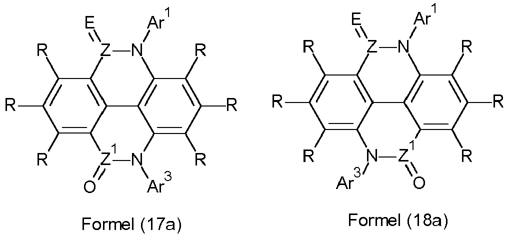 wobei die verwendeten Symbole die oben genannten Bedeutungen aufweisen und Z¹ für PR¹ oder S(=O) steht. Dabei steht E bevorzugt für O, und der Rest R¹, der an Z¹ bindet, steht bevorzugt für ein aromatisches oder heteroaromatisches Ringsystem mit 6 bis 30 aromatischen Ringatomen, bevorzugt mit 6 bis 13 aromatischen Ringatomen, das durch einen oder mehrere Reste R² substituiert sein kann.

In einer bevorzugten Ausführungsform der Erfindung stehen insgesamt maximal drei Reste R, besonders bevorzugt maximal zwei Reste R und ganz besonders bevorzugt maximal ein Rest R in der Verbindung der Formel (1) bzw. den oben aufgeführten bevorzugten Strukturen für eine Gruppe ungleich Wasserstoff.

Besonders bevorzugt sind die Strukturen der folgenden Formeln (1b) bzw. (5b) bis (16b), 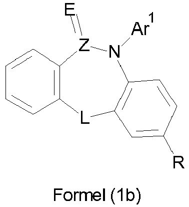 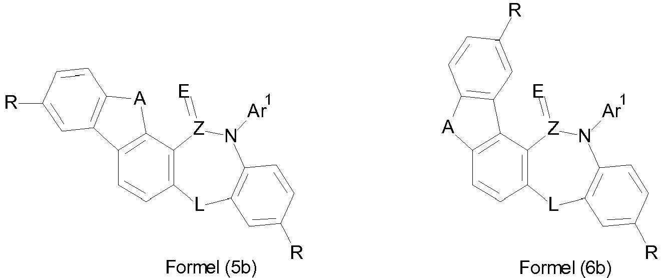 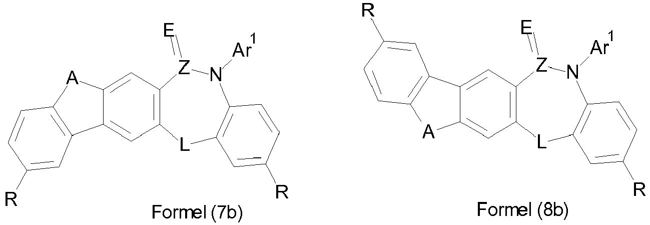 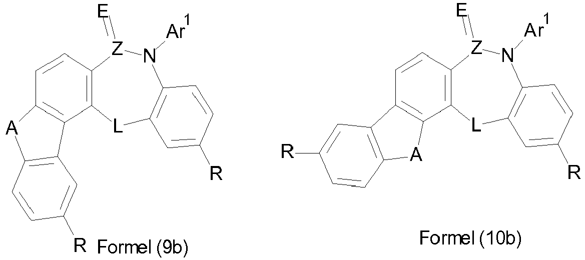 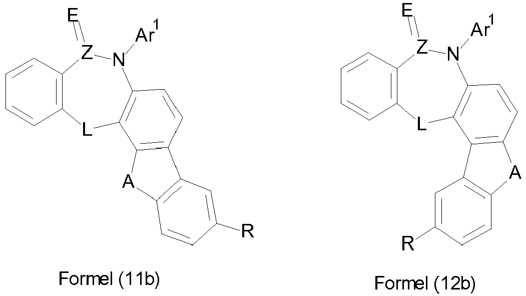 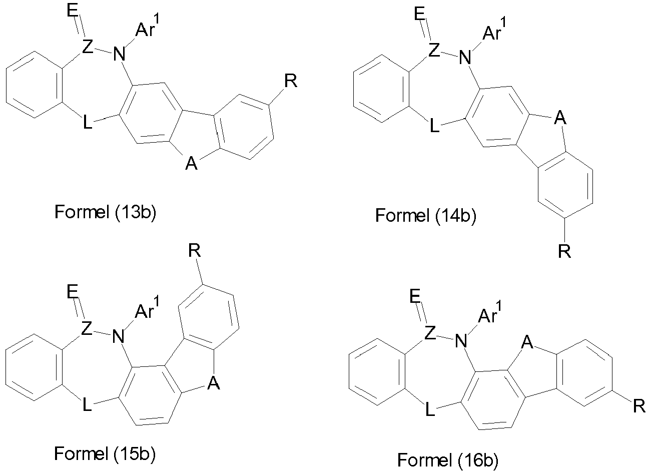 wobei die verwendeten Symbole die oben genannten Bedeutungen aufweisen.

In einer bevorzugten Ausführungsform der Erfindung steht Z gleich oder verschieden bei jedem Auftreten für PAr².

In einer weiteren bevorzugten Ausführungsform der Erfindung steht E für O.

In einer weiteren bevorzugten Ausführungsform der Erfindung steht L für eine Einfachbindung, NAr², O, S, S(=O)₂ oder -CR=CR-, besonders bevorzugt für eine Einfachbindung, NAr² oder O, und ganz besonders bevorzugt für eine Einfachbindung.

In einer besonders bevorzugten Ausführungsform der Erfindung steht Z für PAr², und E steht für O, und L steht für eine Einfachbindung. Besonders bevorzugt sind somit die Verbindungen der folgenden Formeln (1c) und (5c) bis (16c), 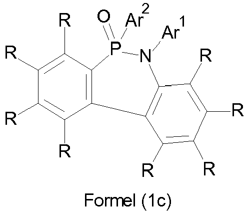 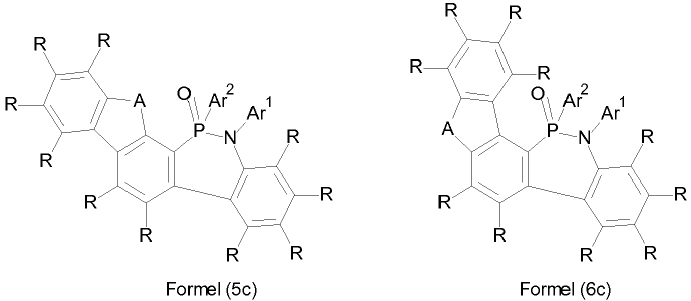 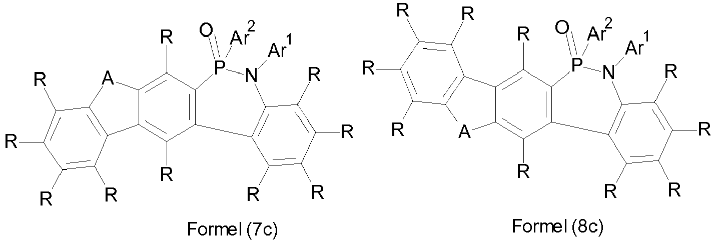 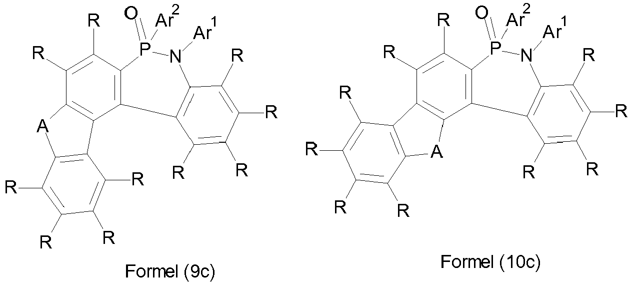 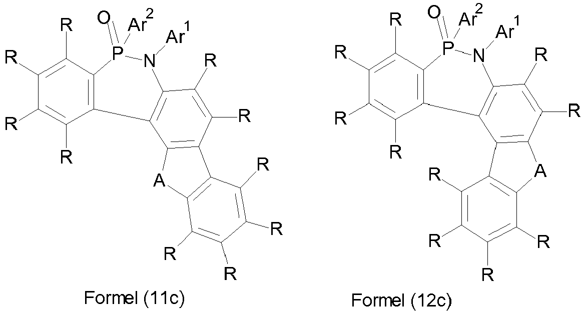 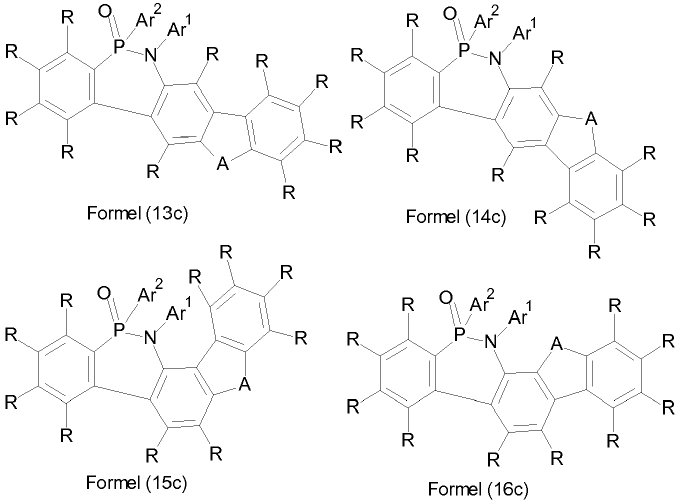 wobei die verwendeten Symbole die oben genannten Bedeutungen aufweisen. Dabei steht A bevorzugt für NAr². Bevorzugt sind die Reste R in den Positionen analog zu den Strukturen der Formeln (1b) und (5b) bis (16b) gebunden.

Im Folgenden werden bevorzugte Substituenten Ar¹, Ar², R, Ar³, R¹ und R² beschrieben. In einer besonders bevorzugten Ausführungsform der Erfindung treten die vorstehend genannten Bevorzugungen für X, Y, Z, E und L und die nachfolgend genannten Bevorzugungen für Ar¹, Ar², R, Ar³, R¹ und R² gleichzeitig auf und gelten für die Strukturen der Formel (1) sowie für alle oben aufgeführten bevorzugten Ausführungsformen.

In einer bevorzugten Ausführungsform der Erfindung sind Ar¹ und Ar² bei jedem Auftreten gleich oder verschieden ein aromatisches oder heteroaromatisches Ringsystem mit 6 bis 30 aromatischen Ringatomen, das durch einen oder mehrere Reste R substituiert sein kann, wobei für Ar¹ die Reste R nicht für Fluor stehen. Besonders bevorzugt sind Ar¹ und Ar² bei jedem Auftreten gleich oder verschieden ein aromatisches oder heteroaromatisches Ringsystem mit 6 bis 24 aromatischen Ringatomen, insbesondere mit 6 bis 12 aromatischen Ringatomen, das durch einen oder mehrere, bevorzugt nicht-aromatische, Reste R substituiert sein kann, wobei für Ar¹ die Reste R nicht für Fluor stehen. Wenn Ar für eine Heteroarylgruppe, insbesondere für Triazin, Pyrimidin, Chinazolin oder Carbazol steht, können auch aromatische oder heteroaromatische Substituenten R an dieser Heteroarylgruppe bevorzugt sein. Weiterhin kann es bevorzugt sein, wenn Ar¹ bzw. Ar² mit einer Gruppe N(Ar³)₂ substituiert ist, so dass der Substituent Ar¹ bzw. Ar² insgesamt eine Triarylamin- bzw. Triheteroarylamin-Gruppe darstellt.

Geeignete aromatische bzw. heteroaromatische Ringsystem Ar¹ bzw. Ar² sind bei jedem Auftreten gleich oder verschieden ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren Resten R, bevorzugt nicht-aromatischen Resten R, substituiert sein können. Wenn Ar für eine Heteroarylgruppe, insbesondere für Triazin, Pyrimidin, Chinazolin oder Carbazol steht, können auch aromatische oder heteroaromatische Reste R an dieser Heteroarylgruppe bevorzugt sein.

Dabei sind Ar¹ und Ar² bevorzugt bei jedem Auftreten gleich oder verschieden gewählt aus den Gruppen der folgenden Formeln Ar-1 bis Ar-76, 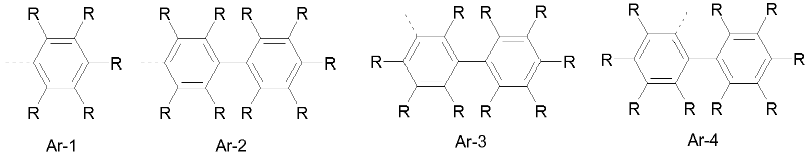 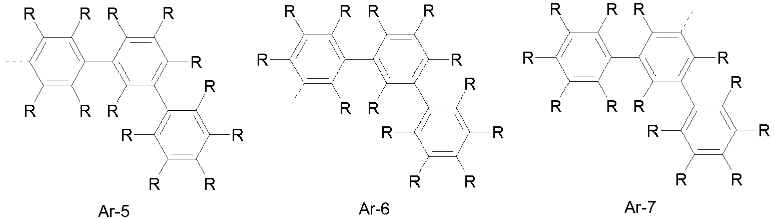 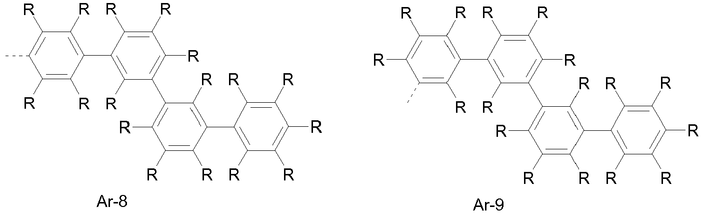 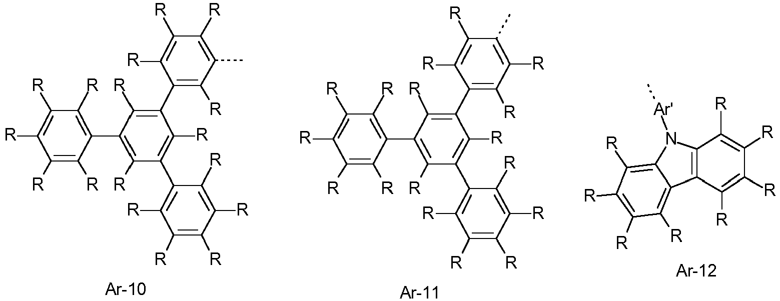 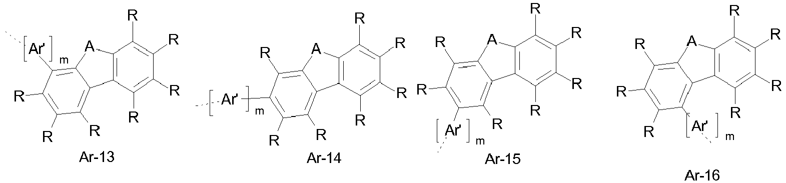 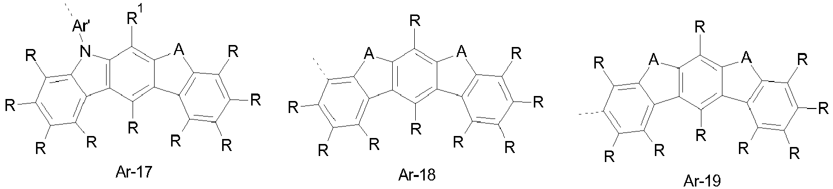 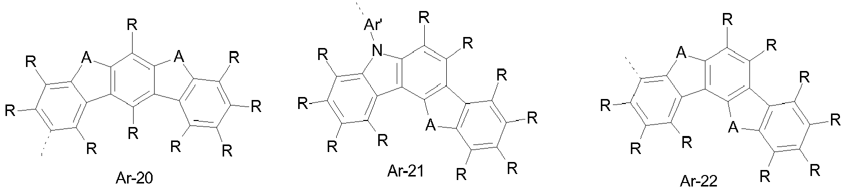 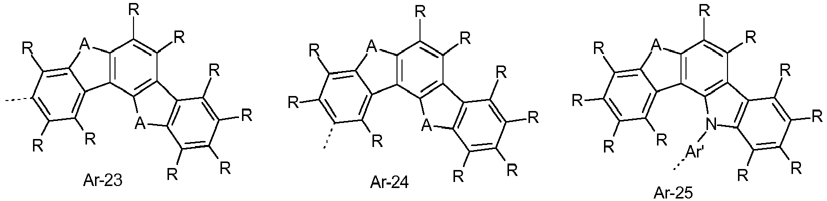 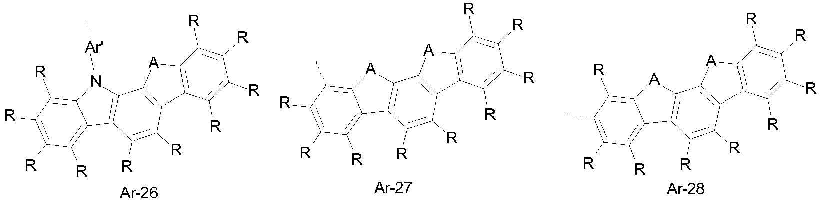 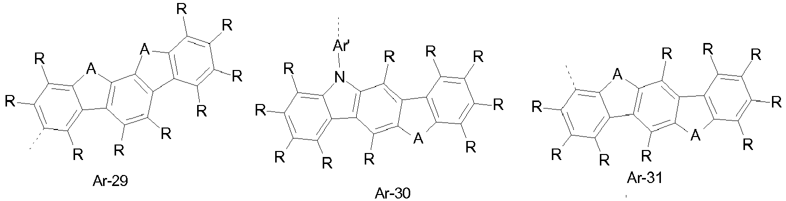 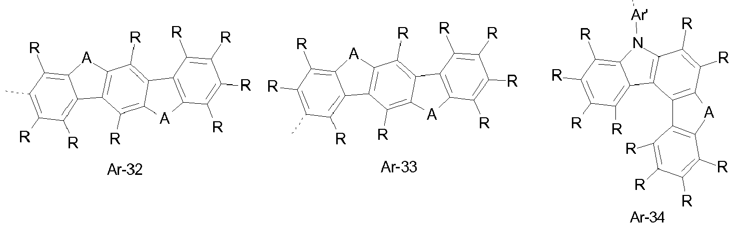 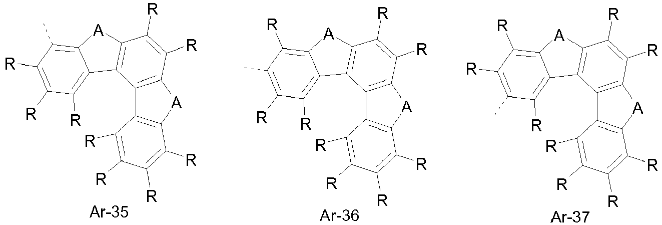 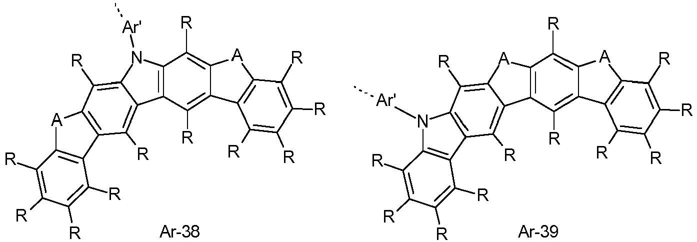 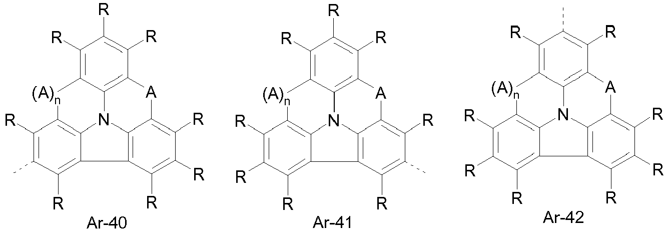 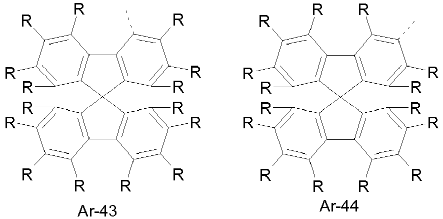 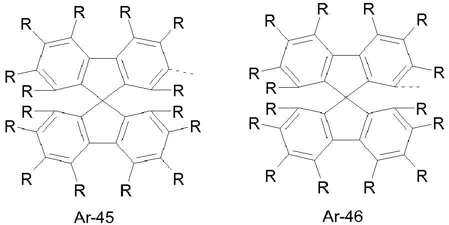 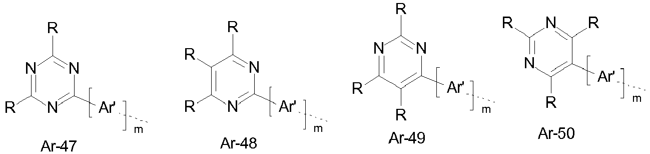 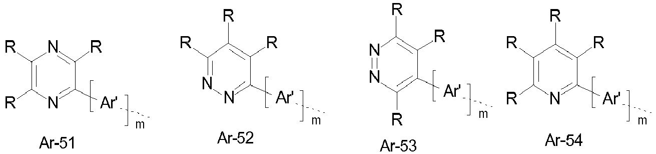 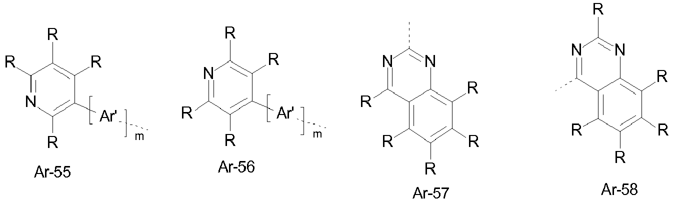 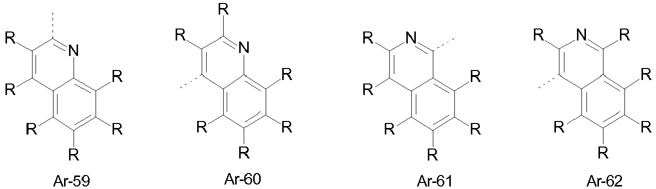 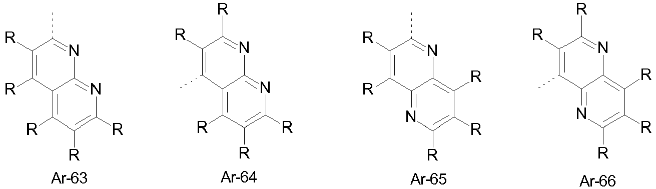 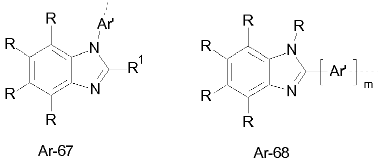 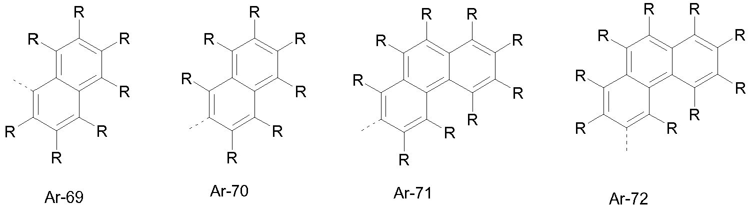 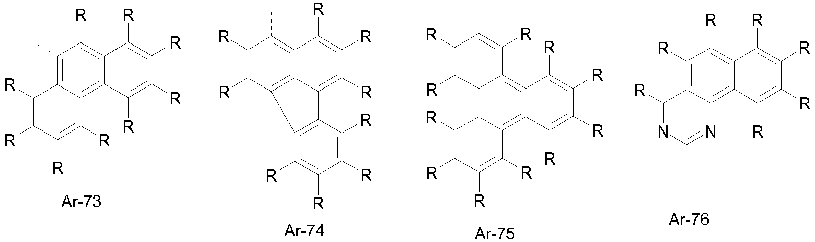 wobei R die oben genannten Bedeutungen aufweist und für Substituenten an Ar¹ nicht für Fluor steht, die gestrichelte Bindung für Ar¹ die Bindung an das Stickstoffatom und für Ar² die Bindung an das Phosphor bzw. Stickstoffatom darstellt und weiterhin gilt:
- Ar': ist bei jedem Auftreten gleich oder verschieden ein bivalentes aromatisches oder heteroaromatisches Ringsystem mit 6 bis 18 aromatischen Ringatomen, bevorzugt mit 6 bis 13 aromatischen Ringatomen, welches jeweils mit einem oder mehreren Resten R substituiert sein kann;
- A: ist bei jedem Auftreten gleich oder verschieden CR2, NR, O oder S;
- n: ist 0 oder 1, wobei n = 0 bedeutet, dass an dieser Position keine Gruppe A gebunden ist und an die entsprechenden Kohlenstoffatome statt dessen Reste R gebunden sind;
- m: ist 0 oder 1, wobei m = 0 bedeutet, dass die Gruppe Ar' nicht vorhanden ist und dass die entsprechende aromatische bzw. heteroaromatische Gruppe direkt an das Stickstoffatom bzw. das Phosphoratom gebunden ist.

In einer bevorzugten Ausführungsform der Erfindung ist R bei jedem Auftreten gleich oder verschieden ausgewählt aus der Gruppe bestehend aus H, D, F, N(Ar³)₂, CN, OR¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R¹ substituiert sein kann, bevorzugt jedoch unsubstituiert ist, und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R¹ substituiert sein kann; dabei können zwei Reste R auch miteinander ein aliphatisches Ringsystem bilden. Besonders bevorzugt ist R bei jedem Auftreten gleich oder verschieden ausgewählt aus der Gruppe bestehend aus H, N(Ar³)₂, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste R¹, bevorzugt nicht-aromatische Reste R¹, substituiert sein kann. Ganz besonders bevorzugt ist R bei jedem Auftreten gleich oder verschieden ausgewählt aus der Gruppe bestehend aus H oder einem aromatischen oder heteroaromatischen Ringsystem mit 6 bis 24 aromatischen Ringatomen, besonders bevorzugt mit 6 bis 13 aromatischen Ringatomen, das jeweils durch einen oder mehrere Reste R¹, bevorzugt nicht-aromatische Reste R¹, substituiert sein kann. Weiterhin kann es bevorzugt sein, wenn R für eine Triaryl- bzw. -heteroarylamingruppe steht, welche durch einen oder mehrere Reste R¹ substituiert sein kann. Diese Gruppe ist eine Ausführungsform eines aromatischen oder heteroaromatischen Ringsystems, wobei dann mehrere Aryl- bzw. Heteroarylgruppen durch ein Stickstoffatom miteinander verknüpft sind. Wenn R für eine Triaryl- bzw. -heteroarylamingruppe steht, weist diese Gruppe bevorzugt 18 bis 30 aromatische Ringatome auf und kann durch einen oder mehrere Reste R¹, bevorzugt nicht-aromatische Reste R¹, substituiert sein.

In einer weiteren bevorzugten Ausführungsform der Erfindung ist Ar³ ein aromatisches oder heteroaromatisches Ringsystem mit 6 bis 24 aromatischen Ringatomen, besonders bevorzugt mit 6 bis 13 aromatischen Ringatomen, das durch einen oder mehrere, bevorzugt nicht-aromatische Reste R¹ substituiert sein kann.

In einer weiteren bevorzugten Ausführungsform der Erfindung ist R¹ gleich oder verschieden bei jedem Auftreten ausgewählt aus der Gruppe bestehend aus H, D, F, CN, OR², einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R² substituiert sein kann und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R² substituiert sein kann; dabei können zwei oder mehrere Reste R¹ miteinander ein aliphatisches Ringsystem bilden. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 24 aromatischen Ringatomen, bevorzugt mit 6 bis 13 aromatischen Ringatomen, das jeweils durch einen oder mehrere Reste R² substituiert sein kann, bevorzugt aber unsubstituiert ist.

In einer weiteren bevorzugten Ausführungsform der Erfindung ist R² gleich oder verschieden bei jedem Auftreten H, F, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Geeignete aromatische bzw. heteroaromatische Ringsysteme R bzw. Ar³ sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren Resten R¹ substituiert sein können. Wenn R bzw. Ar³ für eine Heteroarylgruppe, insbesondere für Triazin, Pyrimidin, Chinazolin oder Carbazol steht, können auch aromatische oder heteroaromatische Reste R¹ an dieser Heteroarylgruppe bevorzugt sein.

Dabei sind die Gruppen R, wenn sie für ein aromatisches bzw. heteroaromatisches Ringsystem stehen, bzw. Ar³ bevorzugt gewählt aus den Gruppen der folgenden Formeln R-1 bis R-76, 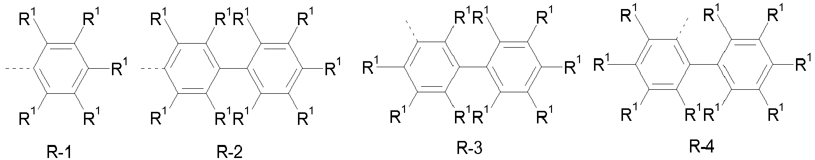 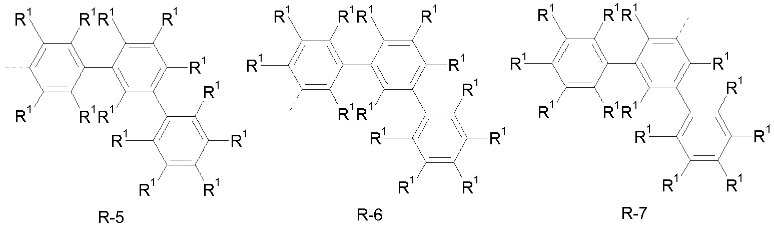 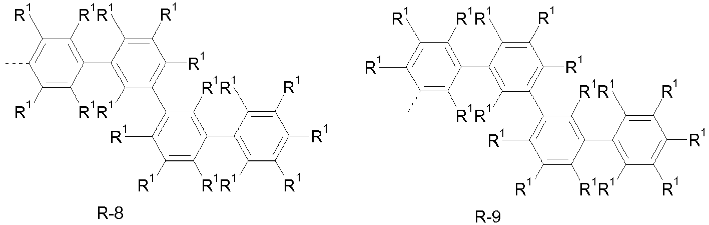 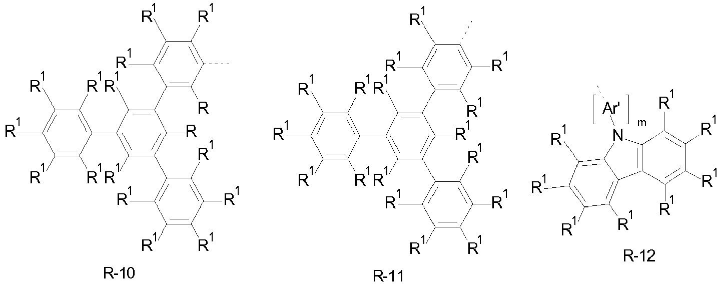 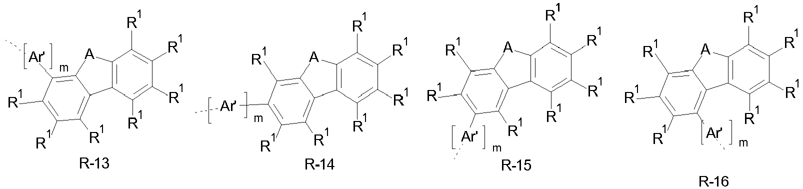 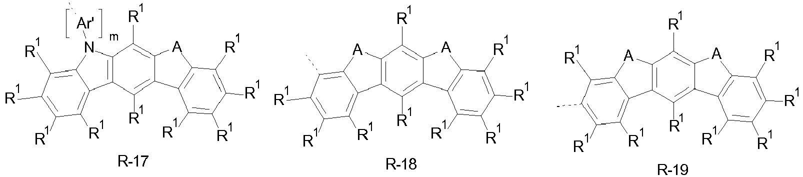 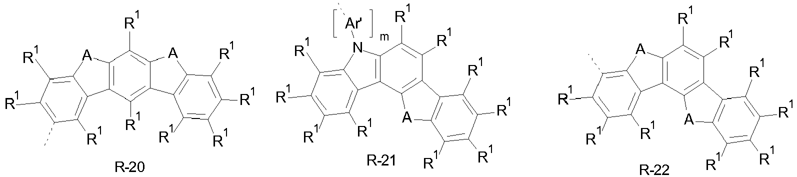 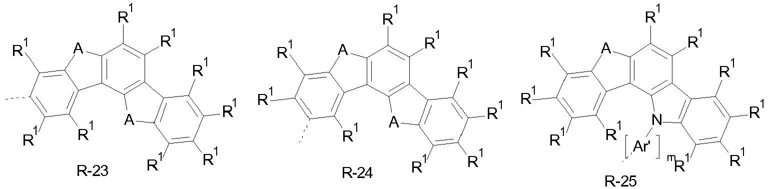 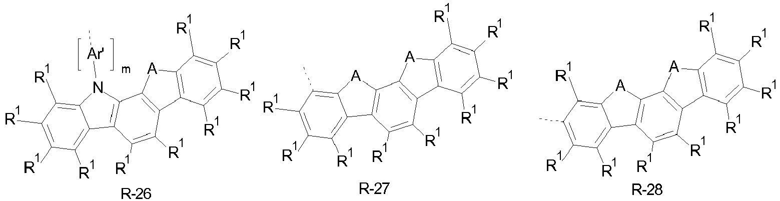 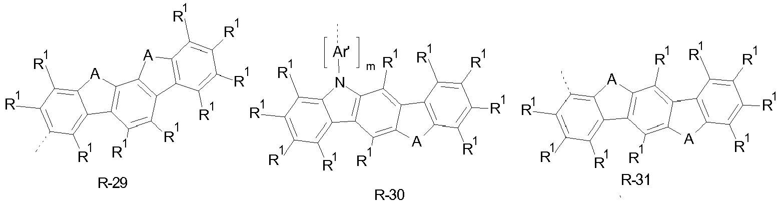 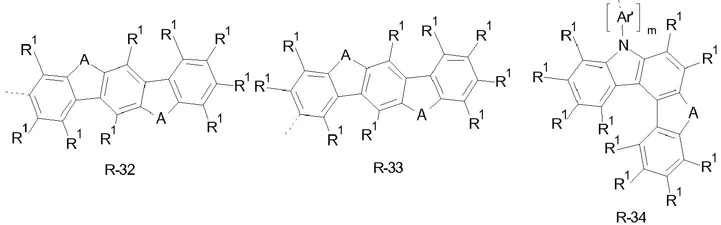 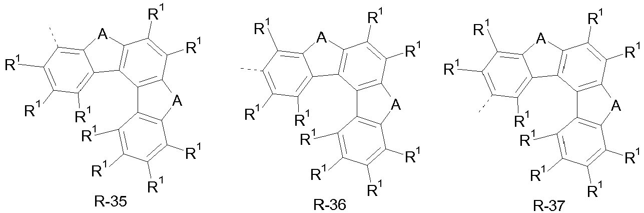 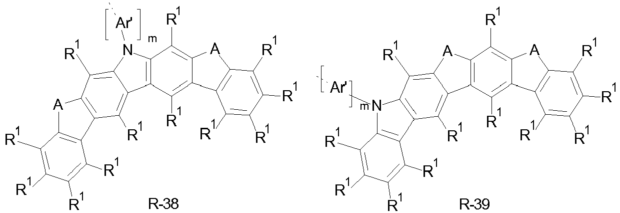 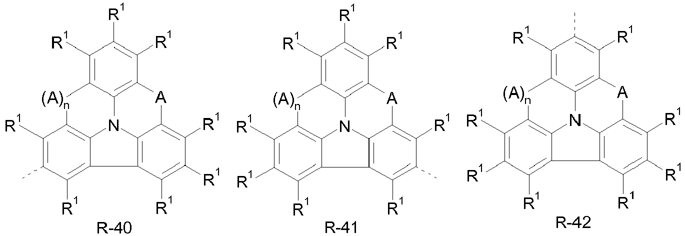 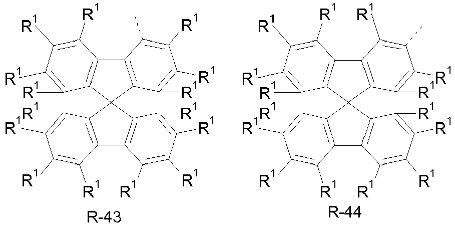 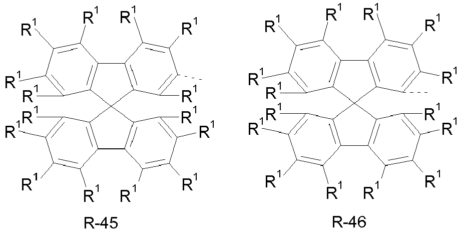 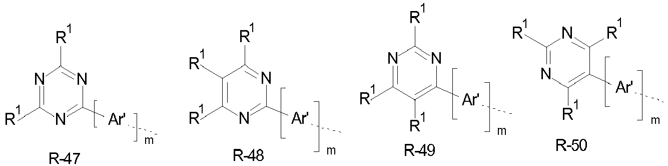 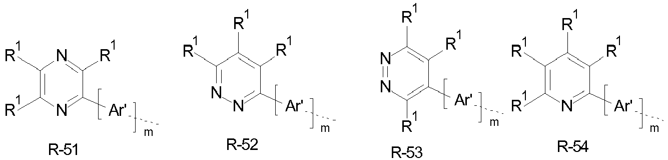 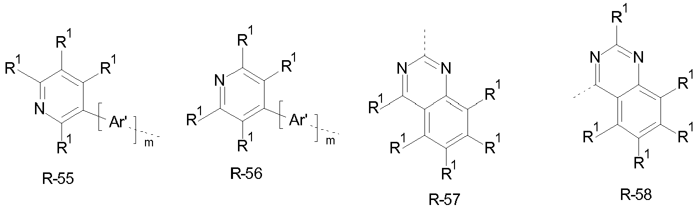 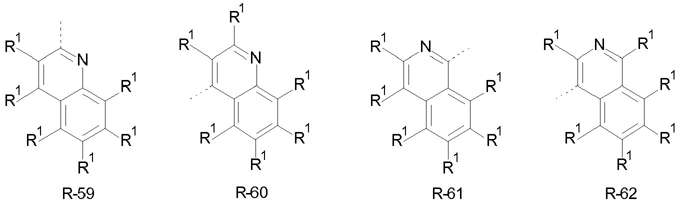 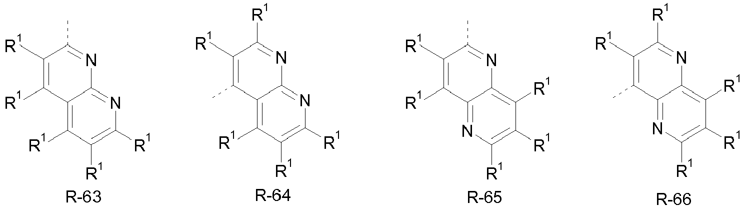 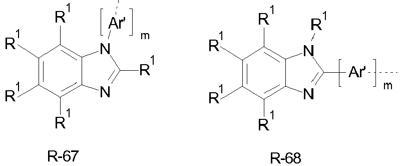 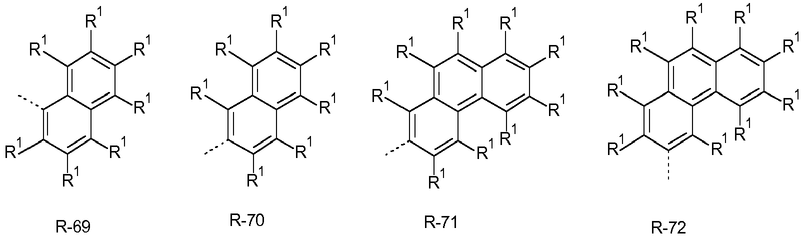 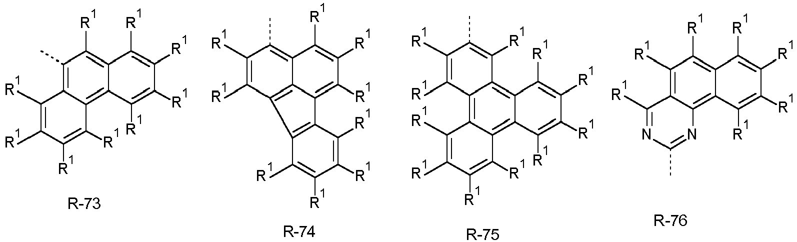 wobei R¹ die oben genannten Bedeutungen aufweist, die gestrichelte Bindung die Verknüpfung dieser Gruppe darstellt und weiterhin gilt:
- Ar': ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- n: ist 0 oder 1, wobei n = 0 bedeutet, dass an dieser Position keine Gruppe A gebunden ist und an den entsprechenden Kohlenstoffatomen statt dessen Reste R¹ gebunden sind;
- m: ist 0 oder 1, wobei m = 0 bedeutet, dass die Gruppe Ar³ nicht vorhanden ist und dass die entsprechende aromatische bzw. heteroaromatische Gruppe direkt an ein Kohlenstoffatom des Grundgerüsts in Formel (1) bzw. in den bevorzugten Ausführungsformen bzw. an Ar¹ bzw. Ar² bzw. an das Stickstoffatom in der Gruppe N(Ar³)₂ gebunden ist; mit der Maßgabe, dass m = 1 ist für die Strukturen (R-12), (R-17), (R-21), (R-25), (R-26), (R-30), (R-34), (R-38) und (R-39), wenn es sich bei diesen Gruppen um Ausführungsformen von Ar³ handelt.

Wenn die oben genannten Gruppen Ar-1 bis Ar-76 für Ar¹ bzw. Ar² bzw. R-1 bis R-76 für R bzw. Ar³ mehrere Gruppen A aufweisen, so kommen hierfür alle Kombinationen aus der Definition von A in Frage. Bevorzugte Ausführungsformen sind dann solche, in denen eine Gruppe A für NR bzw. NR¹ und die andere Gruppe A für C(R)₂ bzw. C(R¹)₂ steht oder in denen beide Gruppen A für NR bzw. NR¹ stehen oder in denen beide Gruppen A für O stehen. In einer besonders bevorzugten Ausführungsform der Erfindung steht in Gruppen Ar¹, Ar², R bzw. Ar³, die mehrere Gruppen A aufweisen, mindestens eine Gruppe A für C(R)₂ bzw. C(R¹)₂ oder für NR bzw. NR¹.

Wenn A in Formel (2) bzw. Formel (2a) bzw. in den Formeln Ar-1 bis Ar-76 bzw. in den Formeln R-1 bis R-76 für NR bzw. NR¹ steht, steht der Substituent R bzw. R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R¹ bzw. R² substituiert sein kann. In einer besonders bevorzugten Ausführungsform steht dieser Substituent R bzw. R¹ gleich oder verschieden bei jedem Auftreten für ein aromatisches oder heteroaromatisches Ringsystem mit 6 bis 24 aromatischen Ringatomen, bevorzugt mit 6 bis 12 aromatischen Ringatomen, welches keine kondensierten Arylgruppen oder Heteroarylgruppen, in denen zwei oder mehr aromatische bzw. heteroaromatische 6-Ring-Gruppen direkt aneinander ankondensiert sind, aufweist, und welches jeweils auch durch einen oder mehrere Reste R¹ bzw. R² substituiert sein kann. Besonders bevorzugt sind Phenyl, Biphenyl, Terphenyl und Quaterphenyl mit Verknüpfungsmustern, wie vorne für Ar-1 bis Ar-11 bzw. R-1 bis R-11 aufgeführt, wobei diese Strukturen durch einen oder mehrere Reste R¹ bzw. R² substituiert sein können, bevorzugt aber unsubstituiert sind.

Wenn A in Formel (2) bzw. Formel (2a) bzw. in den Formeln Ar-1 bis Ar-76 bzw. in den Formeln R-1 bis R-76 für CR₂ bzw. C(R¹)₂ steht, stehen die Substituenten R bzw. R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R¹ bzw. R² substituiert sein kann. Ganz besonders bevorzugt steht R bzw. R¹ für eine Methylgruppe oder für eine Phenylgruppe. Dabei können die Reste R bzw. R¹ auch miteinander ein Ringsystem bilden, was zu einem Spirosystem führt.

Weitere geeignete Gruppen Ar¹, Ar², R bzw. Ar' sind Gruppen der Formel -Ar⁴-N(Ar⁵)(Ar⁶), wobei Ar⁴, Ar⁵ und Ar⁶ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Für Ar¹ bzw. Ar² ergibt sich eine solche Gruppe, indem die Gruppe Ar¹ bzw. Ar² mit einer Gruppe N(Ar³)₂ substituiert ist. Dabei beträgt die Gesamtzahl der aromatischen Ringatome von Ar⁴, Ar⁵ und Ar⁶ maximal 60 und bevorzugt maximal 40.

Dabei können Ar⁴ und Ar⁵ miteinander und/oder Ar⁵ und Ar⁶ miteinander auch durch eine Gruppe, ausgewählt aus C(R¹)₂, NR¹, O oder S, verbunden sein. Bevorzugt erfolgt die Verknüpfung von Ar⁴ und Ar⁵ miteinander bzw. von Ar⁵ und Ar⁶ miteinander jeweils ortho zur Position der Verknüpfung mit dem Stickstoffatom. In einer weiteren Ausführungsform der Erfindung sind keine der Gruppen Ar⁴, Ar⁵ bzw. Ar⁶ miteinander verbunden.

Bevorzugt ist Ar⁴ ein aromatisches oder heteroaromatisches Ringsystem mit 6 bis 24 aromatischen Ringatomen, insbesondere mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe.

Bevorzugt sind Ar⁵ und Ar⁶ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar⁵ bzw. Ar⁶ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren, Triphenylen oder Kombinationen aus zwei, drei oder vier dieser Gruppen, welche jeweils mit einem oder mehreren Resten R¹ substituiert sein können. Besonders bevorzugt stehen Ar⁵ und Ar⁶ gleich oder verschieden bei jedem Auftreten für ein aromatisches Ringsystem mit 6 bis 24 aromatischen Ringatomen, welches durch einen oder mehrere Reste R¹ substituiert sein kann, insbesondere ausgewählt aus den Gruppen bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

Dabei haben die Alkylgruppen in Verbindungen, die durch Vakuumverdampfung verarbeitet werden, bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindungen der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es bevorzugt, wenn die Verbindung keine kondensierten Aryl- bzw. Heteroarylgruppen enthält, in denen mehr als zwei Sechsringe direkt aneinander ankondensiert sind. Eine Ausnahme hiervon bilden Phenanthren und Triphenylen, die aufgrund ihrer hohen Triplettenergie trotz der Anwesenheit kondensierter aromatischer Sechsringe bevorzugt sein können.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für geeignete Verbindungen gemäß den oben aufgeführten Ausführungsformen sind die nachfolgend aufgeführten Verbindungen.

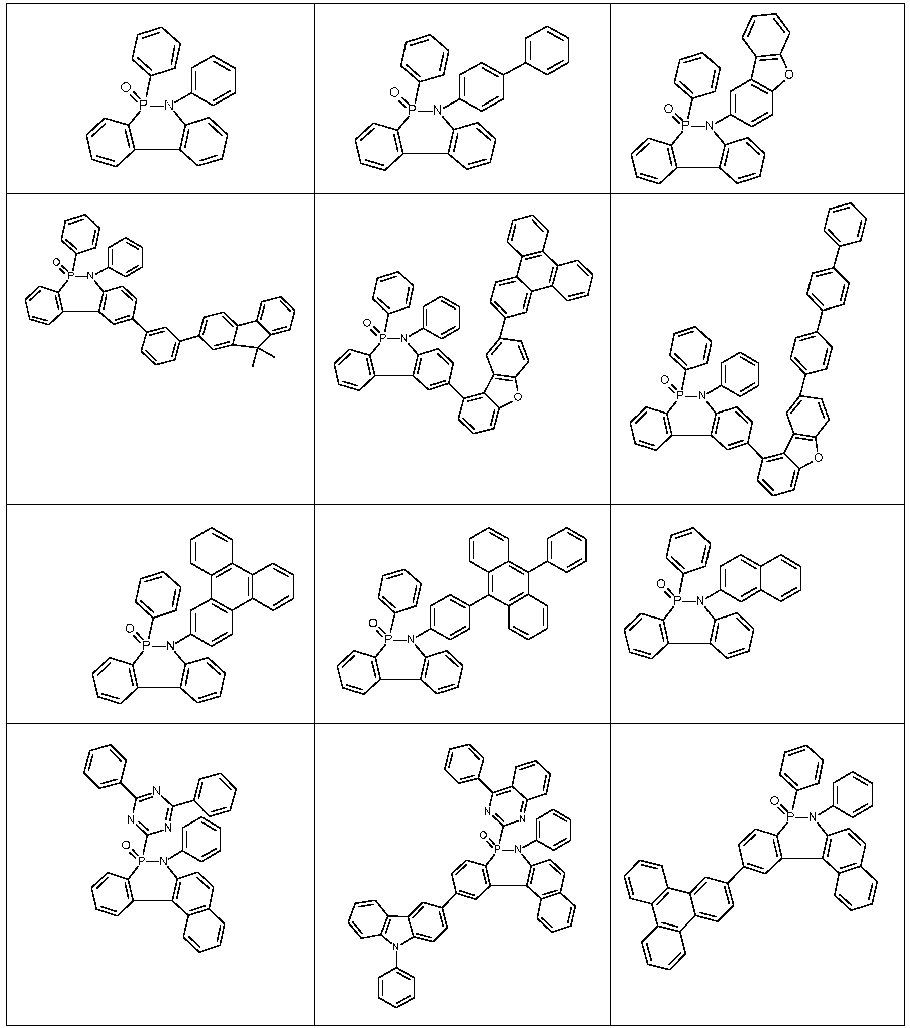

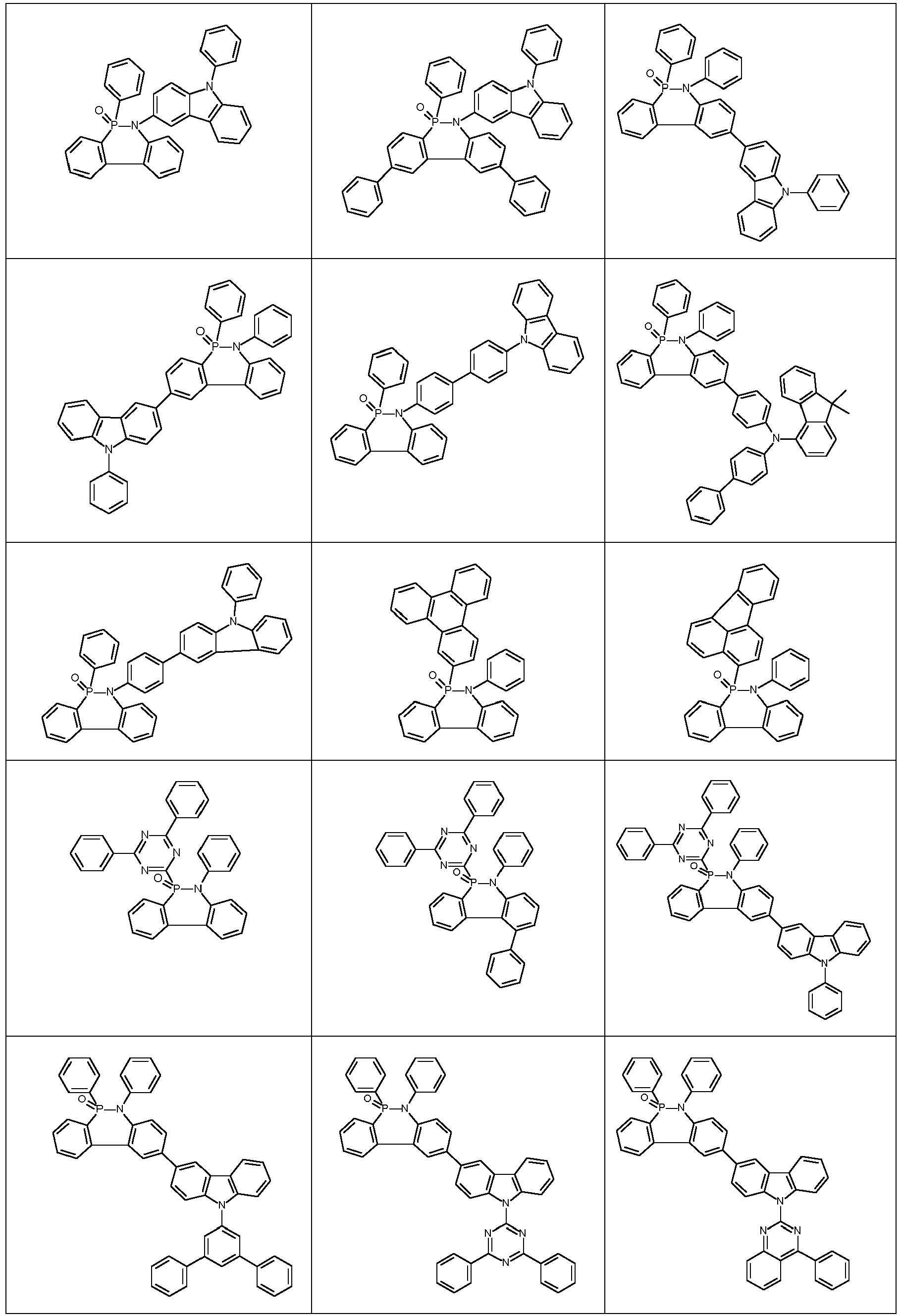

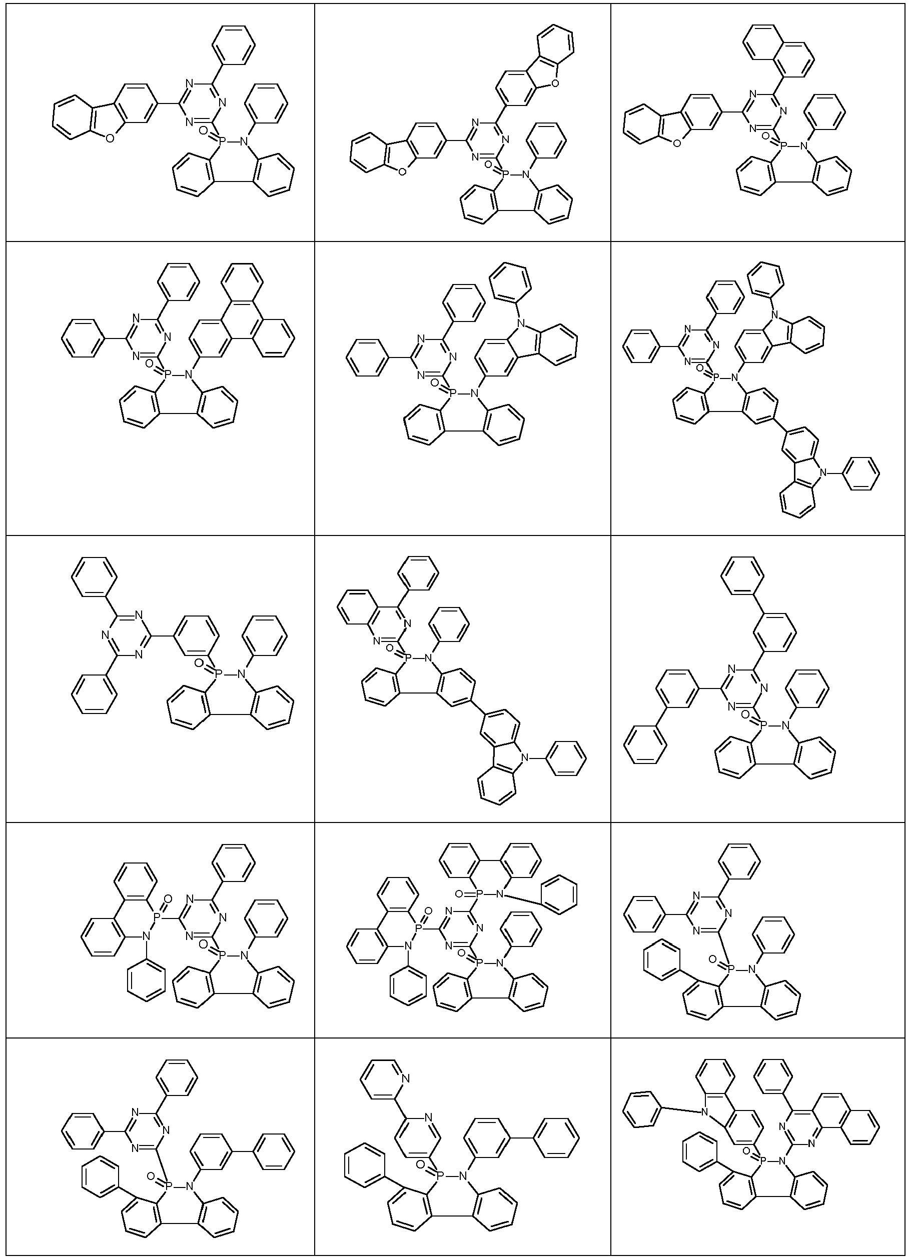

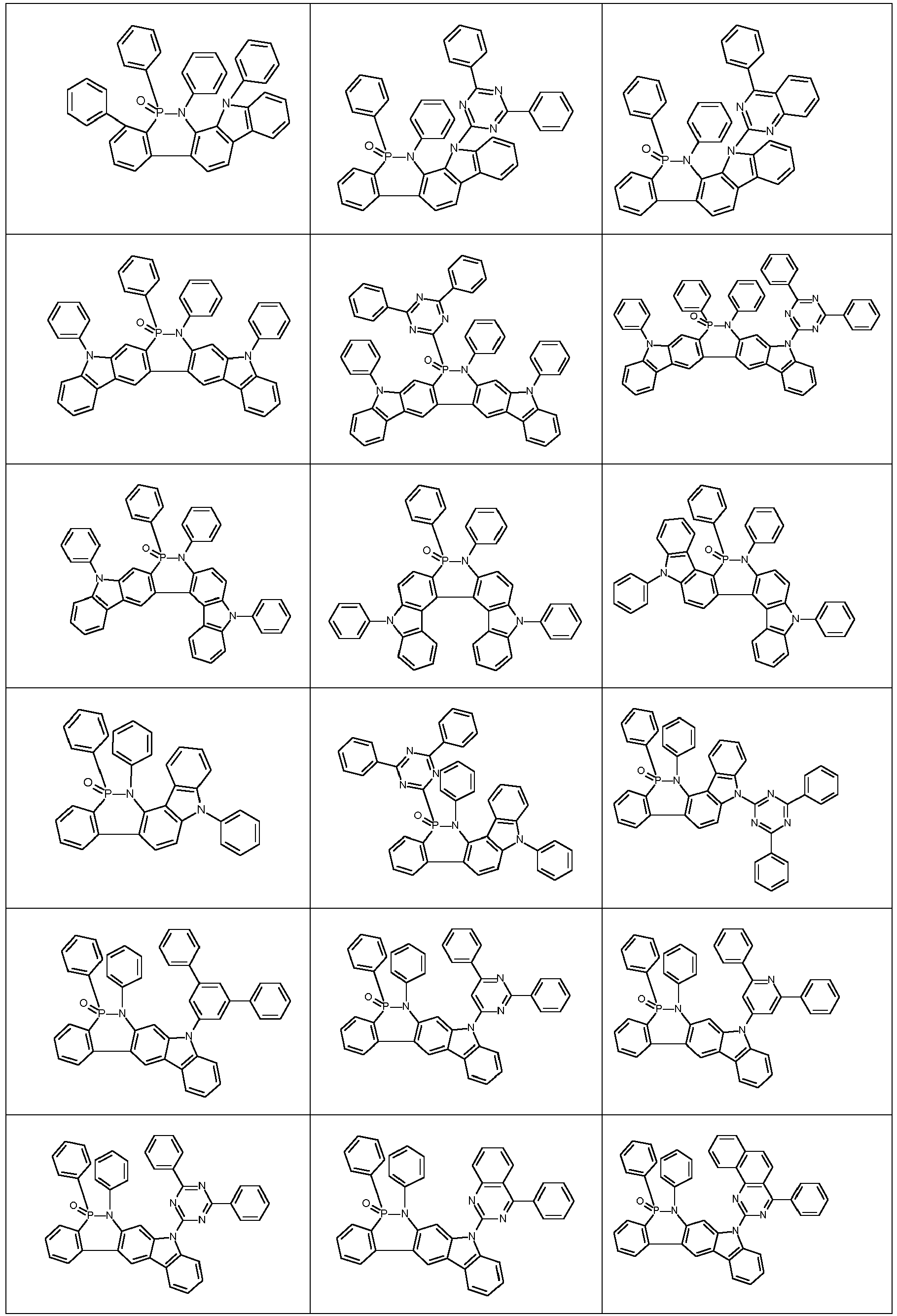

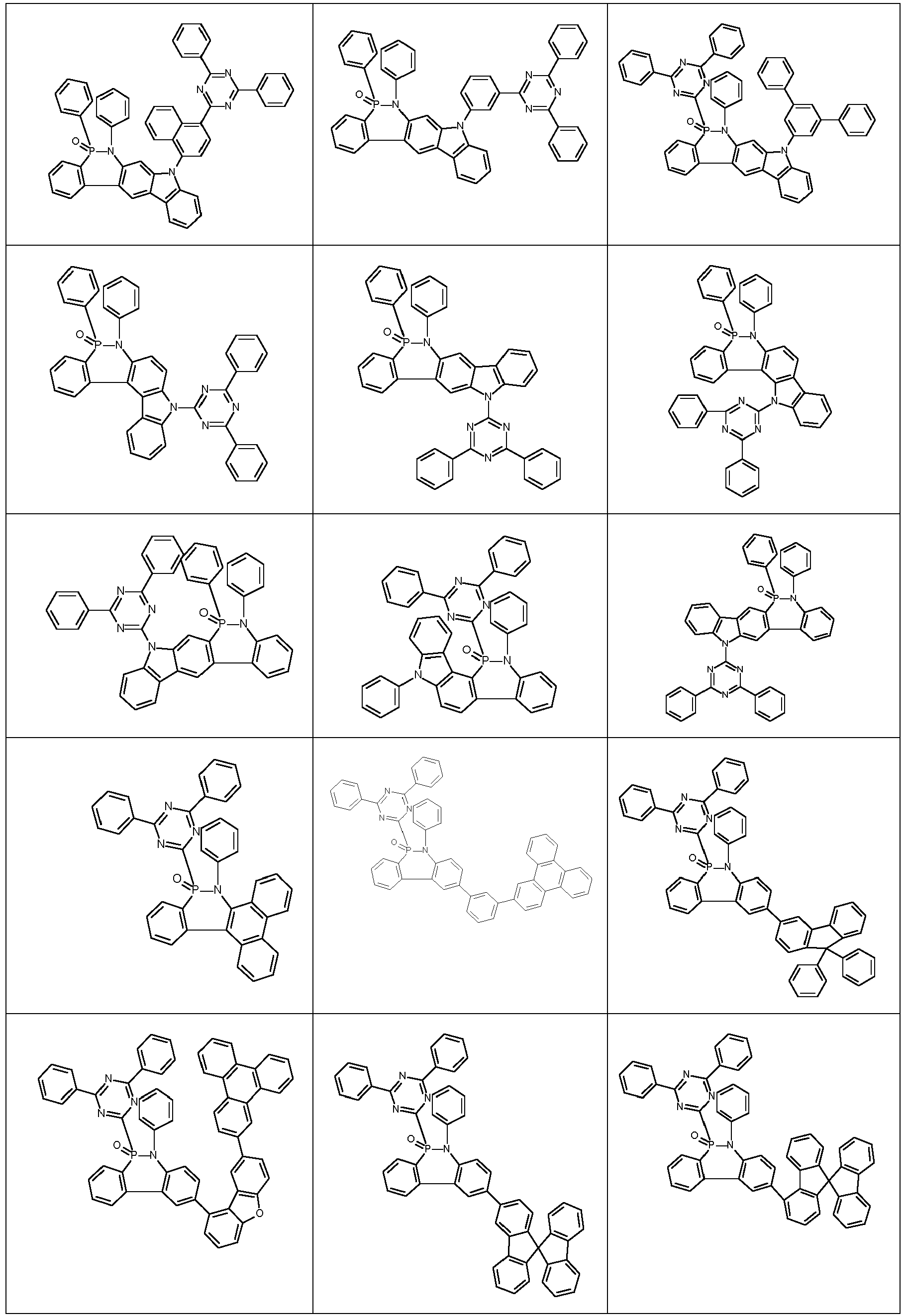

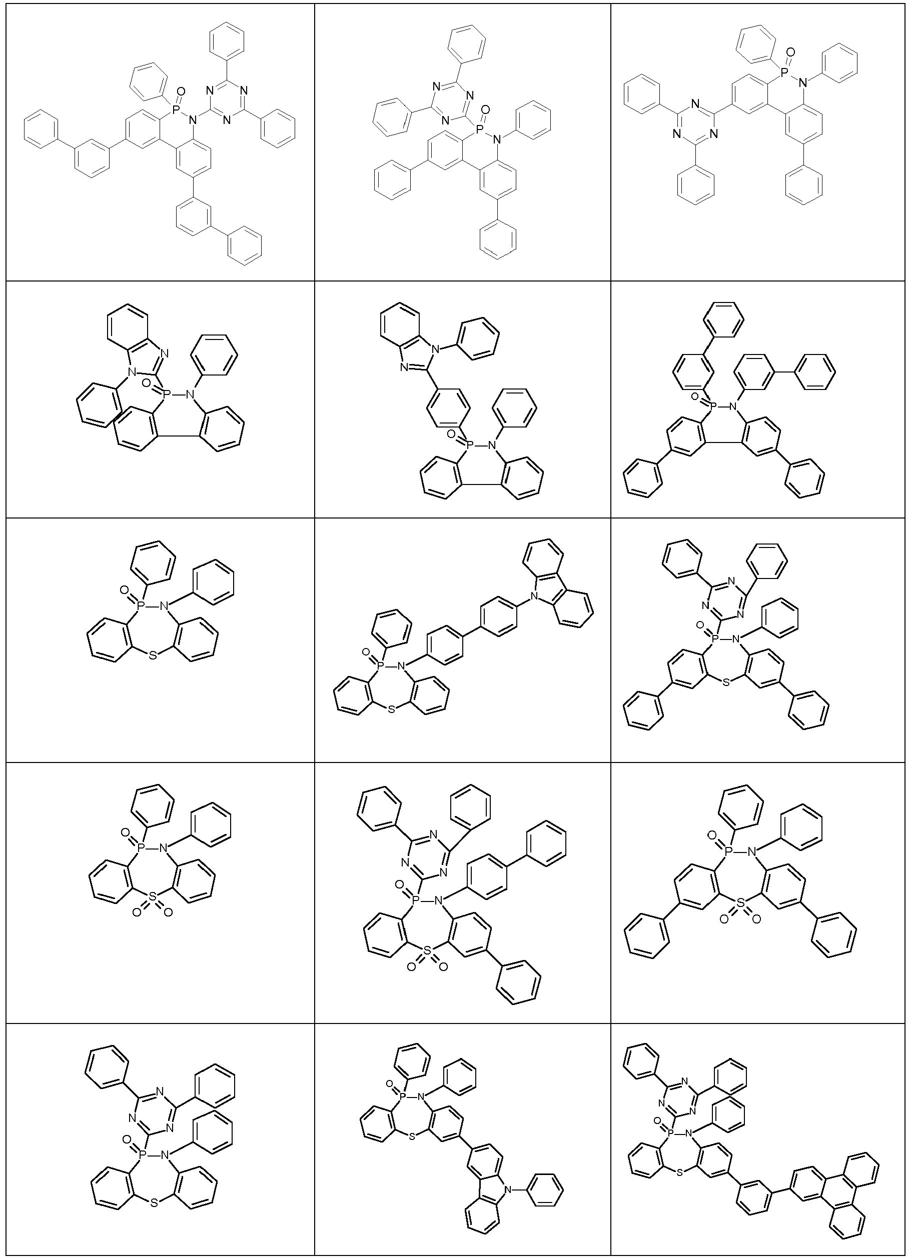

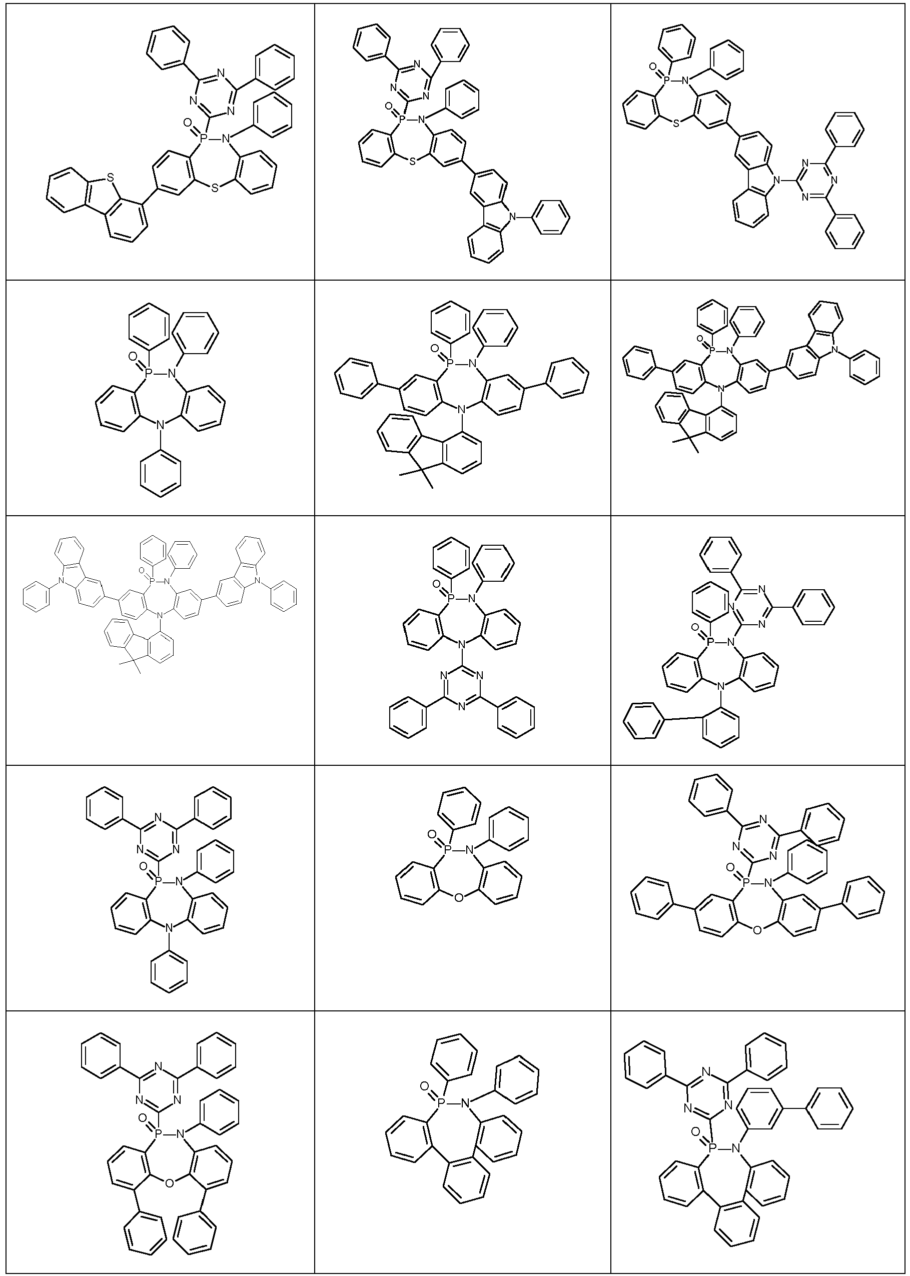

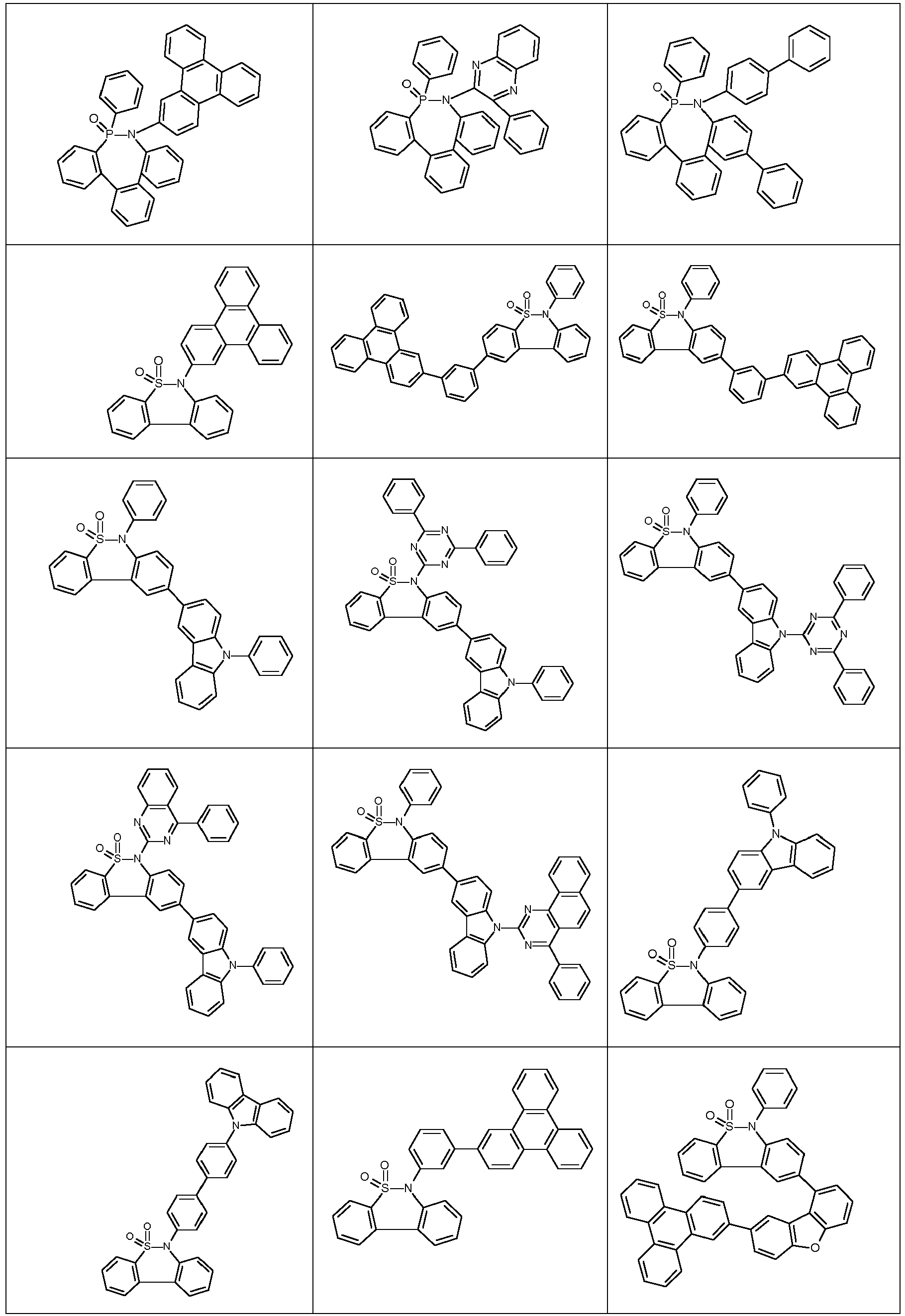

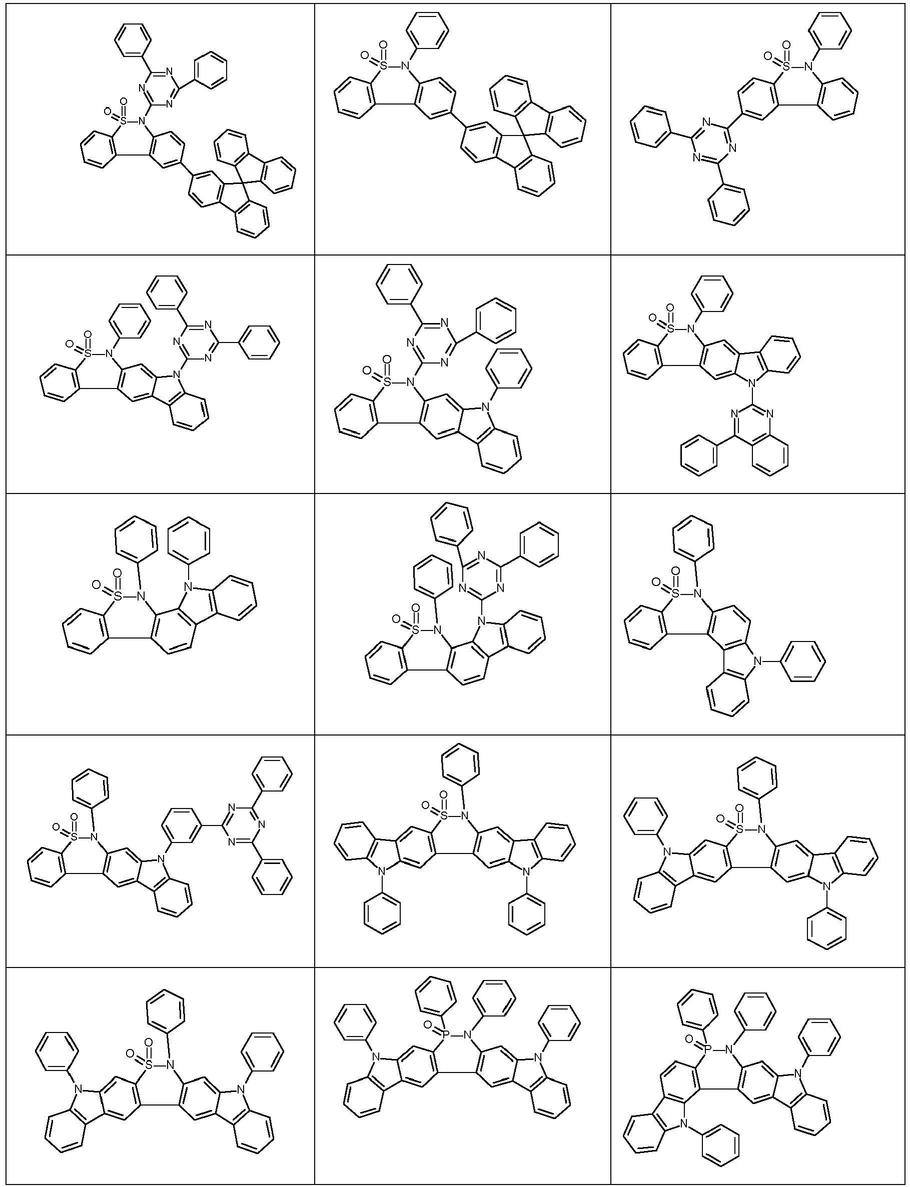

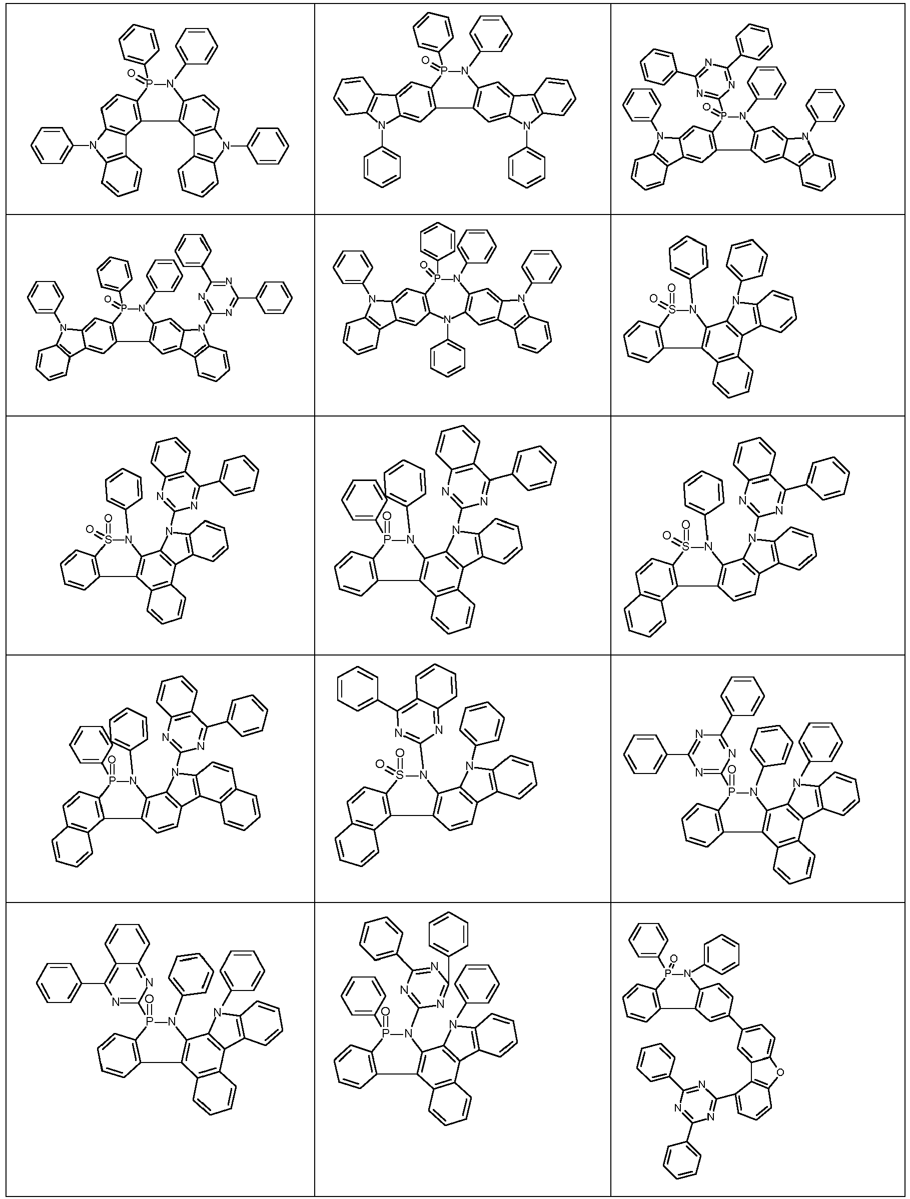

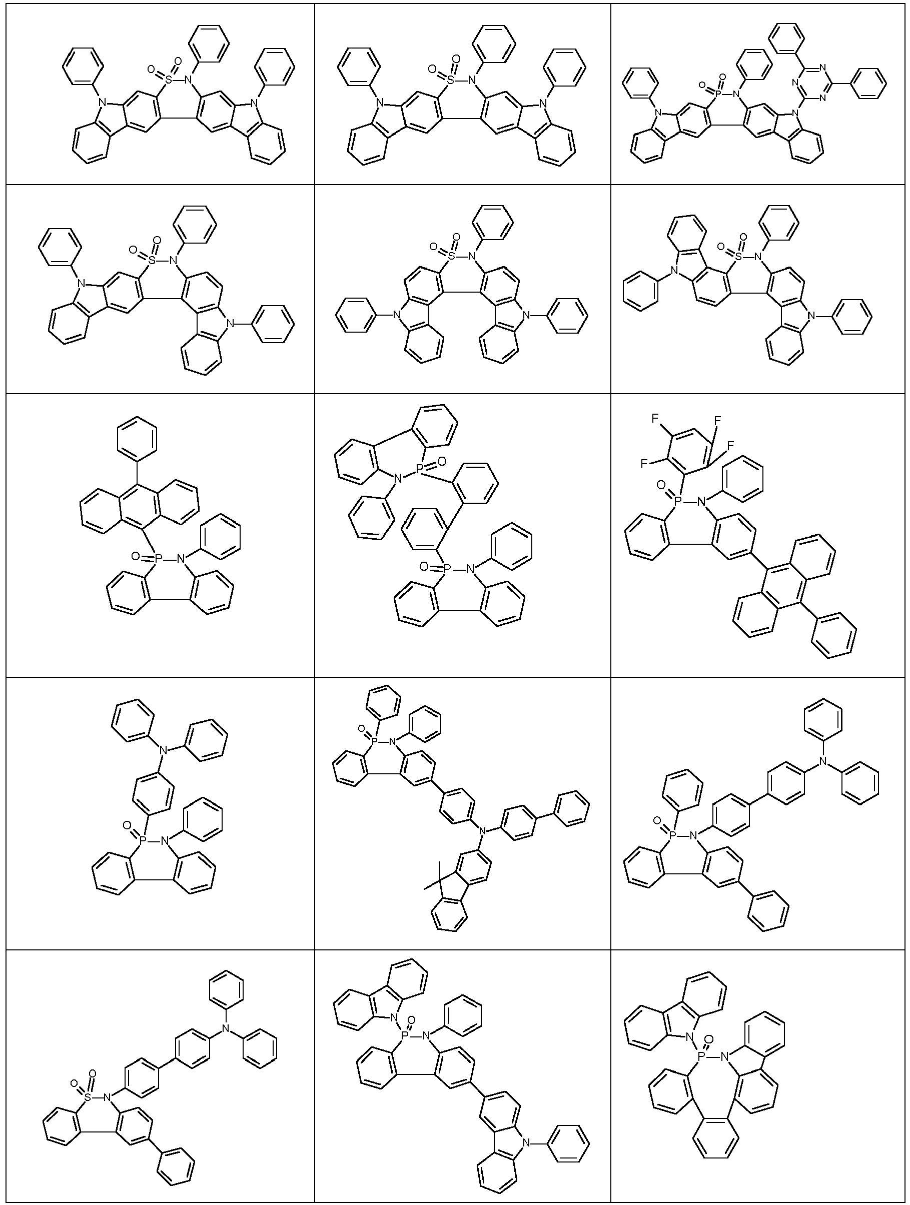

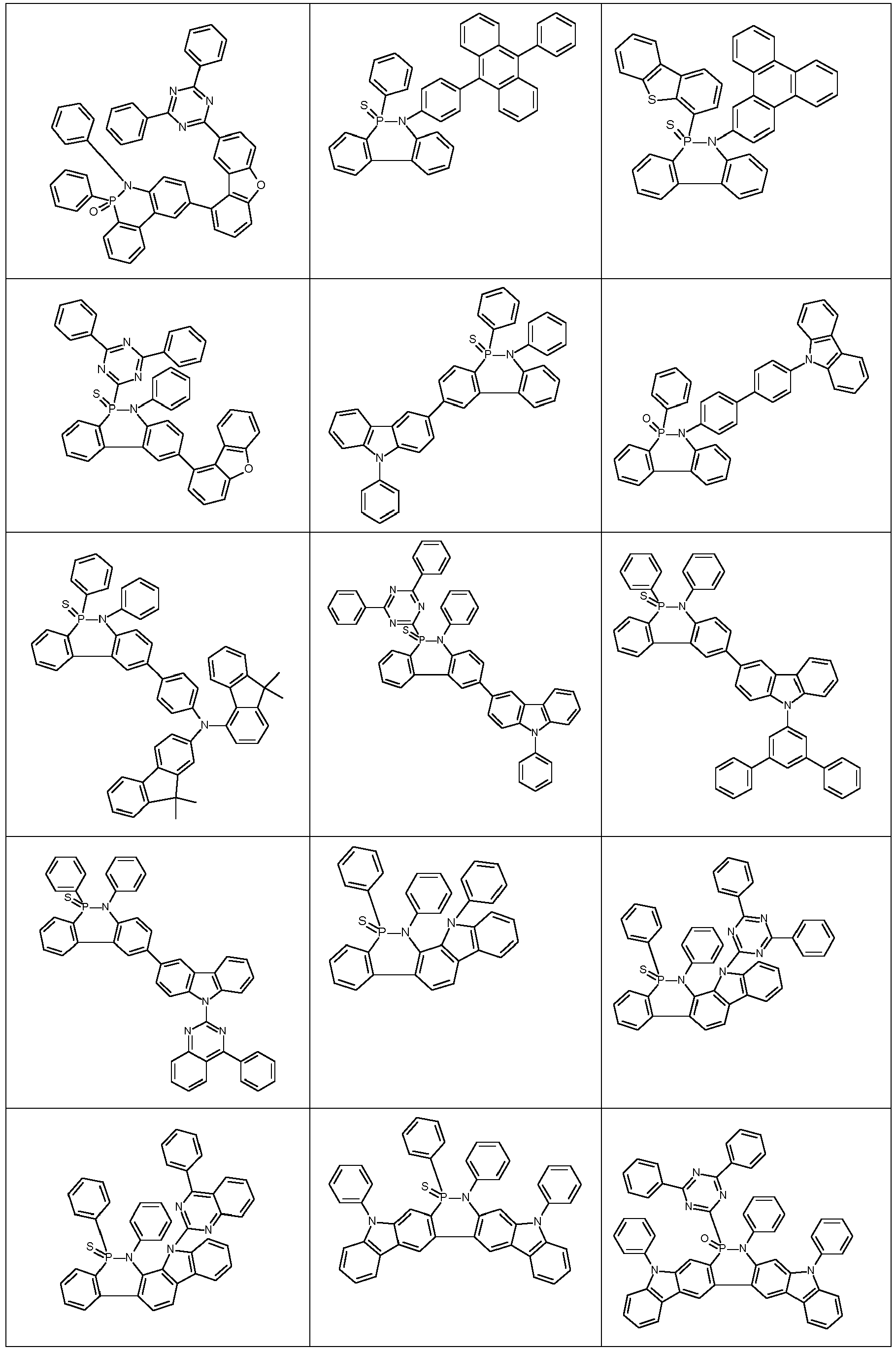

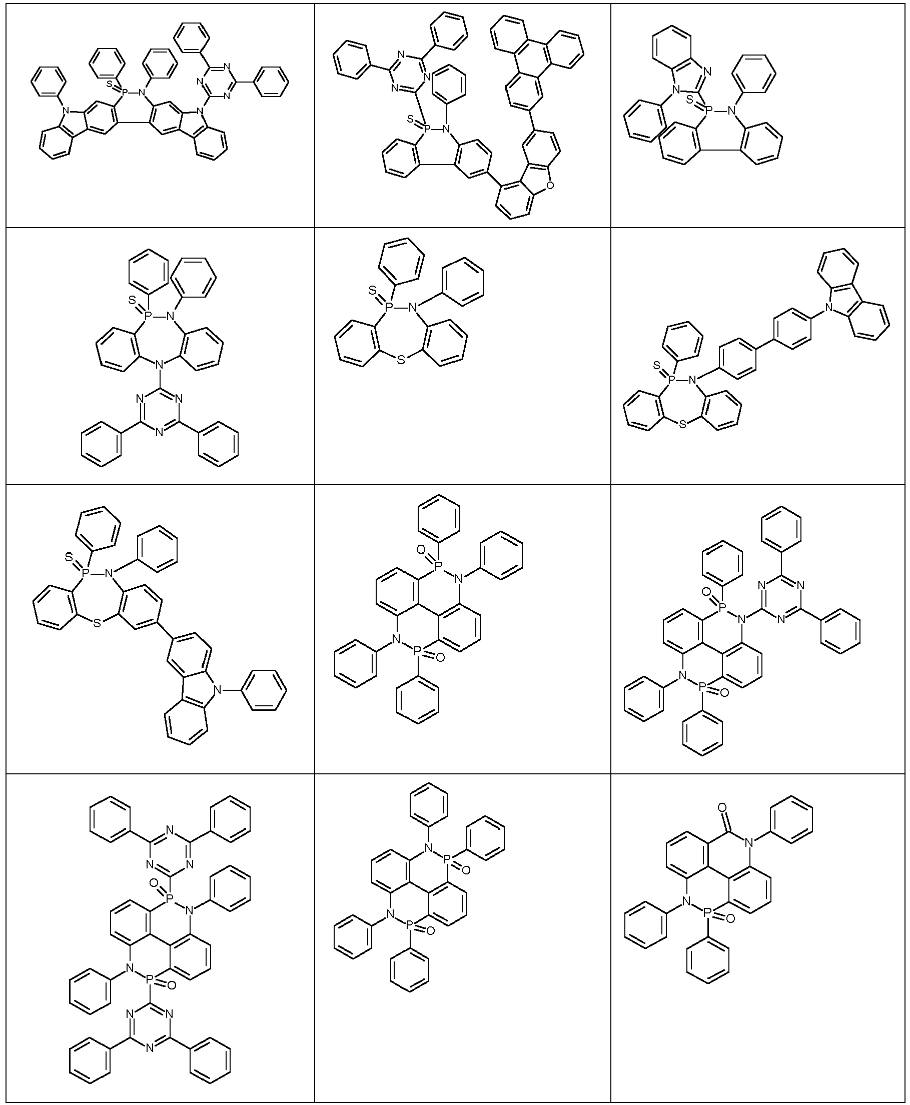

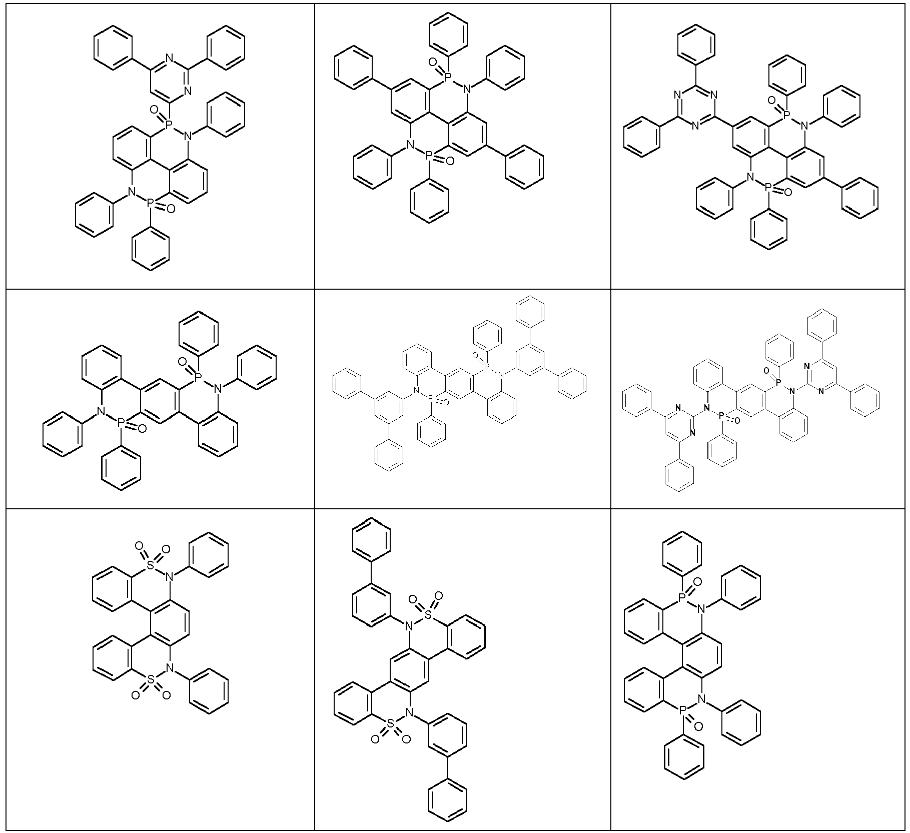

Die Synthese der erfindungsgemäßen Verbindungen kann nach den im Folgenden dargestellten Schemata erfolgen. Dabei sind in den Schemata 1 bis 4 die Synthesen der Verbindungen mit Z = PAr² und in Schema 5 die Synthese der Verbindungen mit Z = S(=O) dargestellt. In den zitierten Literaturstellen werden analoge Reaktionen beschrieben, beispielsweise für Verbindungen mit einer Alkyl- statt einer Aryl- oder Heteroarylgruppe am Stickstoff.

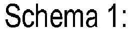

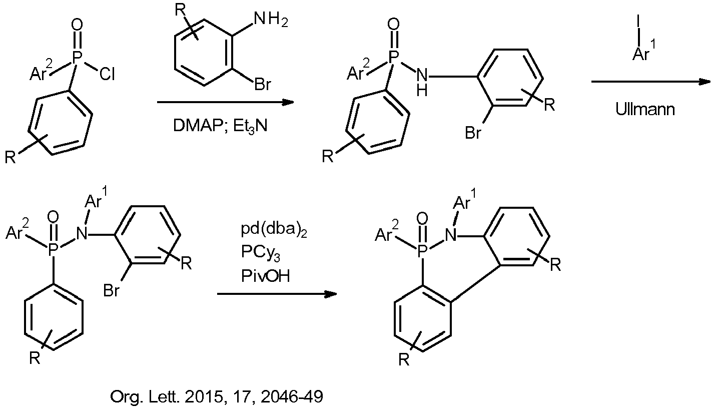

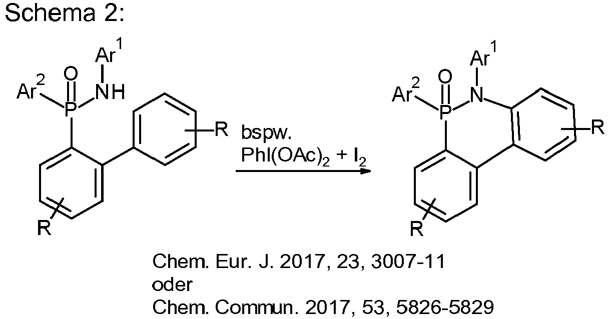

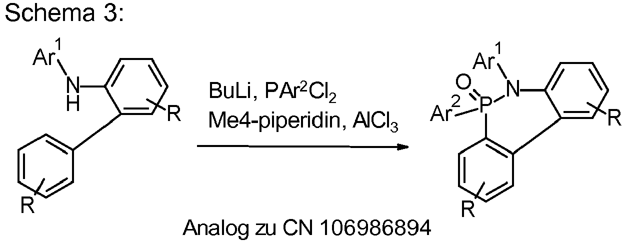

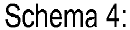

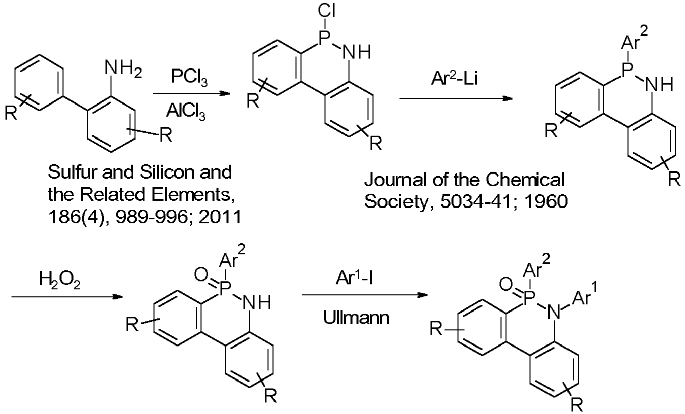

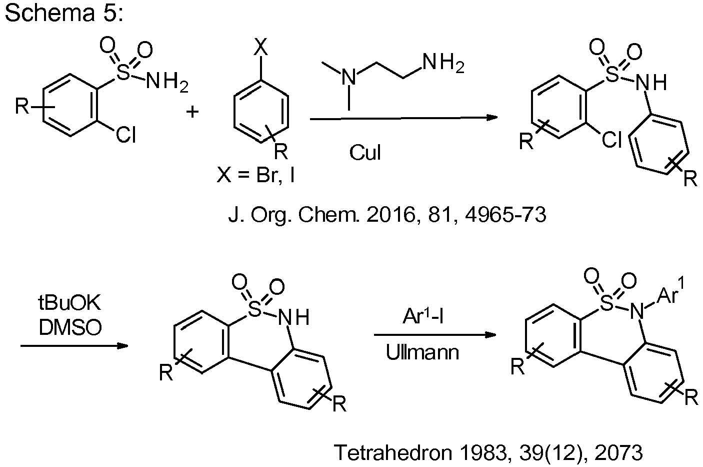

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-OLED handeln, insbesondere für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen in einer emittierenden Schicht als Matrixmaterial für phosphoreszierende Emitter oder für Emitter, die TADF (thermally activated delayed fluorescence) zeigen, insbesondere für phosphoreszierende Emitter. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält. Weiterhin kann die erfindungsgemäße Verbindung auch in einer Elektronentransportschicht und/oder in einer Lochblockierschicht und/oder in einer Lochtransportschicht und/oder in einer Exzitonenblockierschicht eingesetzt werden.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrix-material.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, oder Dibenzofuranderivate, z. B. gemäß WO 2015/169412, WO 2016/015810, WO 2016/023608, WO 2017/148564 oder WO 2017/148565. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in WO 2006/130598, WO 2009/021126, WO 2009/124627 und WO 2010/006680.

In einer bevorzugten Ausführungsform der Erfindung werden die Materialien in Kombination mit einem weiteren Matrixmaterial eingesetzt. Bevorzugte Co-Matrixmaterialien sind gewählt aus der Gruppe der Biscarbazole, der verbrückten Carbazole, der Triarylamine, der Dibenzofuran-Carbazol-Derivate bzw. Dibenzofuran-Amin-Derivate, der Carbazolamine und der Triazinderivate.

Bevorzugte Biscarbazole sind die Strukturen der folgenden Formeln (19) und (20), 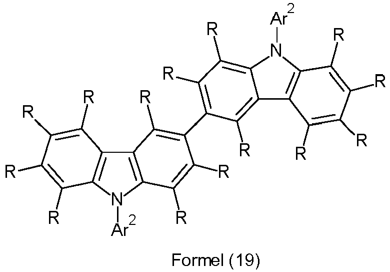 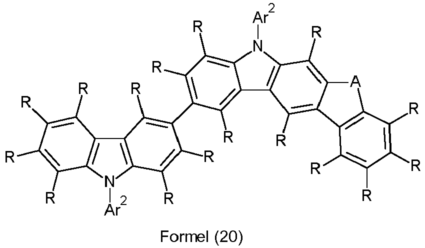 wobei Ar² und A die oben genannten Bedeutungen aufweisen und R die oben genannten Bedeutungen aufweist, jedoch Reste R hier auch miteinander ein aromatisches oder heteroaromatisches Ringsystem bilden können. In einer bevorzugten Ausführungsform der Erfindung steht A für CR₂.

Bevorzugte Ausführungsformen der Verbindungen der Formeln (19) bzw. (20) sind die Verbindungen der folgenden Formeln (19a) bzw. (20a), 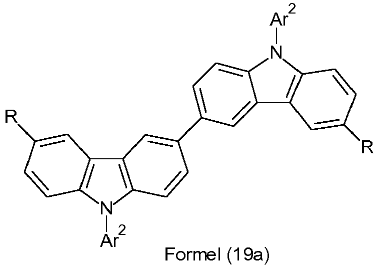 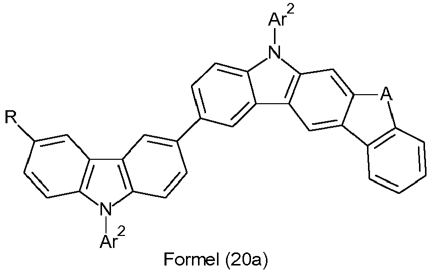 wobei die verwendeten Symbole die oben genannten Bedeutungen aufweisen.

Beispiele für geeignete Verbindungen gemäß Formel (19) oder (20) sind die nachfolgend abgebildeten Verbindungen.

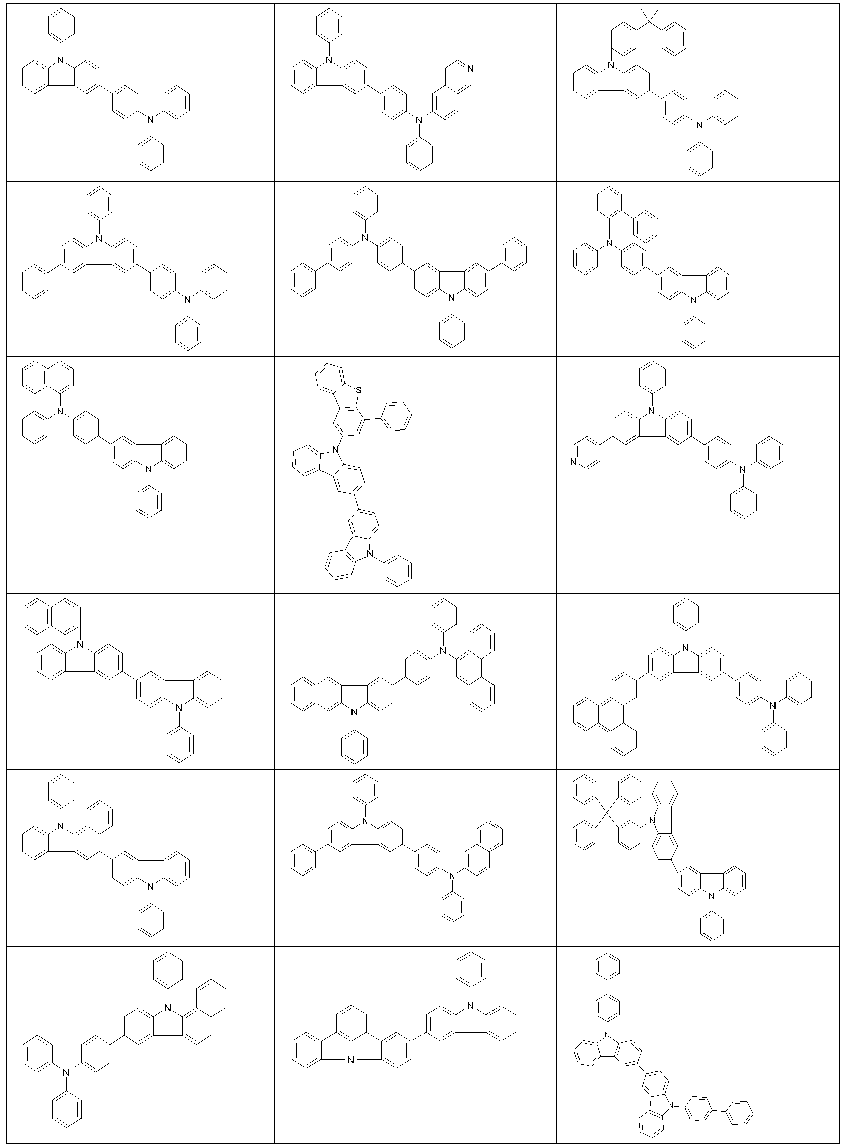

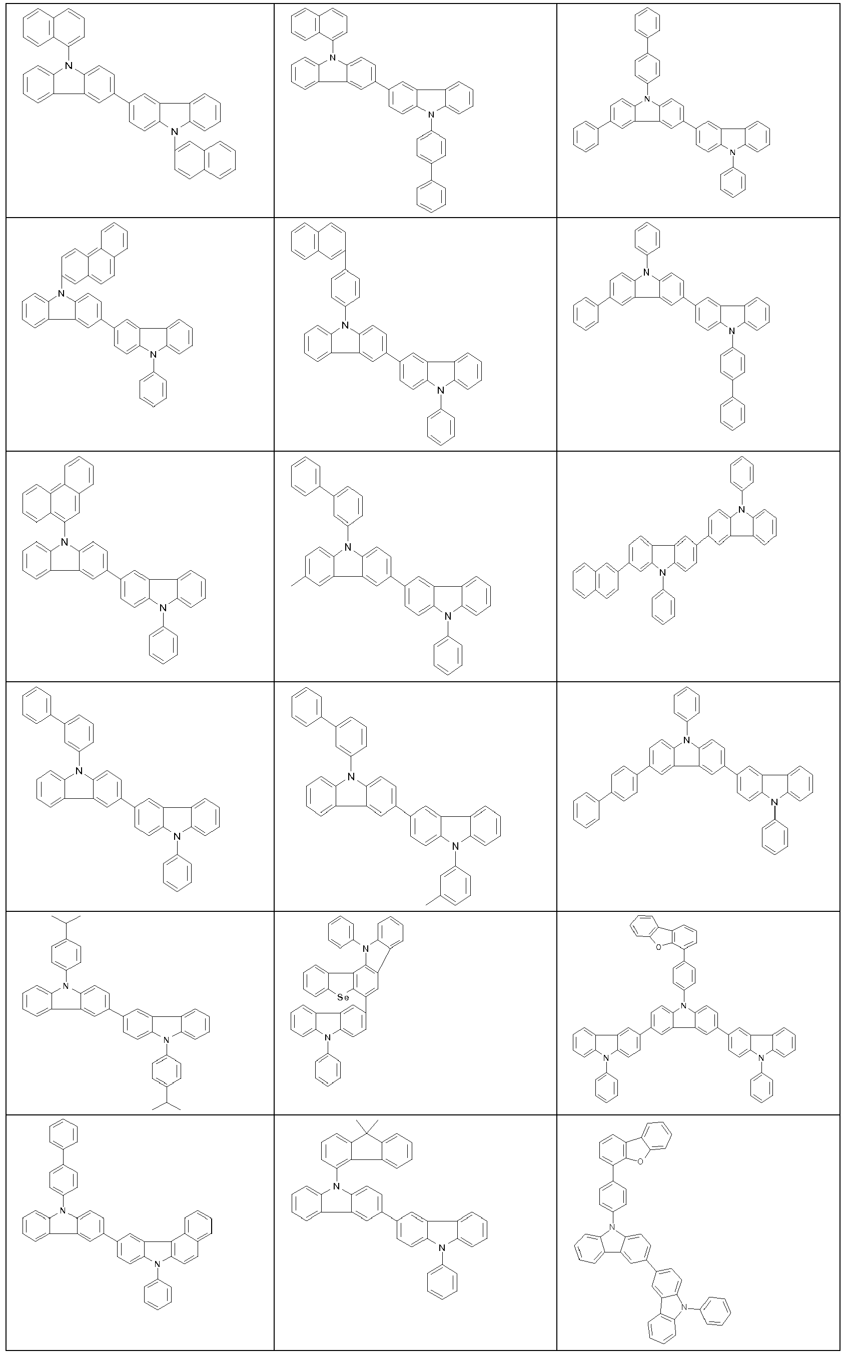

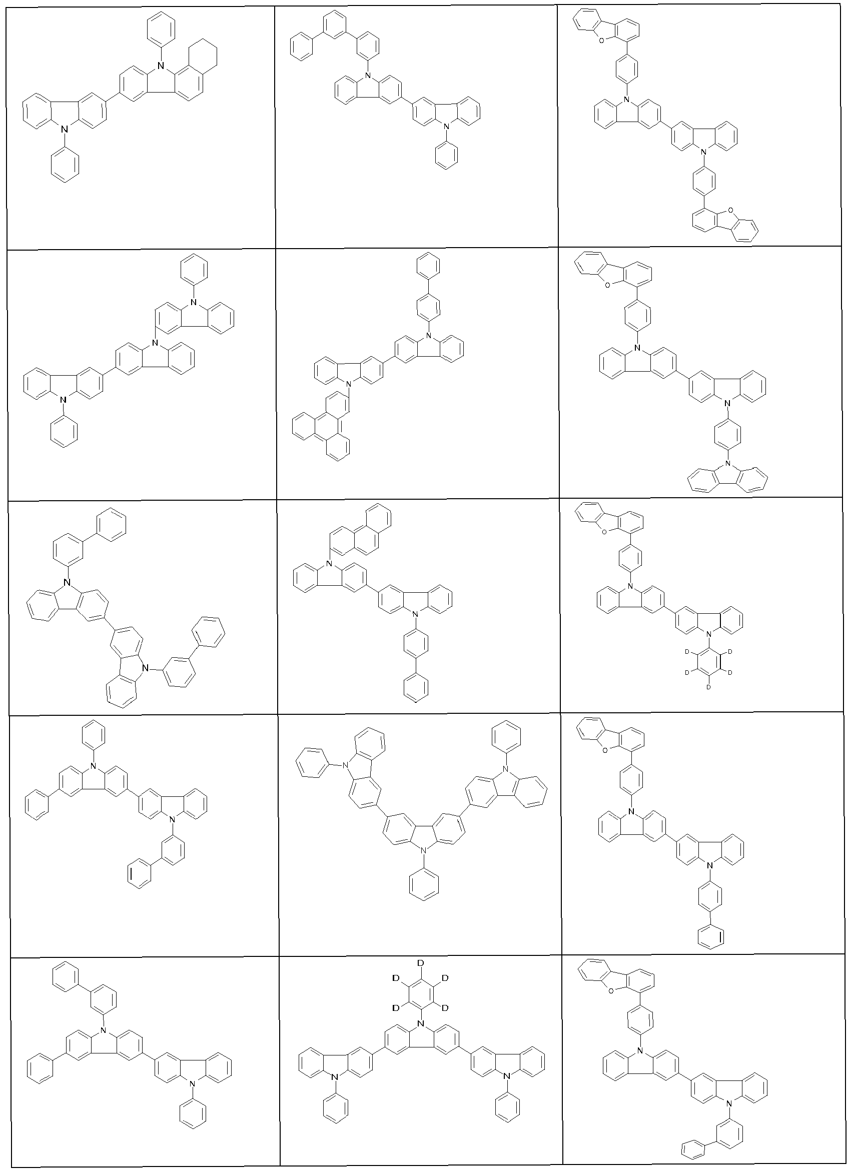

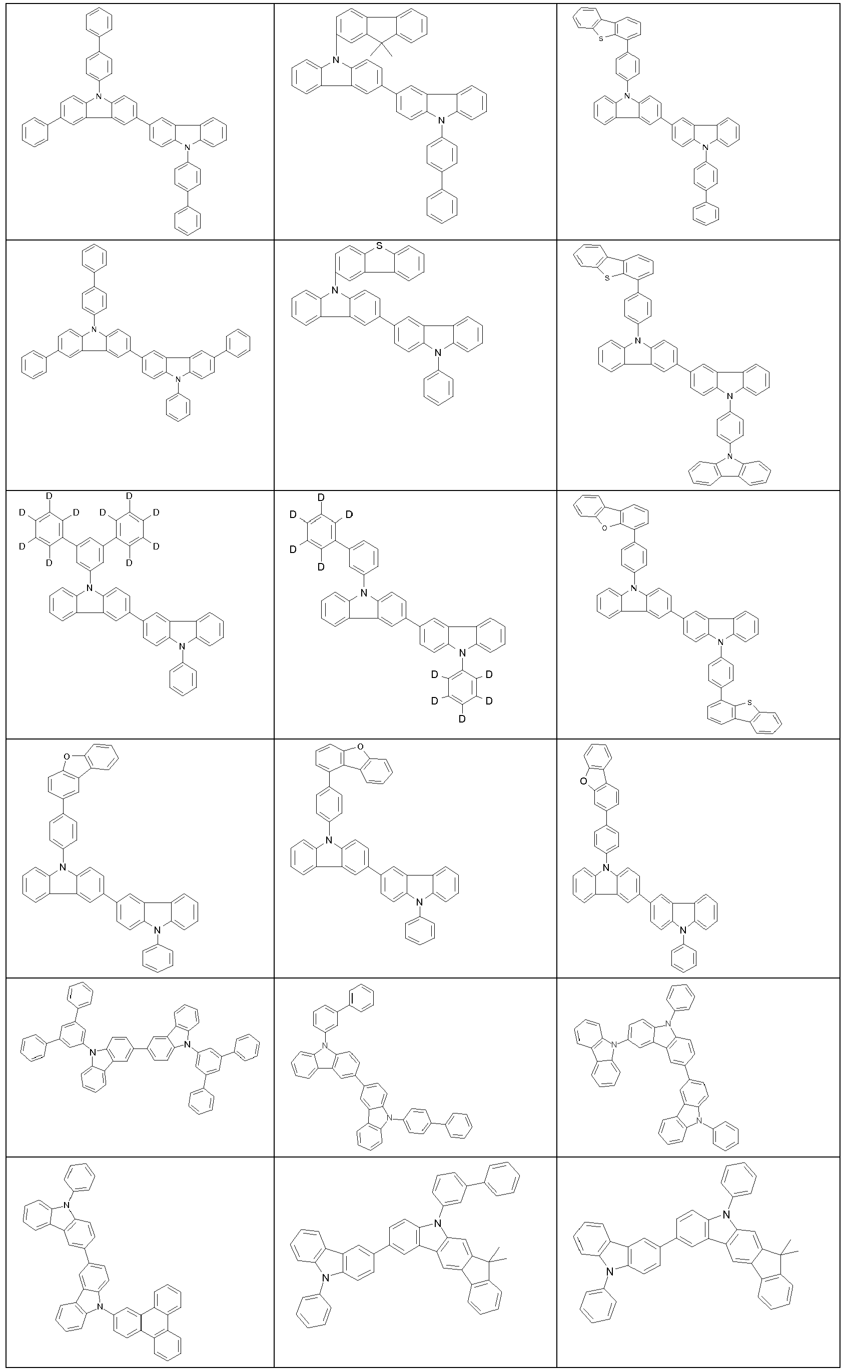

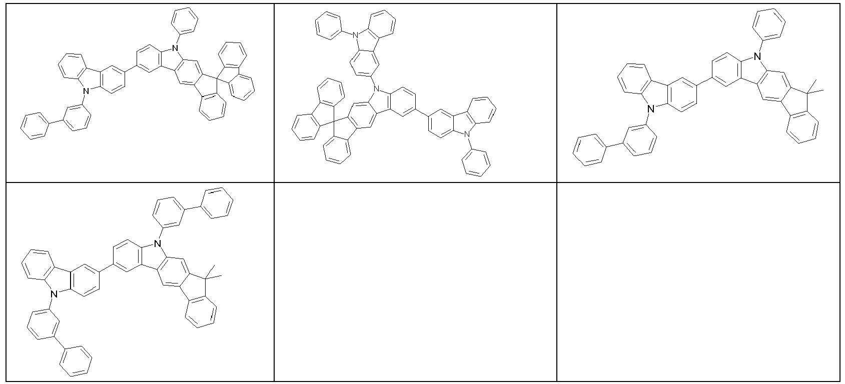

Bevorzugte verbrückte Carbazole sind die Strukturen der folgenden Formel (21), 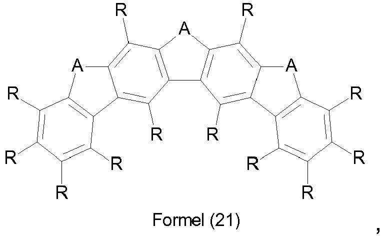 wobei A und R die oben genannten Bedeutungen aufweisen und A bevorzugt gleich oder verschieden bei jedem Auftreten ausgewählt ist aus der Gruppe bestehend aus NAr² und CR₂.

Eine bevorzugte Ausführungsform der Formel (21) sind die Verbindungen der Formel (21a), besonders bevorzugt sind die Verbindungen der Formel (21b), und ganz besonders bevorzugt sind die Verbindungen der Formel (21c), 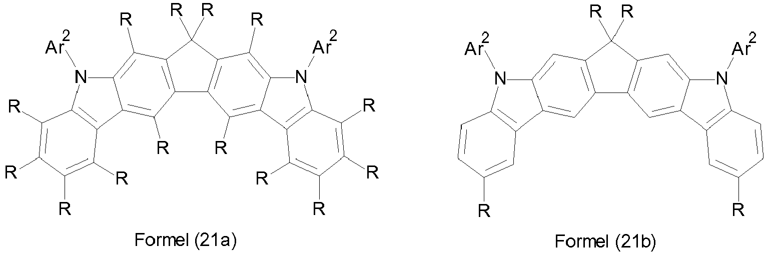 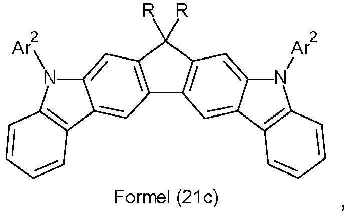 wobei Ar² und R die oben genannten Bedeutungen aufweisen. Dabei steht Ar² bevorzugt gleich oder verschieden bei jedem Auftreten für ein aromatisches Ringsystem mit 6 bis 18 aromatischen Ringatomen, beispielsweise für Phenyl, ortho-, meta- oder para-Biphenyl oder Terphenyl. Der Rest R am Inden-Kohlenstoffatom steht bevorzugt gleich oder verschieden bei jedem Auftreten für eine Alkylgruppe mit 1 bis 5 C-Atomen, insbesondere für Methyl, oder ein aromatisches Ringsystem mit 6 bis 18 aromatischen Ringatomen, insbesondere Phenyl.

Bevorzugte Triarylamine sind die Strukturen der folgenden Formel (22), 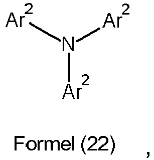 wobei Ar² die oben genannten Bedeutungen aufweist.

Bevorzugte Dibenzofuran-Derivate sind die Verbindungen der folgenden Formel (23), 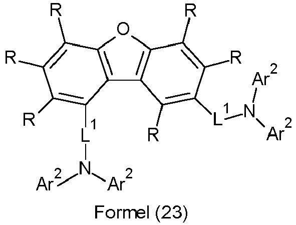 wobei der Sauerstoff auch durch Schwefel ersetzt sein kann, so dass ein Dibenzothiophen entsteht, L¹ für eine Einfachbindung oder ein aromatisches oder heteroaromatisches Ringsystem mit 5 bis 30 aromatischen Ringatomen steht, welches auch durch eine oder mehrere Reste R substituiert sein kann, und R und Ar² die oben genannten Bedeutungen aufweisen. Dabei können die beiden Gruppen Ar², die an dasselbe Stickstoffatom binden, oder eine Gruppe Ar² und eine Gruppe L¹, die an dasselbe Stickstoffatom binden, auch miteinander verbunden sein, beispielsweise zu einem Carbazol.

Bevorzugte Ausführungsformen der Verbindungen der Formel (23) sind die Verbindungen der folgenden Formeln (23a) und (23b), 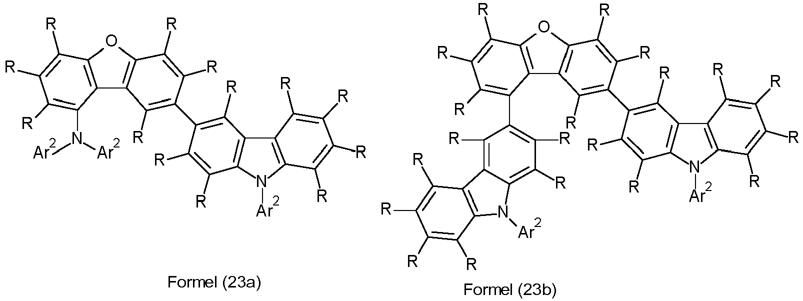 wobei der Sauerstoff auch durch Schwefel ersetzt sein kann, so dass ein Dibenzothiophen entsteht, und R und Ar² die oben genannten Bedeutungen aufweisen, wobei zwei benachbarte Reste R, insbesondere am Carbazol, hier auch miteinander ein aromatisches oder heteroaromatisches Ringsystem bilden können.

Besonders bevorzugte Ausführungsformen sind die Verbindungen der folgenden Formeln (23c) und (23d), 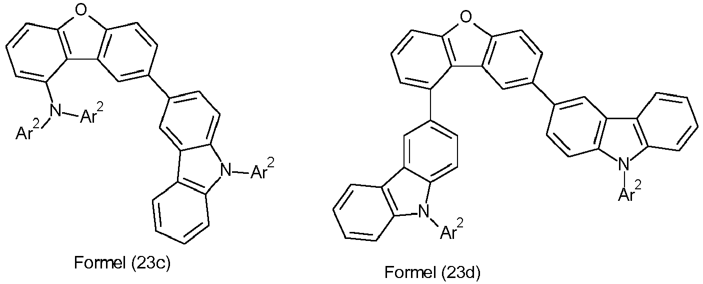 wobei der Sauerstoff auch durch Schwefel ersetzt sein kann, so dass ein Dibenzothiophen entsteht, und Ar² die oben genannten Bedeutungen aufweist, wobei zwei benachbarte Reste R, insbesondere am Carbazol, hier auch miteinander ein aromatisches oder heteroaromatisches Ringsystem bilden können.

Beispiele für geeignete Dibenzofuran-Derivate sind die nachfolgend abgebildeten Verbindungen.

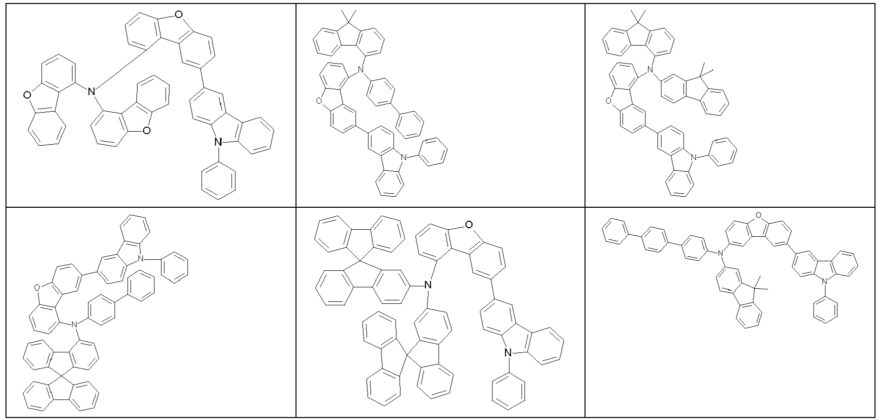

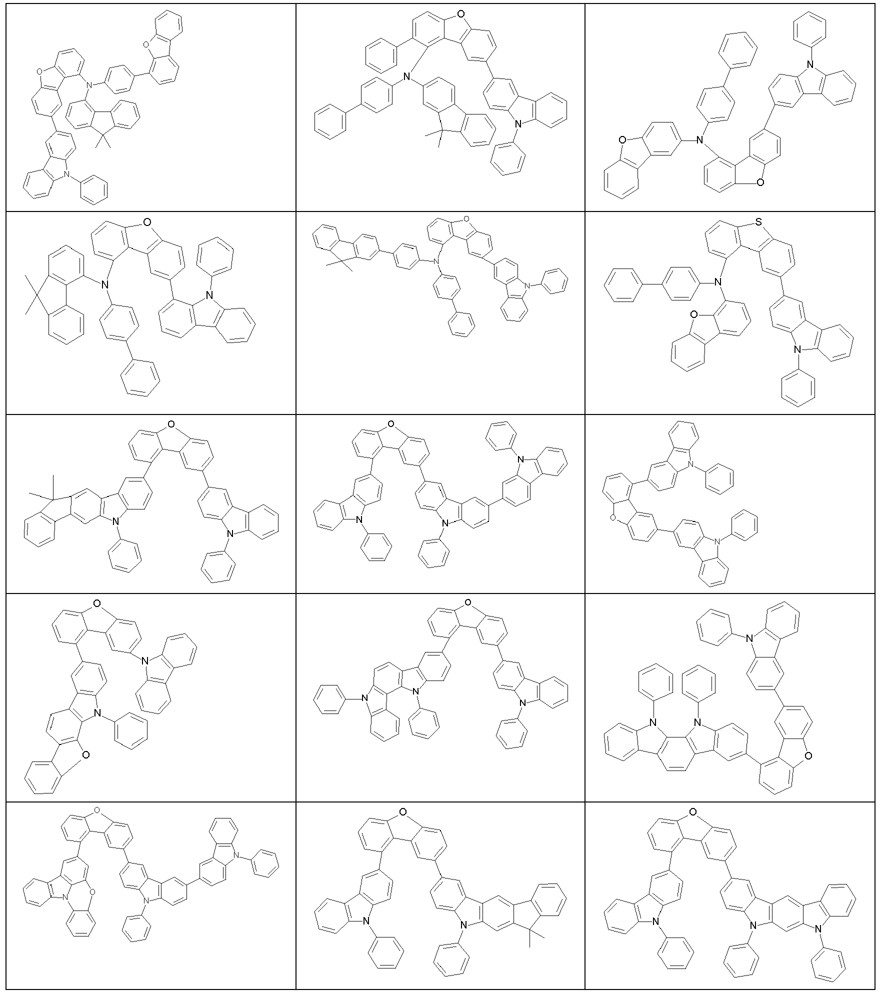

Bevorzugte Carbazolamine sind die Strukturen der folgenden Formeln (24), (25) und (26), 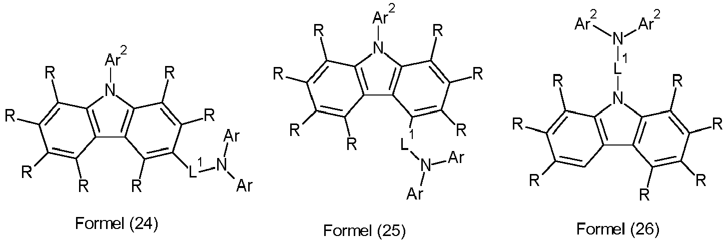 wobei L¹ für ein aromatisches oder heteroaromatisches Ringsystem mit 5 bis 30 aromatischen Ringatomen steht, welches mit einem oder mehreren Resten R substituiert sein kann, und R und Ar² die oben genannten Bedeutungen aufweisen, wobei zwei benachbarte Reste R hier auch ein aromatisches Ringsystem bilden können.

Beispiele für geeignete Carbazolamin-Derivate sind die nachfolgend abgebildeten Verbindungen.

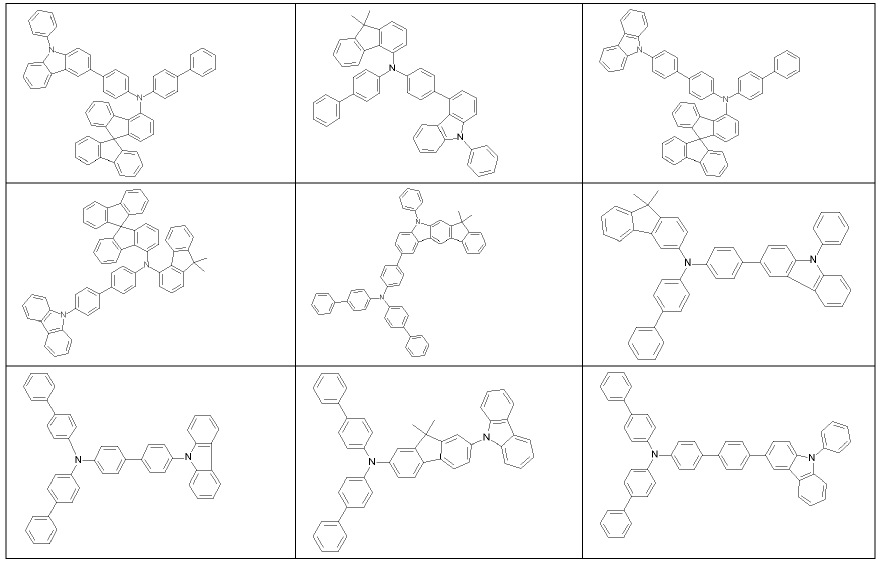

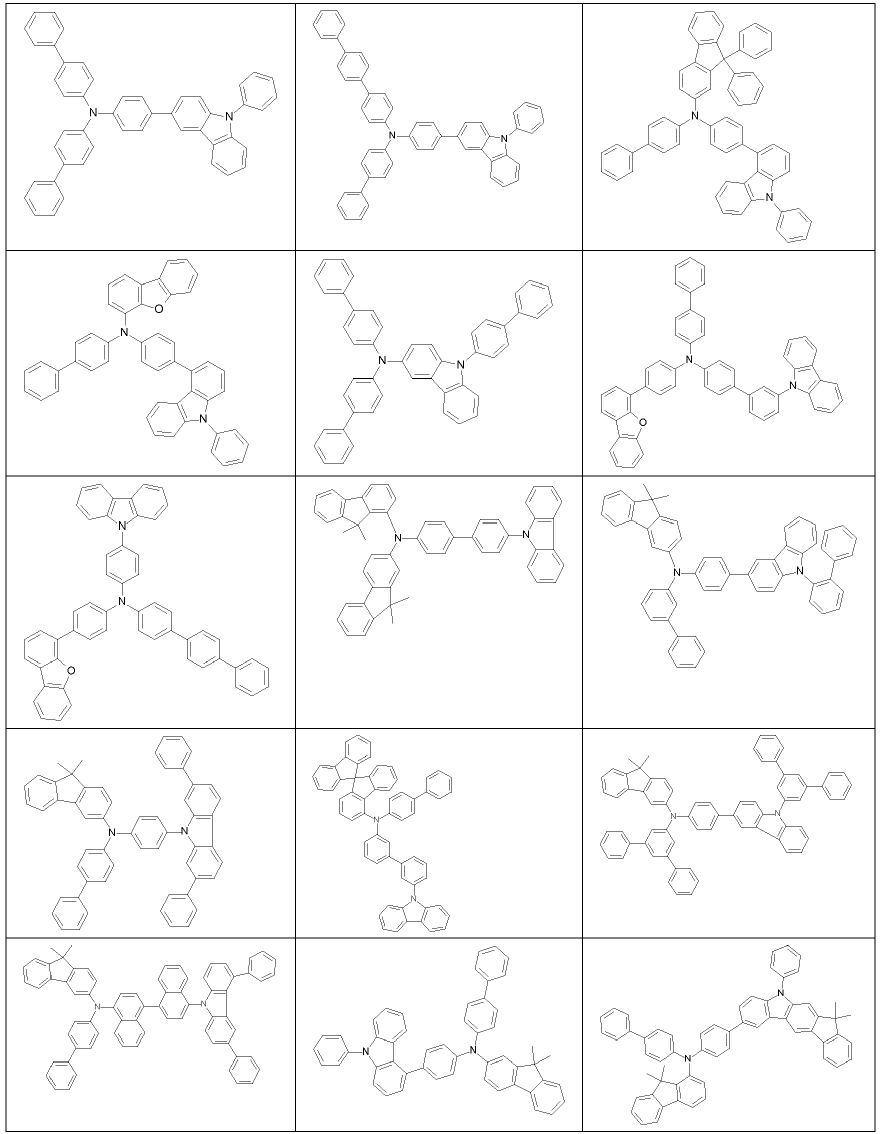

Bevorzugte Triazin- bzw. Pyrimidinderivate, welche als Mischung zusammen mit den erfindungsgemäßen Verbindungen eingesetzt werden können, sind die Verbindungen der folgenden Formeln (27) und (28), 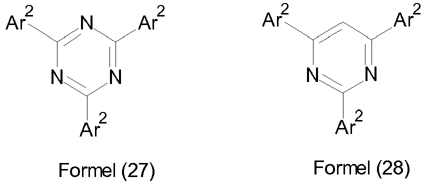 wobei Ar² die oben genannten Bedeutungen aufweist. Dabei gelten die oben aufgeführten bevorzugten Ausführungsformen für Ar² auch für die Verbindungen der Formeln (27) und (28). Besonders bevorzugt sind die Triazinderivate der Formel (27).

Beispiele für Triazinderivate, welche als Matrixmaterialien zusammen mit den erfindungsgemäßen Verbindungen eingesetzt werden können, sind die nachfolgend aufgeführten Verbindungen:

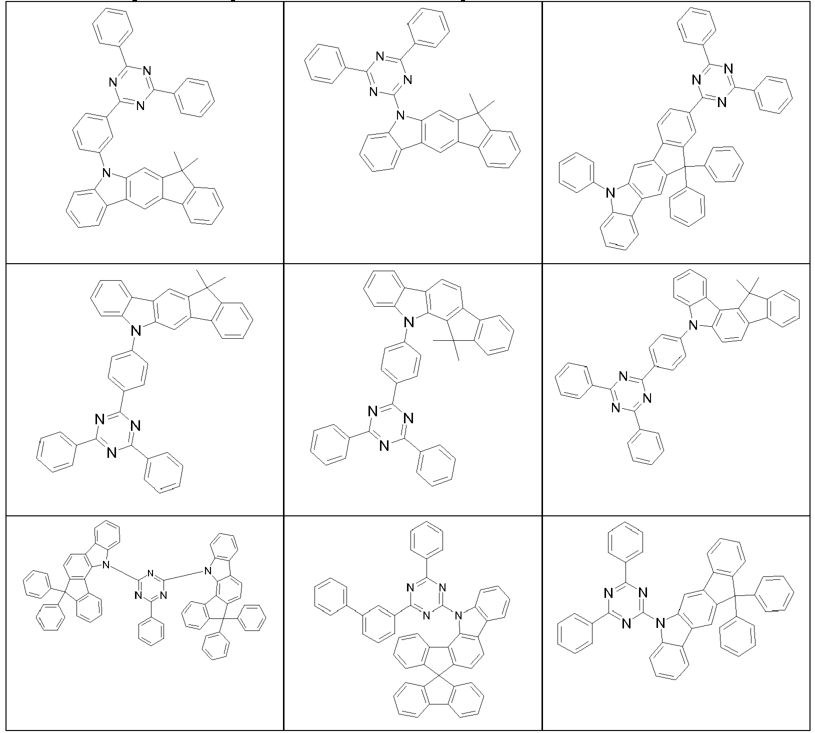

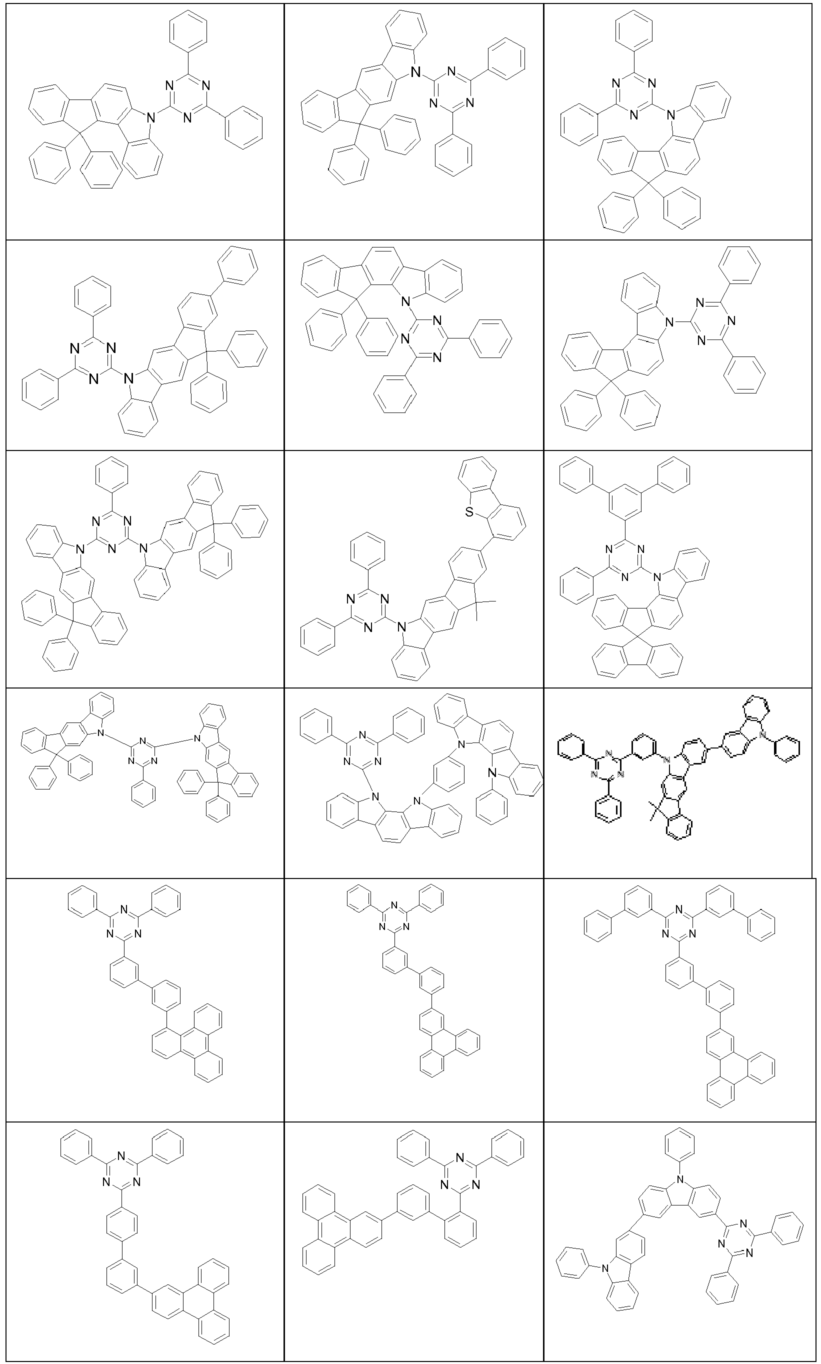

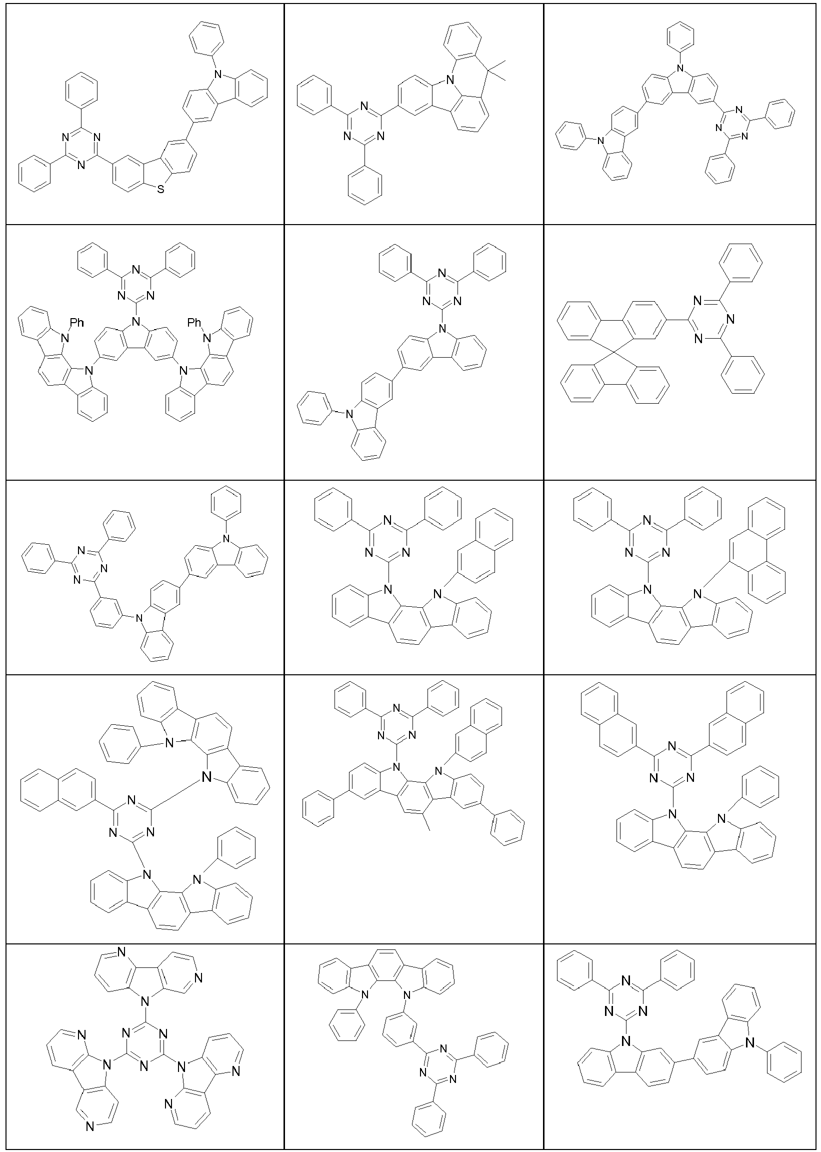

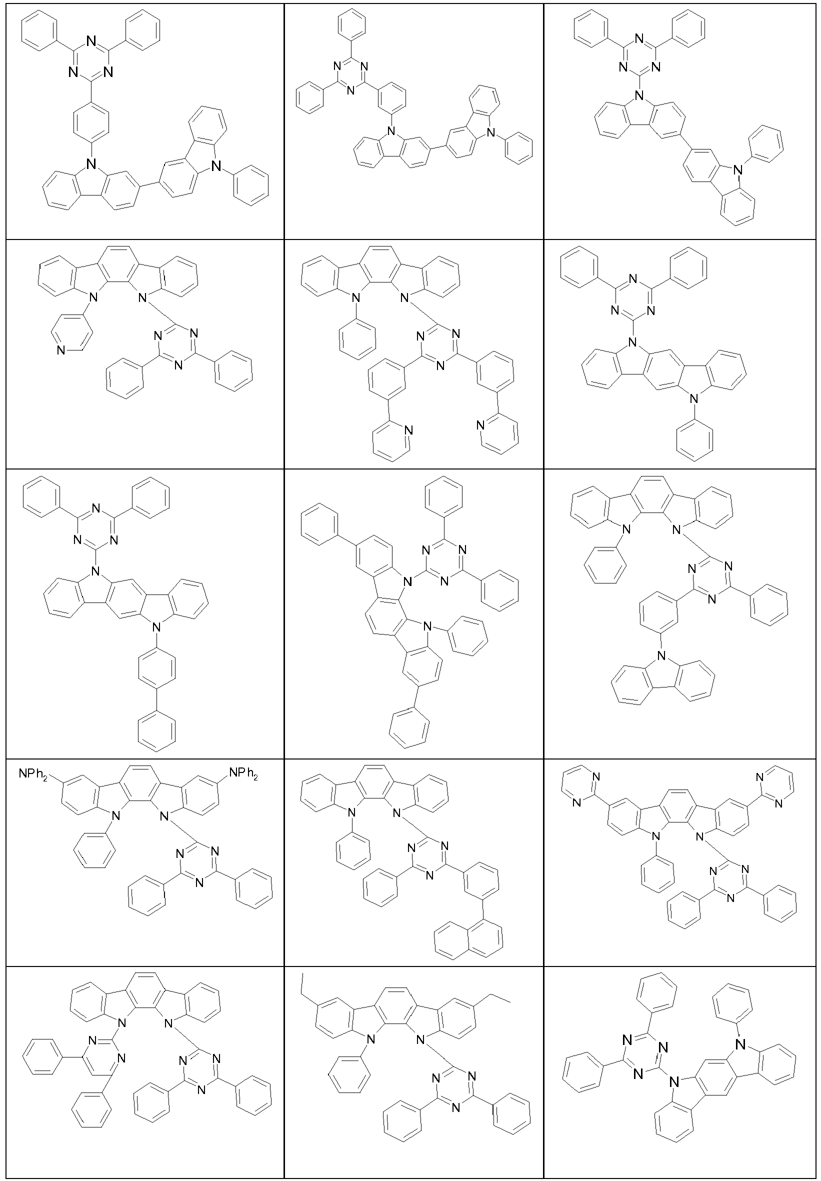

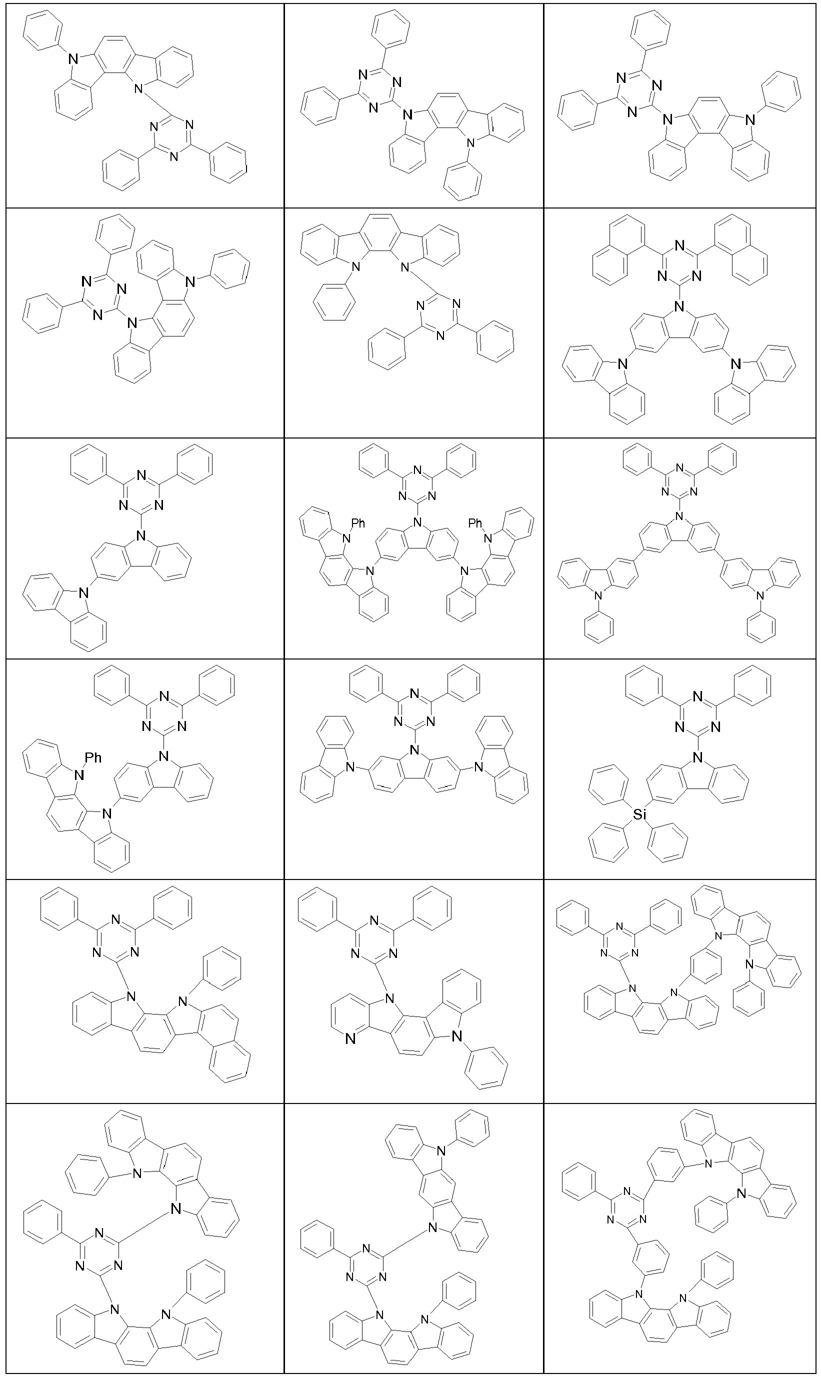

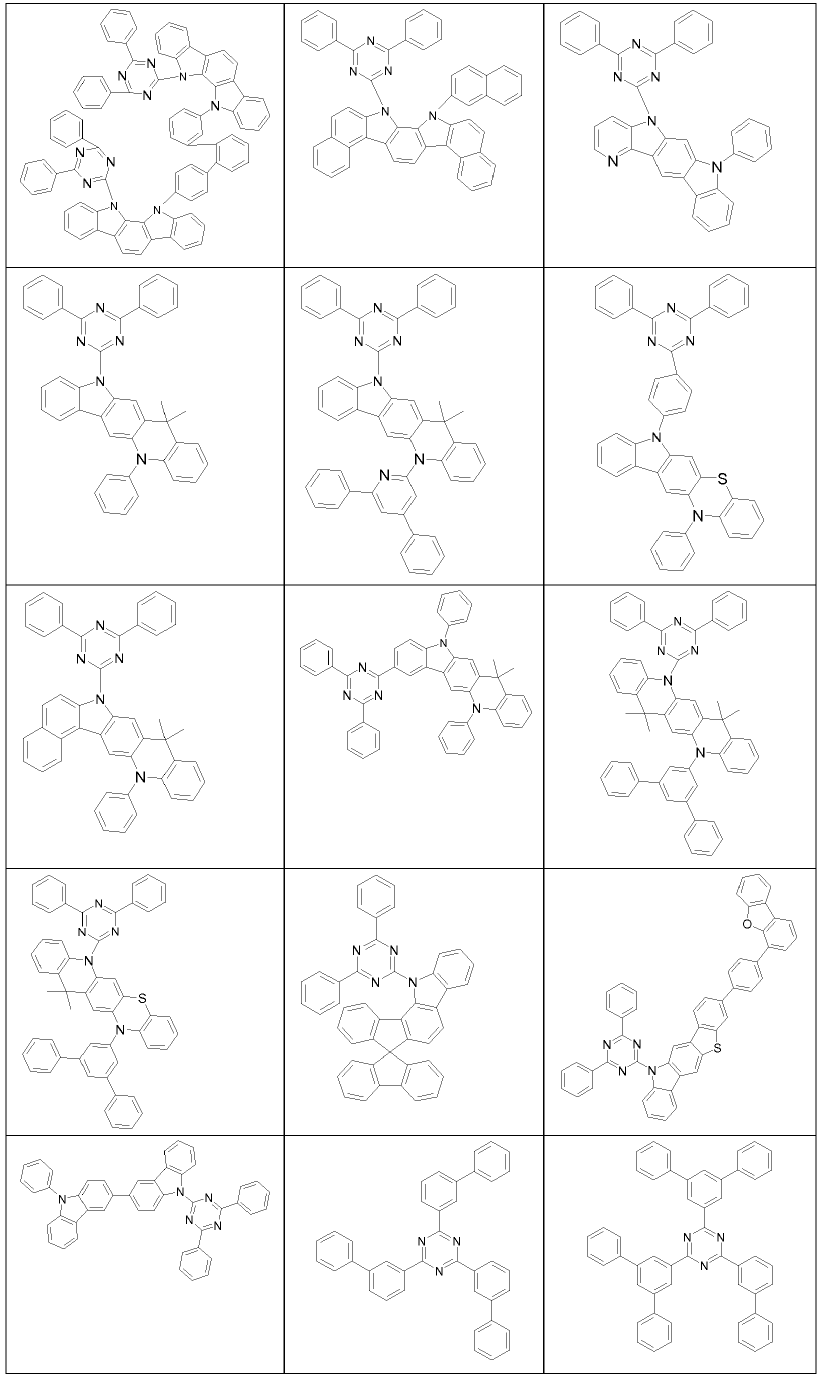

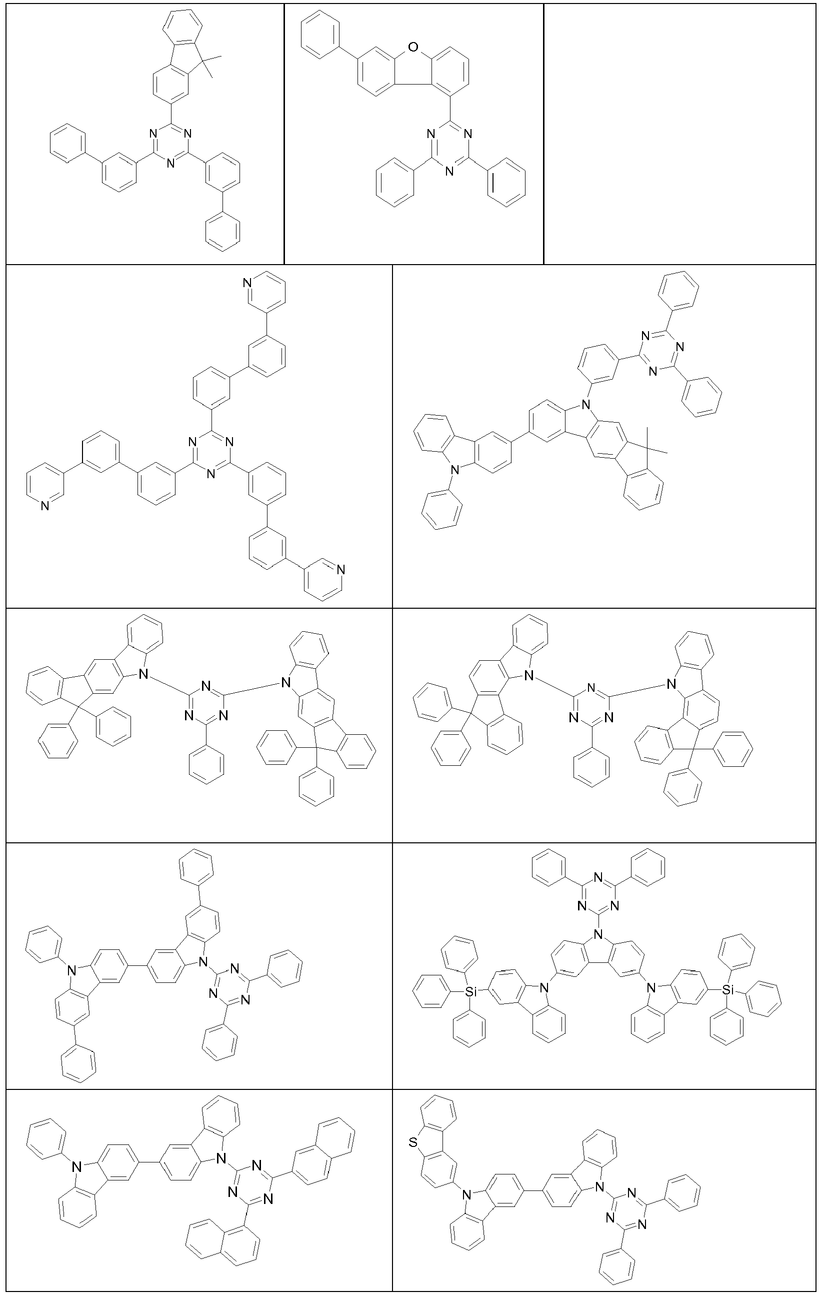

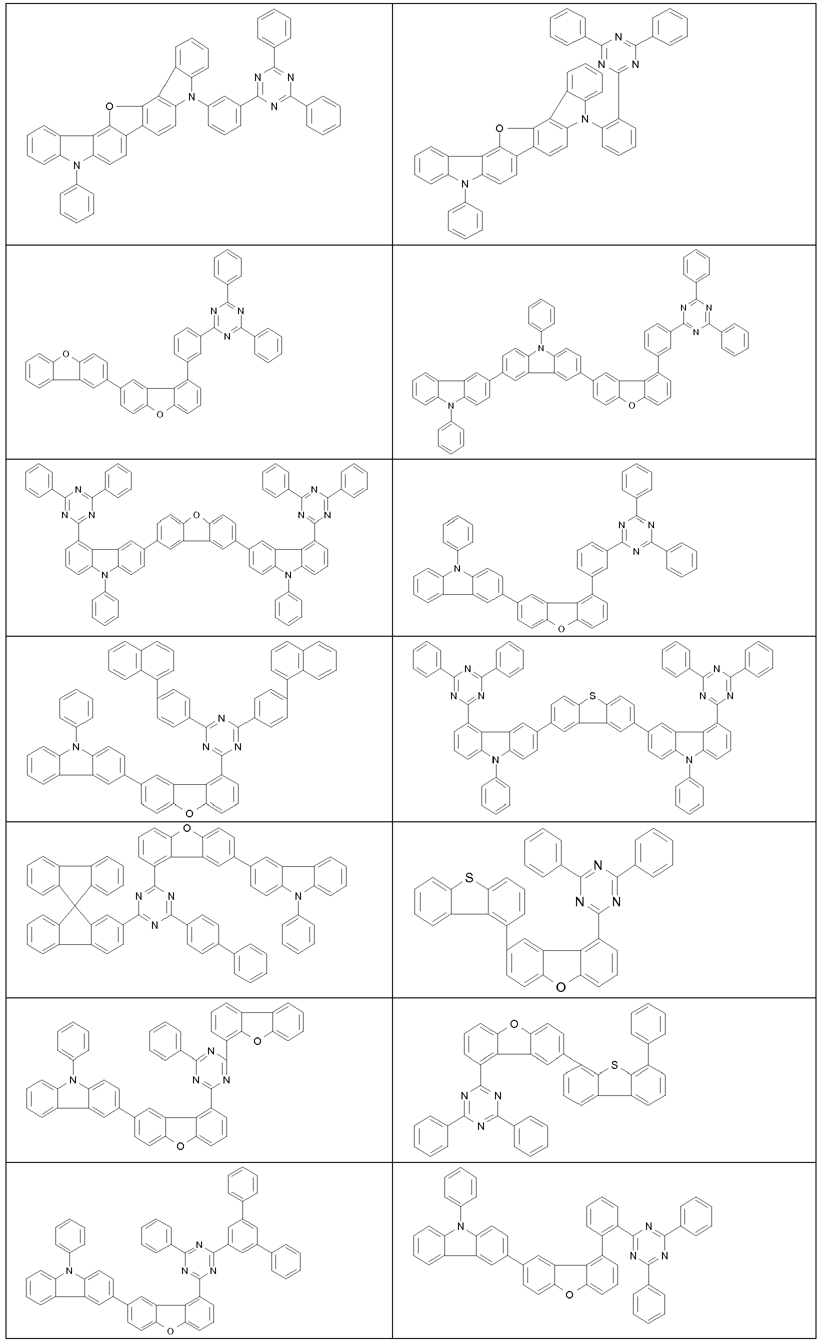

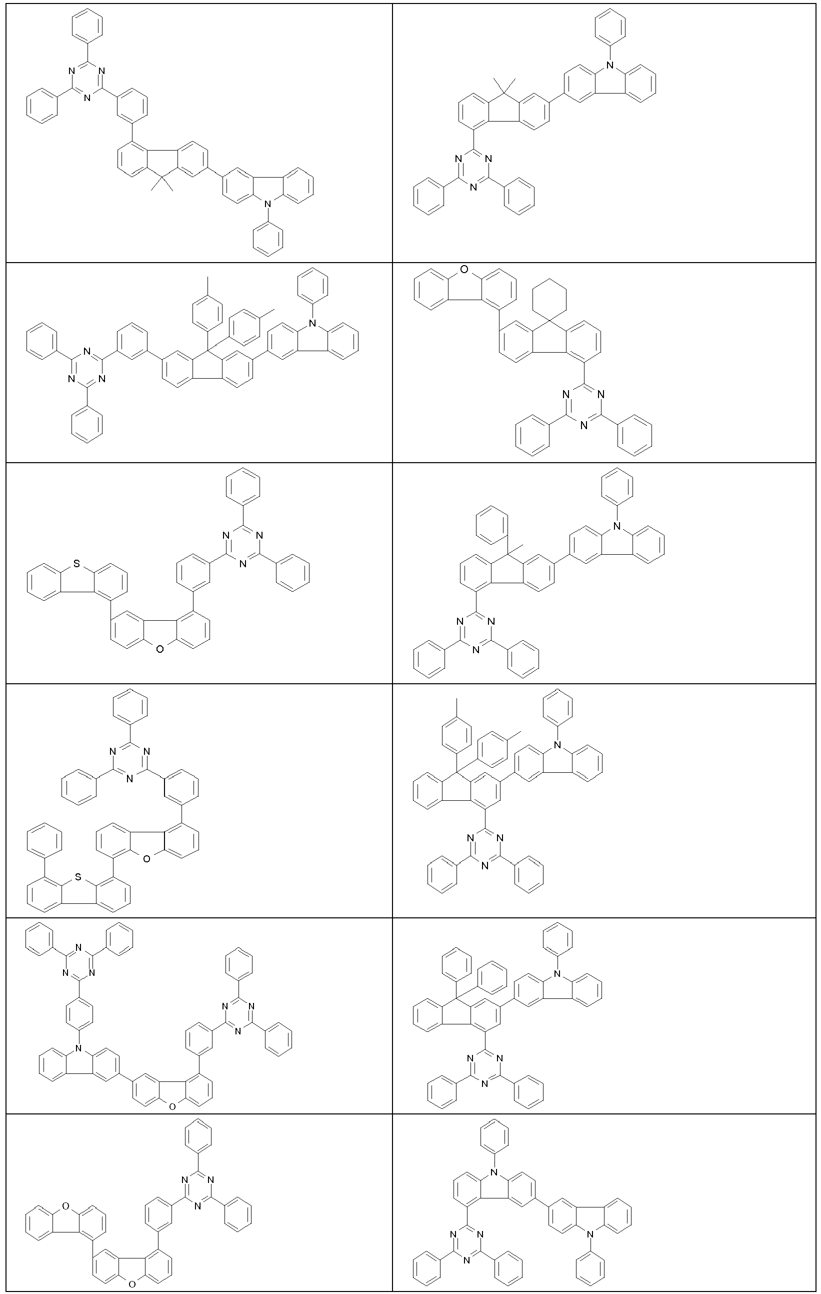

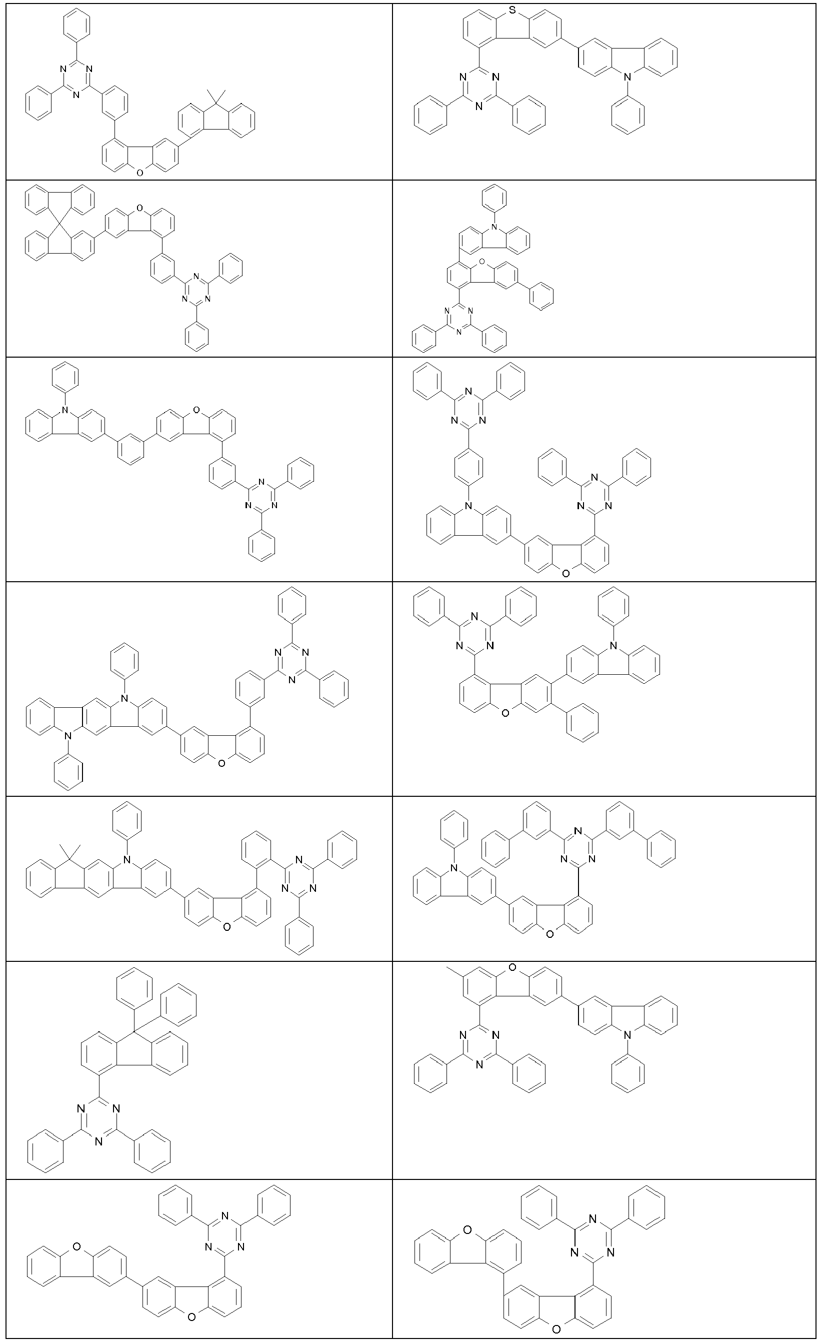

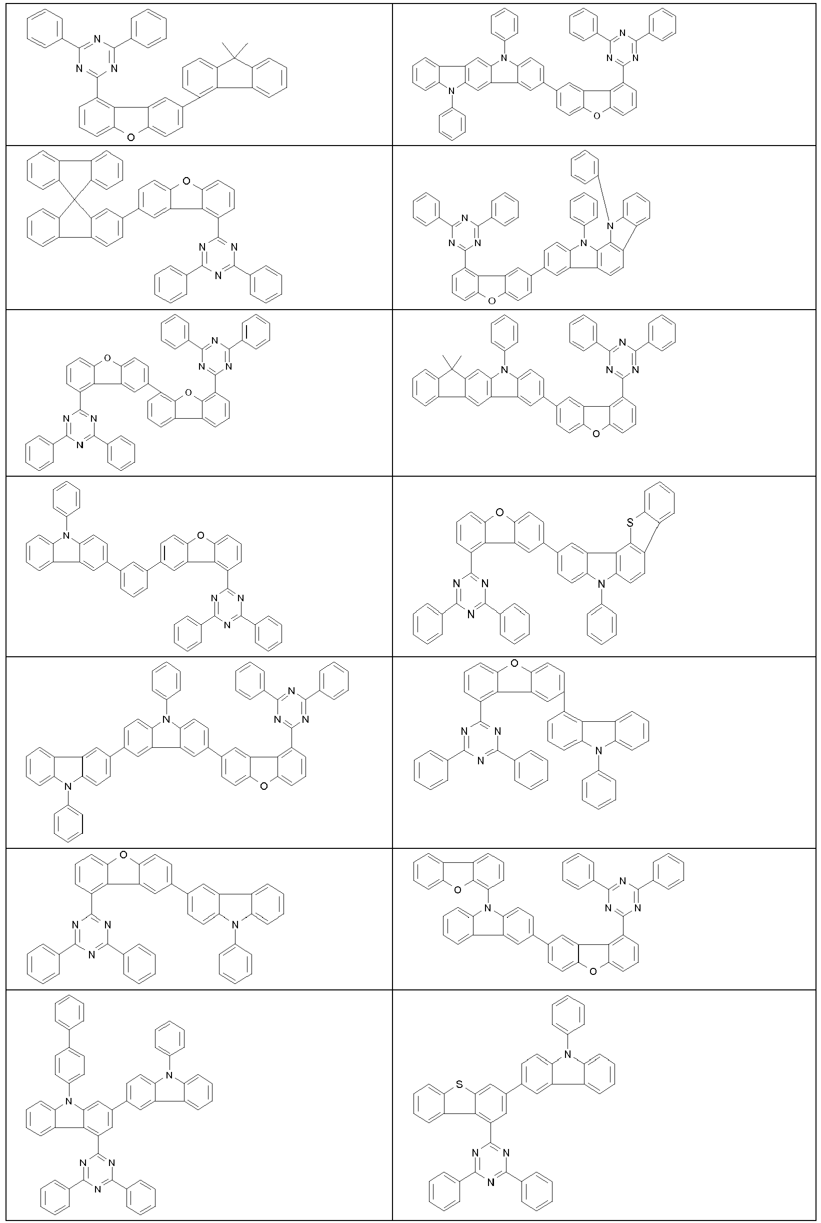

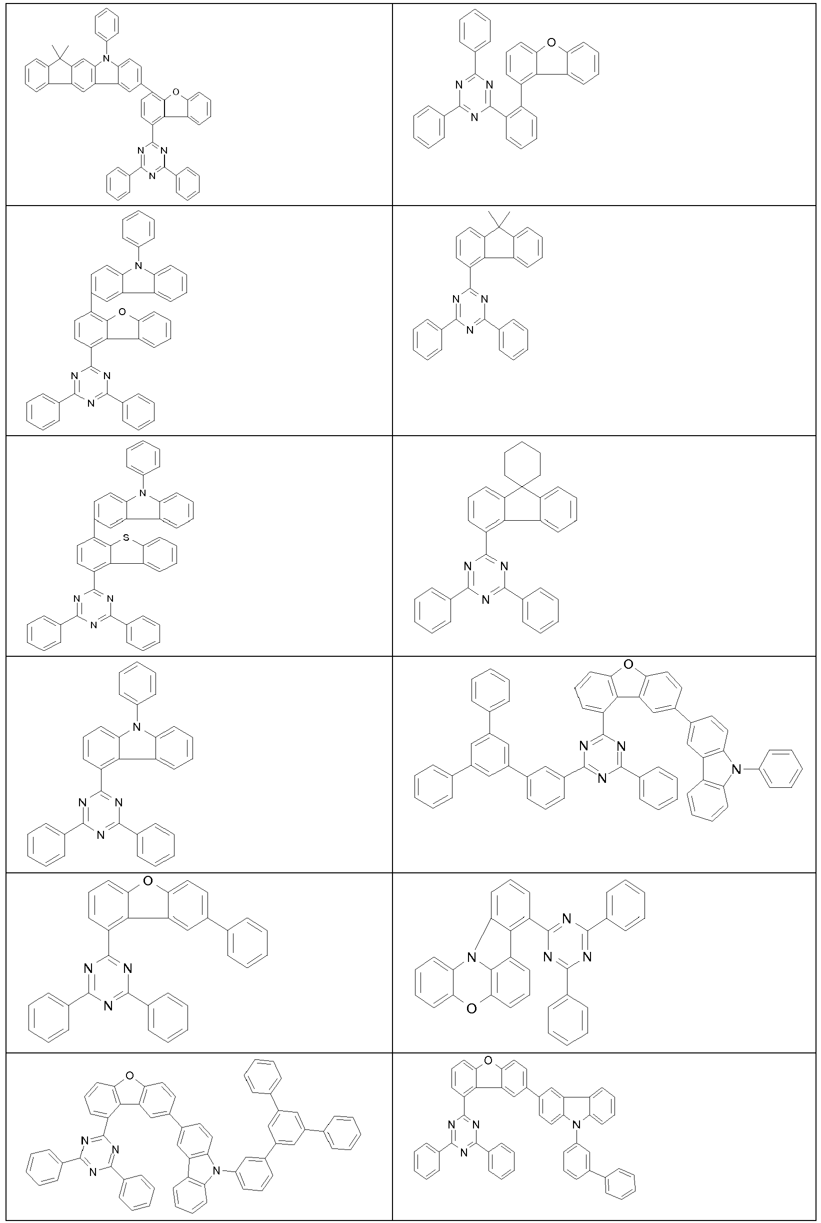

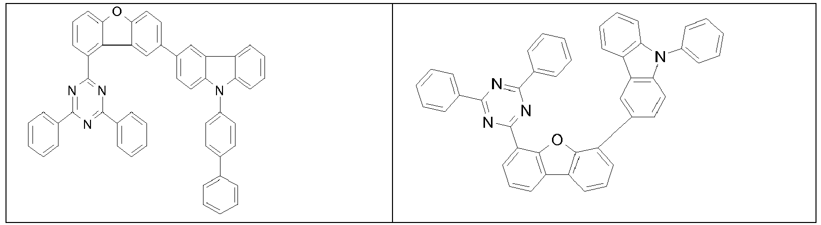

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als PhosphoreszenzemitterVerbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186 und WO 2018/041769, WO 2019/020538, WO 2018/178001, WO 2019/115423 sowie der noch nicht offen gelegten Patentanmeldung EP 18156388.3 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind nachfolgend aufgeführt.

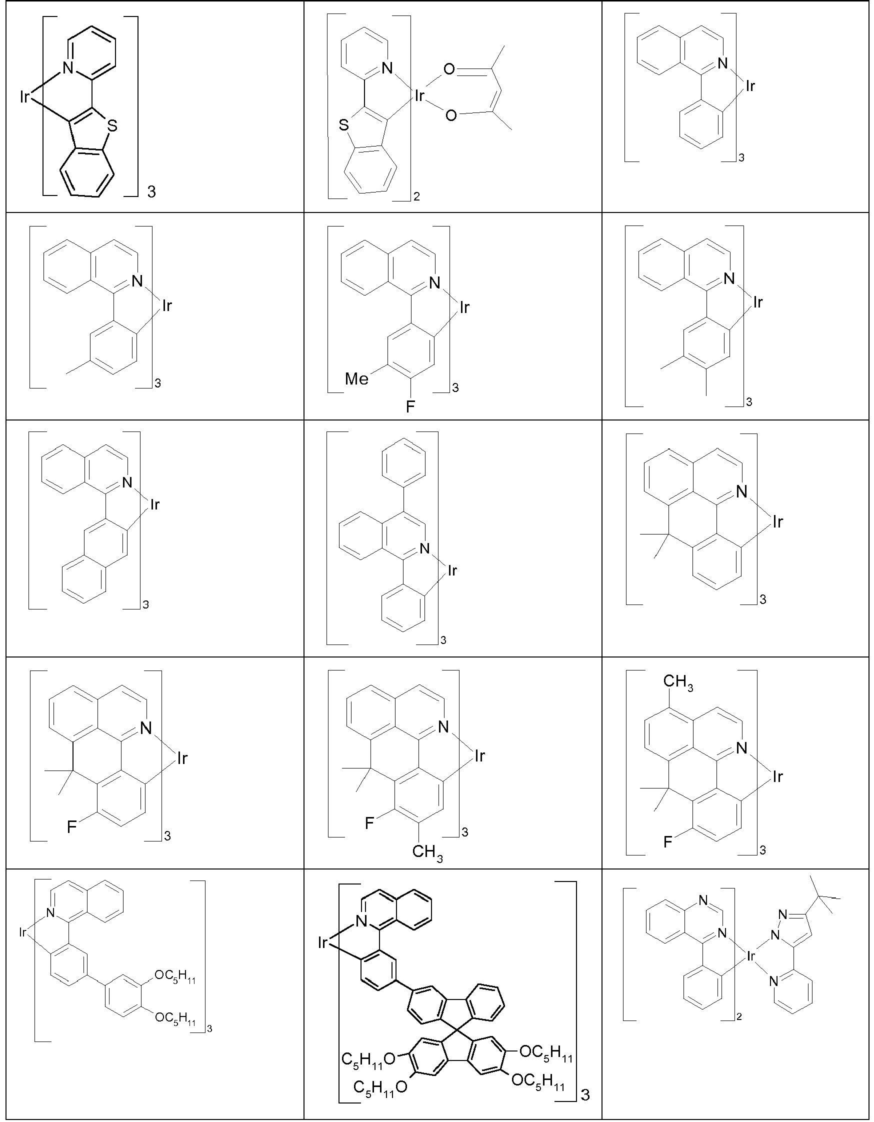

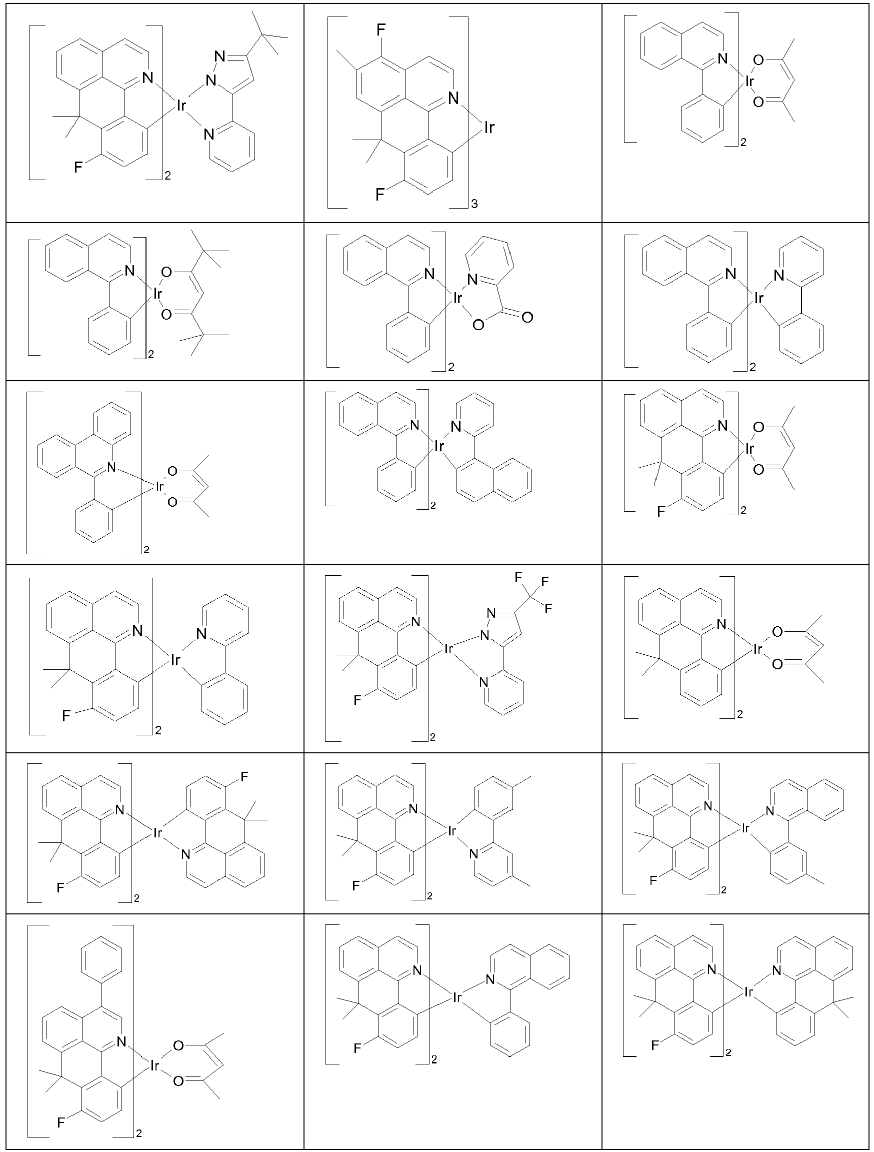

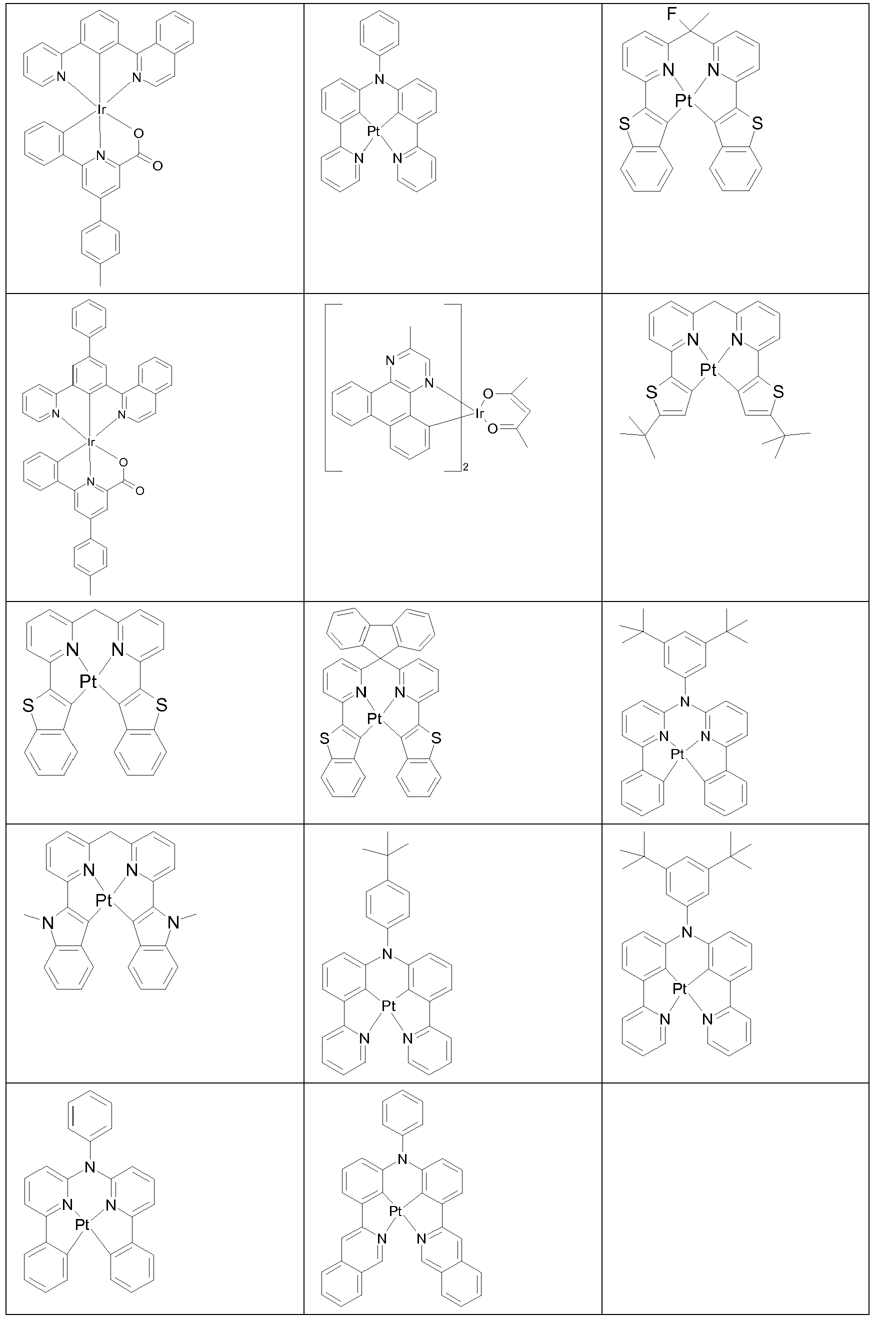

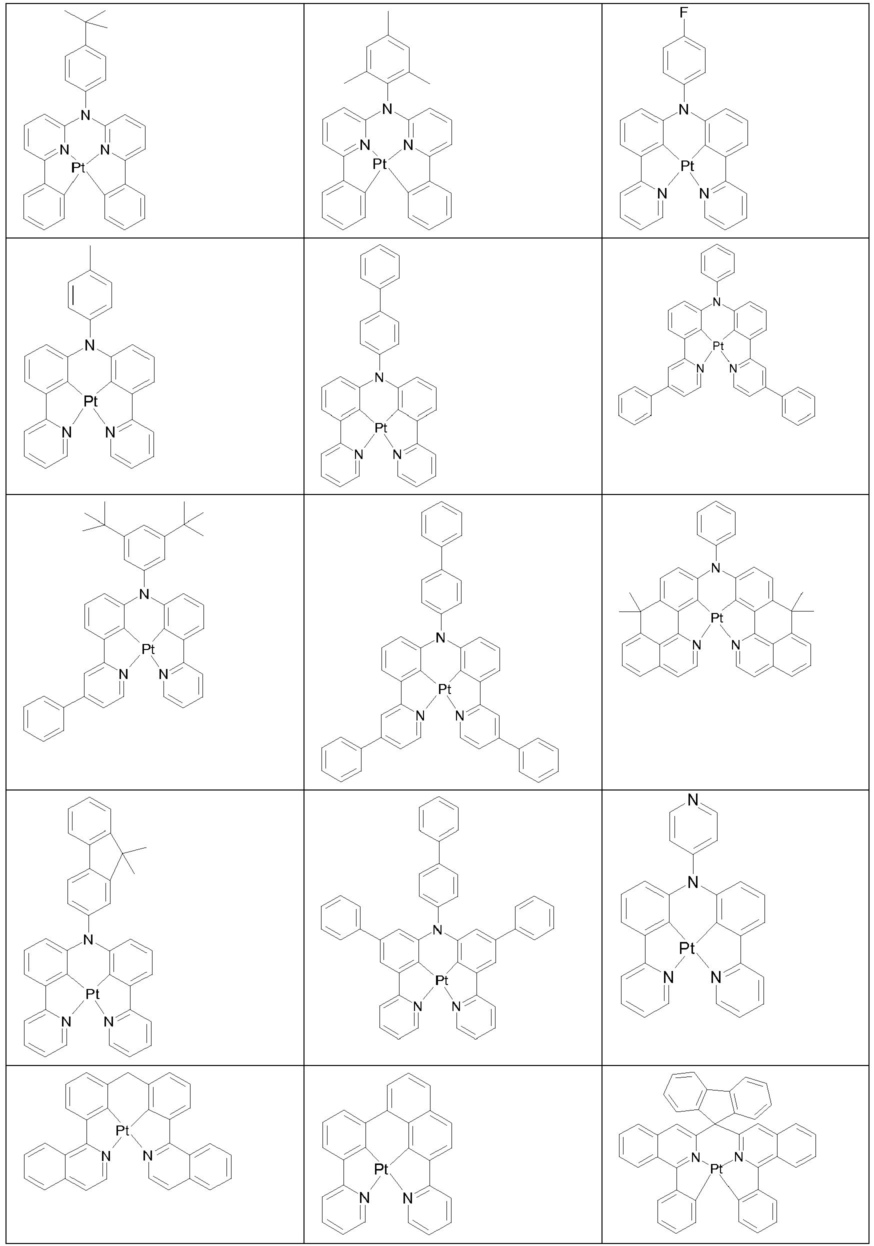

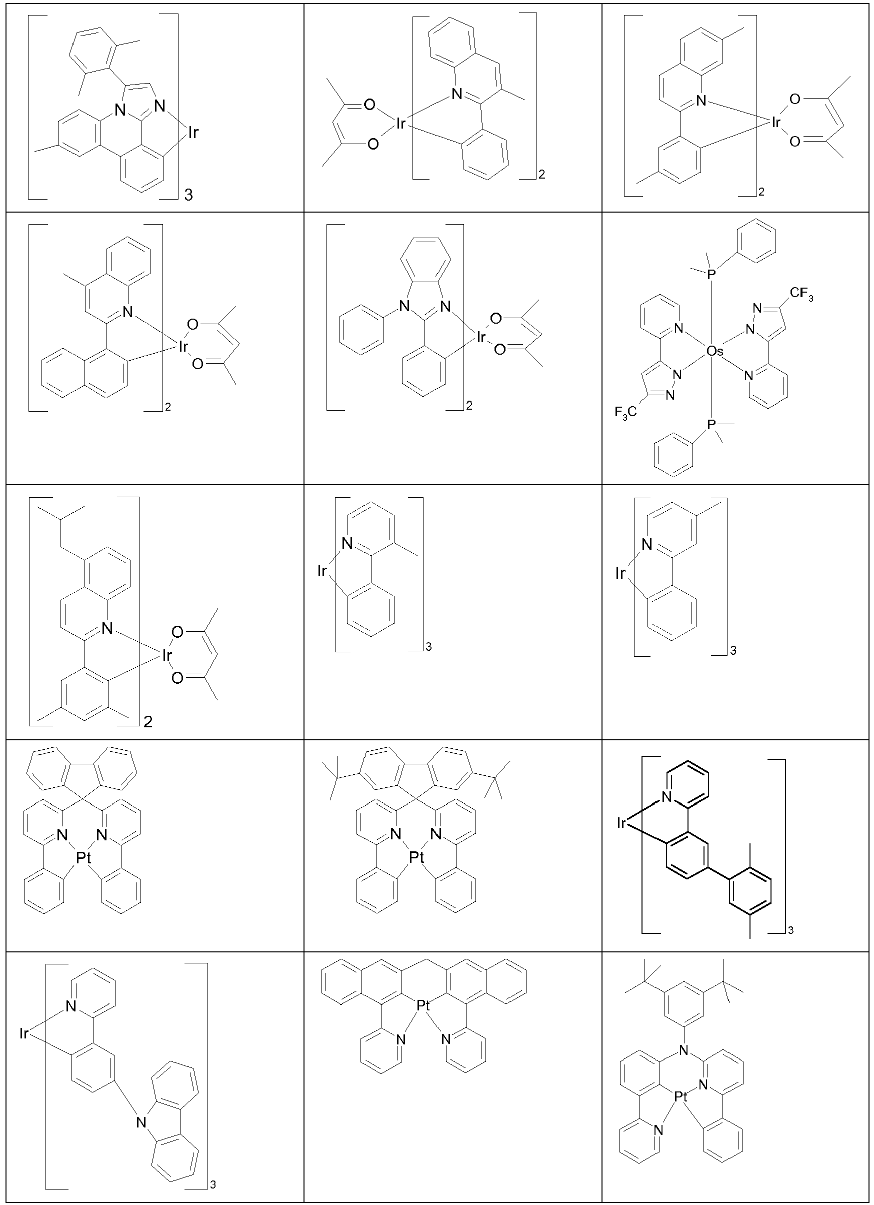

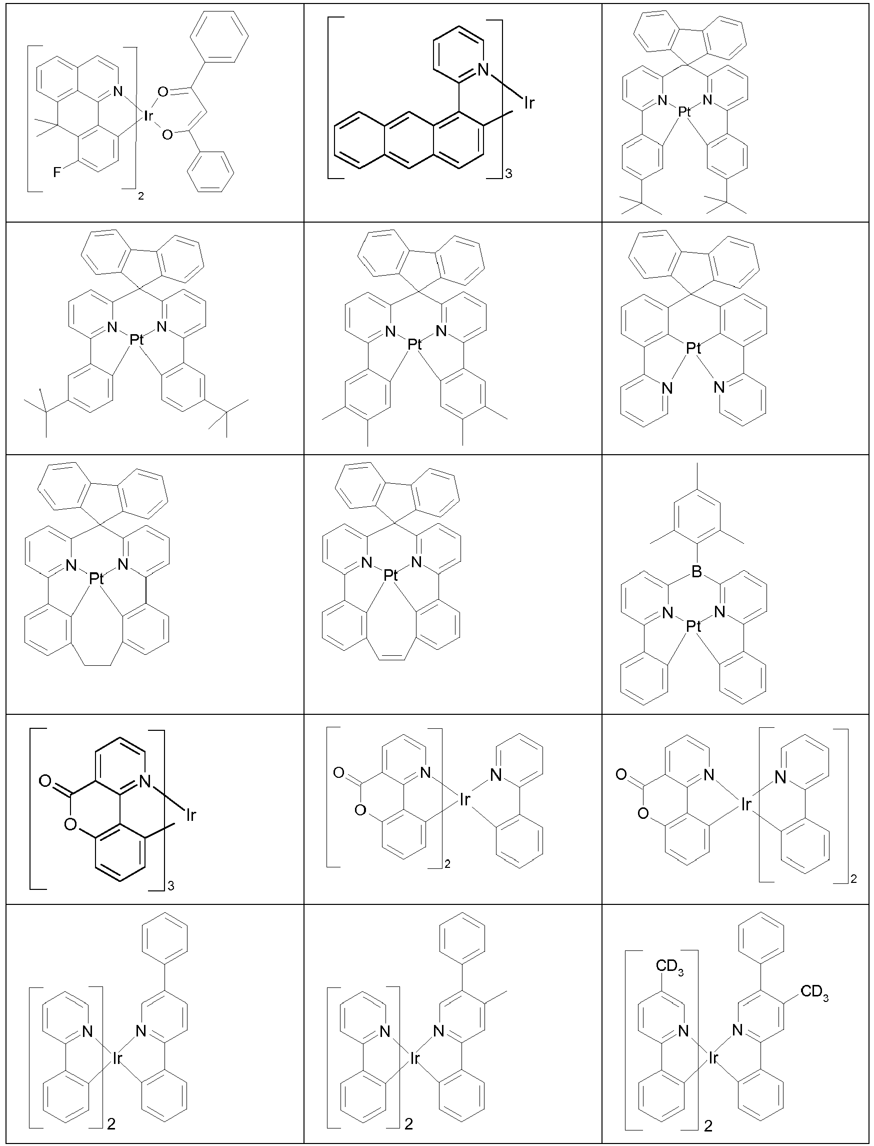

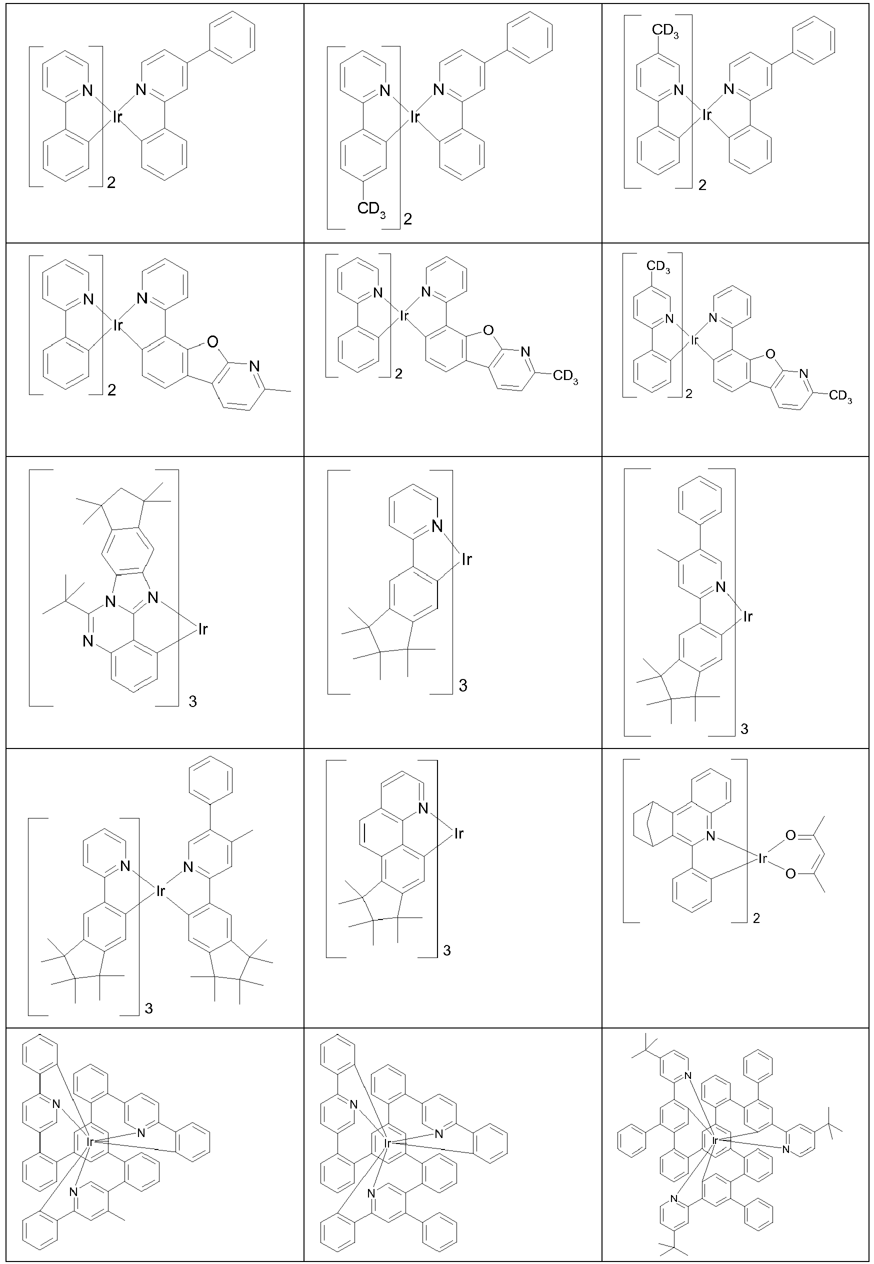

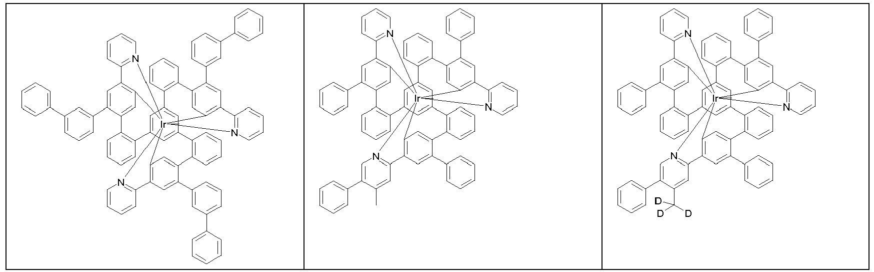

Die erfindungsgemäßen Verbindungen sind auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) oder (2) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für phosphoreszierende Emitter, führen zu langen Lebensdauern.
2. Die erfindungsgemäßen Verbindungen führen zu hohen Effizienzen. Dies gilt insbesondere, wenn die Verbindungen als Matrixmaterial für einen phosphoreszierenden Emitter eingesetzt werden.
3. Die erfindungsgemäßen Verbindungen führen zu geringen Betriebsspannungen. Dies gilt insbesondere, wenn die Verbindungen als Matrixmaterial für einen phosphoreszierenden Emitter eingesetzt werden.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

### Synthesebeispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern.

### a) 6-Chlor-5H-benzo[c][2,1]benzazaphosphinin

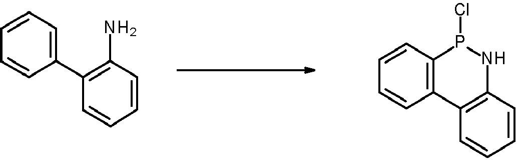

8 g (50 mmol) 2-Aminobiphenyl werden in 70 ml Phosphortrichlorid 8 h unter Rückfluss gekocht, anschließend wird das restliche PCl₃ im Vakuum abdestilliert und der Rückstand (Ar-NH-PCl₂) mit 0.5 g AlCl₃ für 6 h auf 180-220 °C erhitzt. Die Mischung wird in Toluol gelöst und über Glaswolle filtriert und somit von Salzen abgetrennt. Die Aufreinigung erfolgt durch Sublimation bei 180-190 °C (0.05 mm) als weiße Nadeln, welche sehr hydrolyseempfindlich sind. Ausbeute: 4.2 g (24 mmol); 42% d. Th.; Reinheit: 97 % n. NMR

Analog können die folgenden Verbindungen hergestellt werden:

| **Bsp.** | **Edukt** | **Produkt** | **Ausbeute** |
|---|---|---|---|
| 1a | 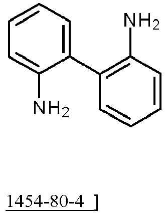 | 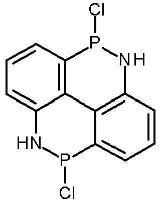 | **30%** |
| 2a | 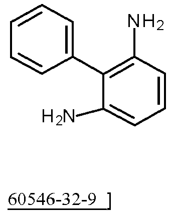 | 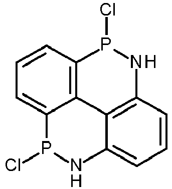 | **34%** |
| 3a | 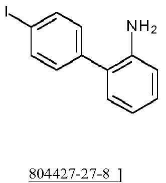 | 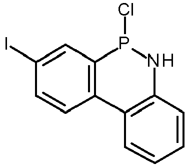 | **47%** |
| 4a | 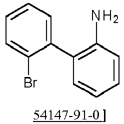 | 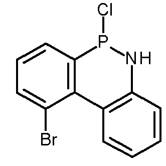 | **41%** |
| 5a | 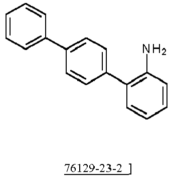 | 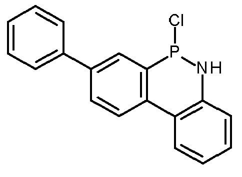 | **45%** |
| 6a | 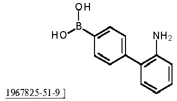 | 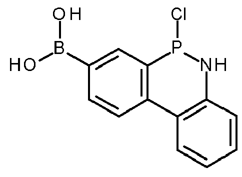 | **38%** |
| 7a | 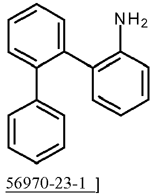 | 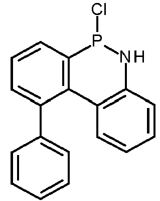 | **42%** |
| 8a | 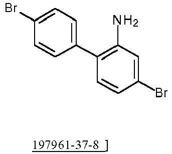 | 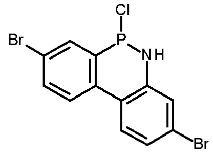 | **36%** |
| 9a | 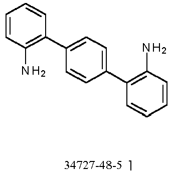 | 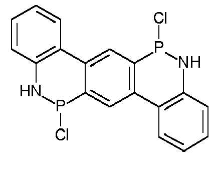 | **32%** |
| 10a | 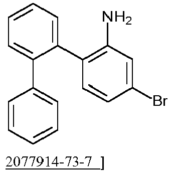 | 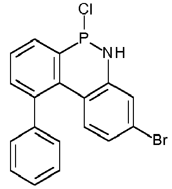 | **41%** |
| 11a | 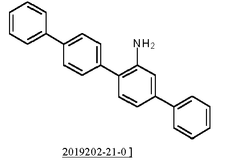 | 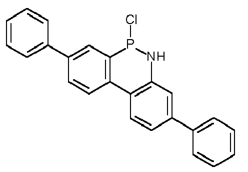 | **48%** |

### b) 6-Phenyl-5H-benzo[c][2,1]benzazaphosphinin

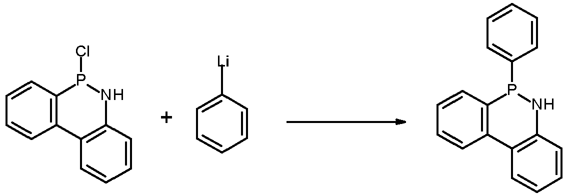

Unter Schutzgas werden 4.2 g (24 mmol) 6-Chlor-5H-benzo[c][2,1]benzazaphosphinin in 120 ml trockenem Toluol gelöst. Diese Lösung wird innerhalb von 15 min. zu einer Phenyllithium-Lösung, synthetisiert aus 16.2 g (103 mmol) Brombenzol und 1.5 g Lithium in 100 ml Ether, zugegeben und 1 h unter Rückfluss gekocht. Nach dem Abkühlen wird die Mischung auf Eiswasser gegeben und die organische Phase abgetrennt. Die organische Phase wird im Vakuum eingeengt, und das Produkt aus Toluol umkristallisiert. Ausbeute: 3 g (11 mmol); 61% d. Th.; Reinheit: 98 % n. NMR.

Analog können folgende Verbindungen hergestellt werden:

| **Bsp.** | **Edukt 1** | **Edukt 2** | **Produkt** | **Ausbeute** |
|---|---|---|---|---|
| 1b | 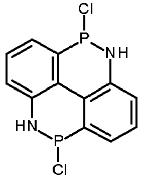 | 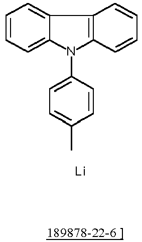 | 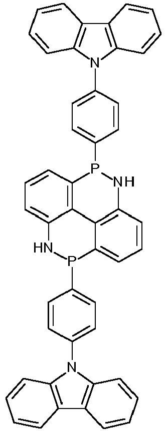 | 64% |
| 2b | 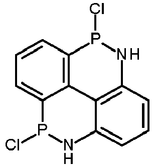 | 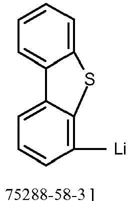 | 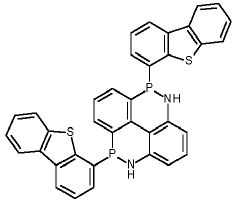 | 61% |
| 3b | 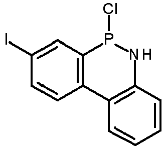 | 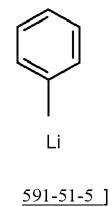 | 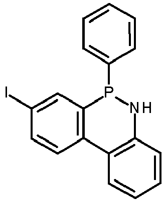 | 56% |
| 4b | 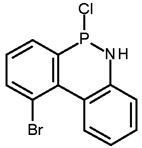 | 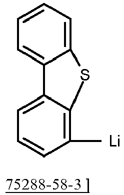 | 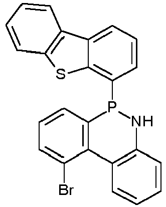 | 50% |
| 5b | 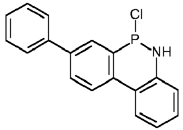 | 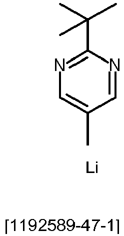 | 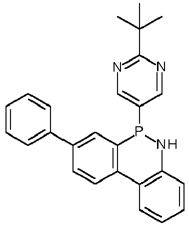 | 62% |
| 6b | 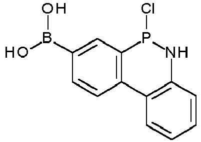 | 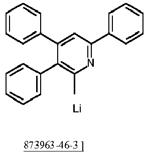 | 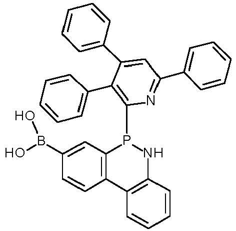 | 47% |
| 7b | 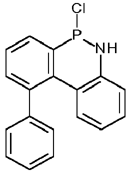 | 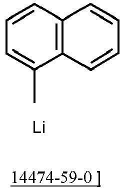 | 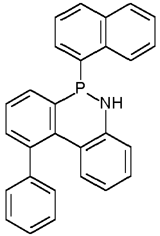 | **63%** |
| 8b | 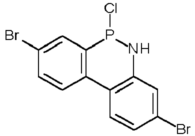 | 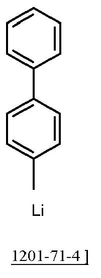 | 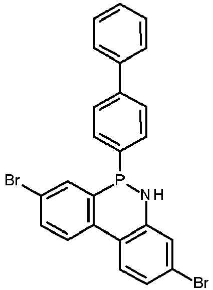 | **59%** |
| 9b | 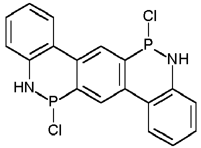 | 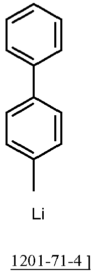 | 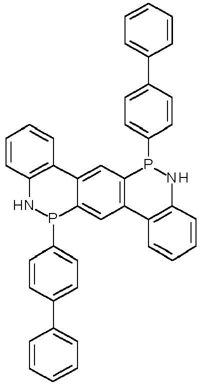 | **66%** |
| 10b | 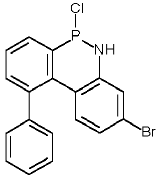 | 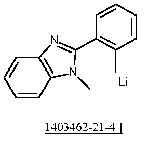 | 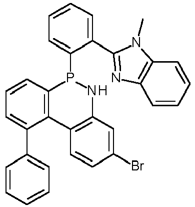 | **48%** |
| 11b | 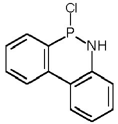 | 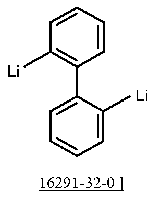 | 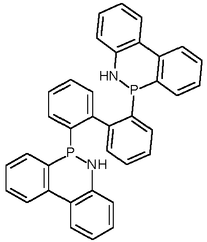 | **62%** |

### c) 5,6-Diphenylbenzo[c][2,1]benzazaphosphinin

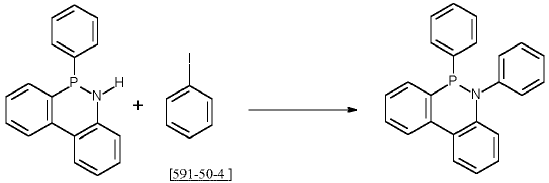

6.8 g (25 mmol, 1.00 eq.) 6-Phenyl-5H-benzo[c][2,1]benzazaphosphinin, 21.3 ml (128 mmol, 5.2 eq.) lodbenzol und 7.20 g Kaliumcarbonat (52.1 mmol, 2.10 eq.) werden in 220 ml trockenem DMF vorgelegt und mit Argon inertisiert. Anschließend werden 0.62 g (2.7 mmol, 0.11 eq.) 1,3-Di(2-pyridyl)-1,3-propandion und 0.52 g (2.7 mmol, 0.11eq) Kupfer(I)-iodid zugegeben und das Gemisch für drei Tage bei 140 °C erhitzt. Nach beendeter Reaktion wird der Ansatz vorsichtig am Rotationsverdampfer eingeengt, der ausgefallene Feststoff abgesaugt und mit Wasser und Ethanol gewaschen. Das Rohprodukt wird zweimal mittels Heißextraktion (Toluol/Heptan 1:1) aufgereinigt und der erhaltene Feststoff aus Toluol umkristallisiert. Ausbeute: 4.5 g (12.8 mmol); 52 % d. Th..

Analog können folgende Verbindungen hergestellt werden:

| **Bsp.** | **Edukt 1** | **Edukt 2** | **Produkt** | **Ausbeute** |
|---|---|---|---|---|
| **1c** | 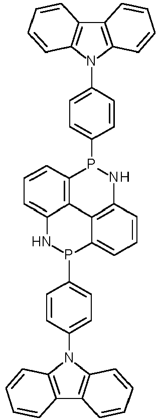 | 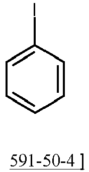 | 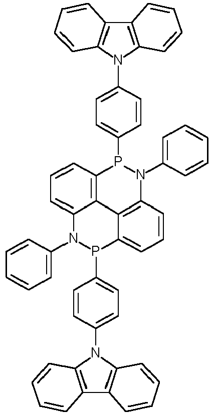 | **55%** |
| **2c** | 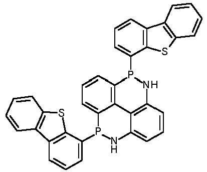 | 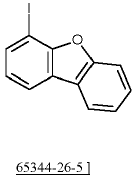 | 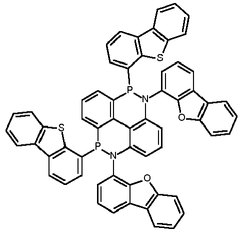 | **51%** |
| **3c** | 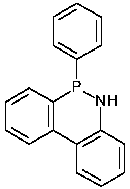 | 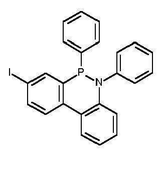 | 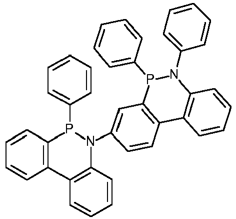 | **60%** |
| **4c** | 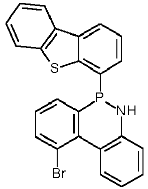 | 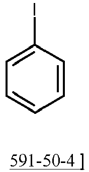 | 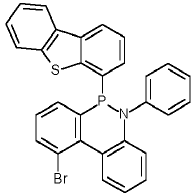 | **46%** |
| **5c** | 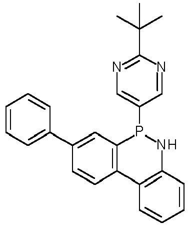 | 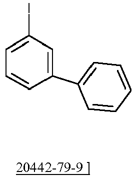 | 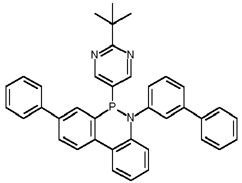 | **50%** |
| **6c** | 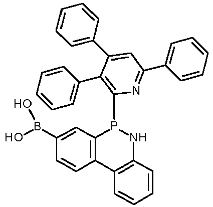 | 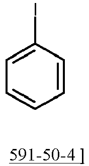 | 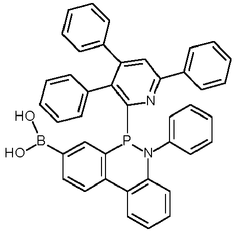 | **34%** |
| **7c** | 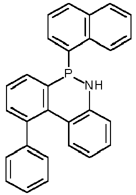 | 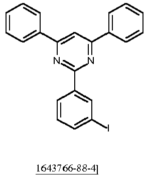 | 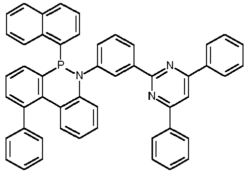 | **49%** |
| **8c** | 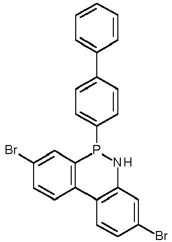 | 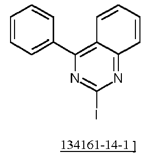 | 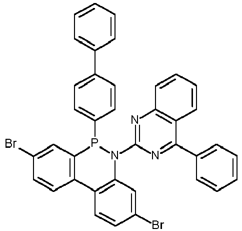 | **51%** |
| **9c** | 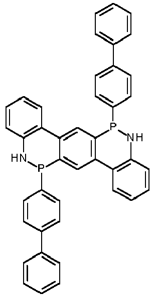 | 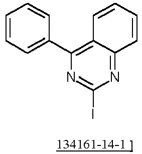 | 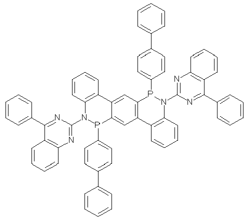 | **50%** |
| 10c | 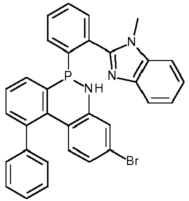 | 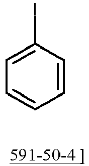 | 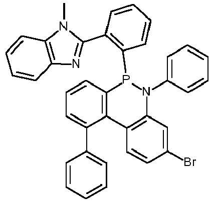 | **47%** |
| **11c** | 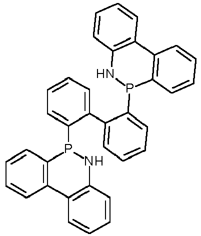 | 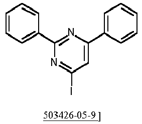 | 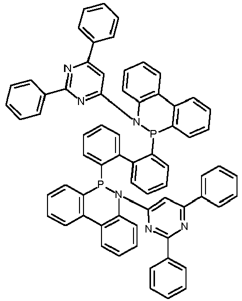 | **44%** |
| **12c** | 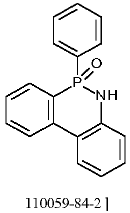 | 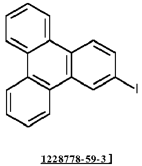 | 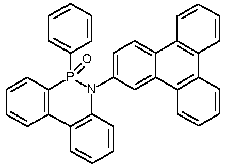 | **56%** |
| **13c** | 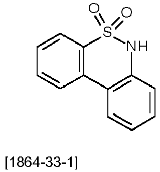 | 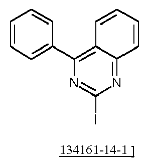 | 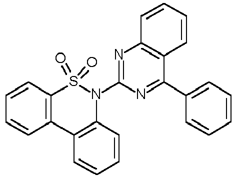 | **55%** |
| **14c** | 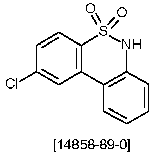 | 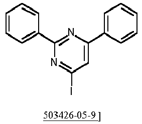 | 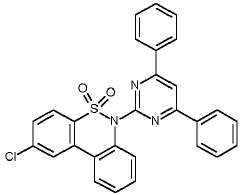 | **61%** |
| **15c** | 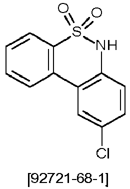 | 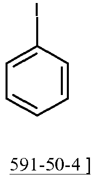 | 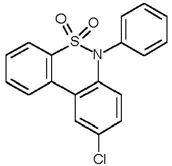 | **60%** |
| **16c** | 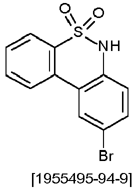 | 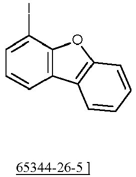 | 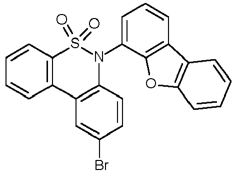 | **57%** |
| **17c** | 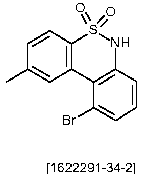 | 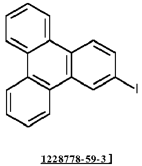 | 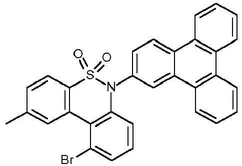 | **53%** |
| **18c** | 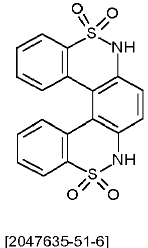 | 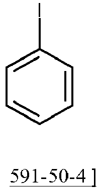 | 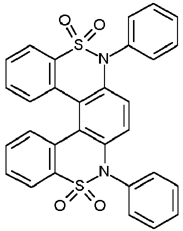 | **54%** |
| **19c** | 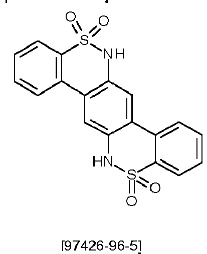 | 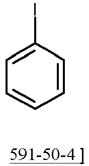 | 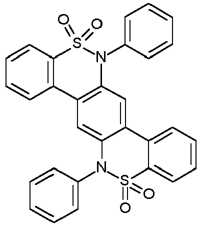 | **50%** |
| **20c** | 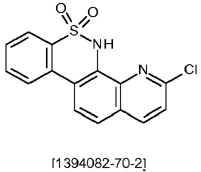 | 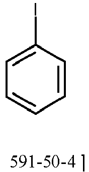 | 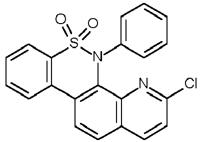 | **47%** |
| **21c** | 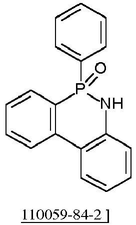 | 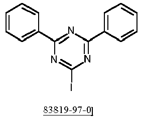 | 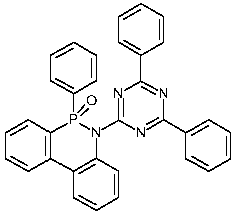 | **50%** |
| **22c** | 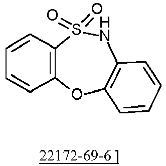 | 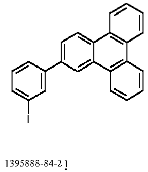 | 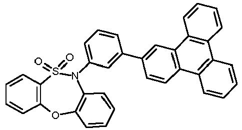 | **45%** |
| **23c** | 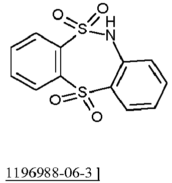 | 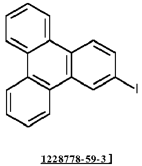 | 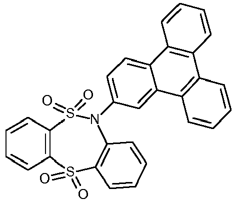 | **48%** |
| **24c** | 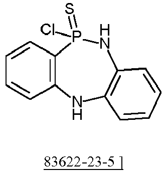 | 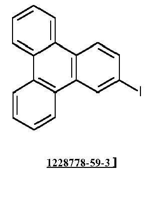 | 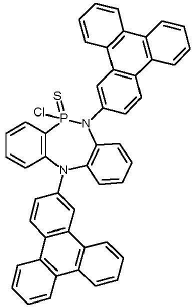 | **42%** |
| **25c** | 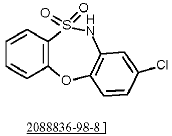 | 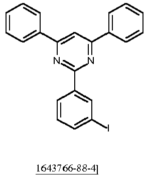 | 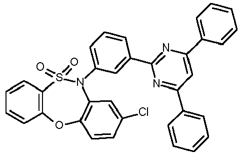 | **50%** |

Die Verbindungen **19c, 20c, und 22c-24c** werden durch Sublimation auf eine Reinheit von 99.9 % aufgereinigt.

### d) 5 ,6-Diphenylbenzo[c][2,1]benzazaphosphinin-6-oxid

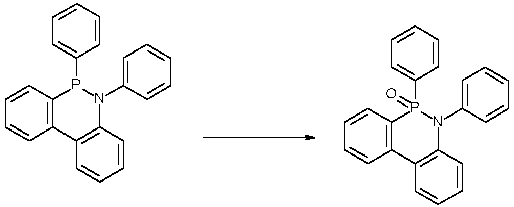

4.5 g (12.8 mmol) werden bei Raumtemperatur in 40 ml Ethanol gelöst und innerhalb von 30 min. tropfenweise mit 80 ml H₂O₂(30%) versetzt. Nach 1 h Rühren bei 70 °C kühlt man ab, versetzt die Lösung mit 100 ml Dichlormethan, trennt die Phasen, engt die organische Phase ein und kristallisiert aus Heptan/ Dichlormethan 2:1 aus. Ausbeute: 3.9 g (10.5 mmol); 93 % d. Th..

| **Bsp.** | **Edukt** | **Produkt** | **Ausbeute** |
|---|---|---|---|
| **1d** | 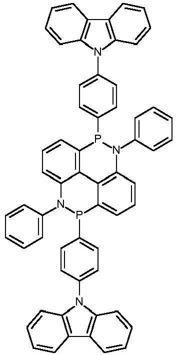 | 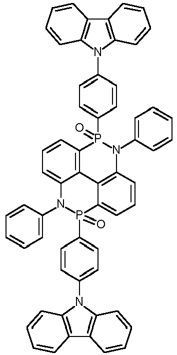 | **94%** |
| **2d** | 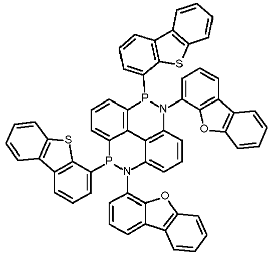 | 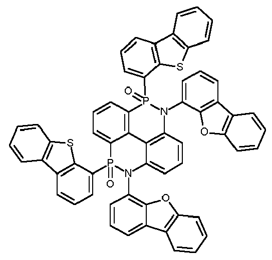 | **91%** |
| **3d** | 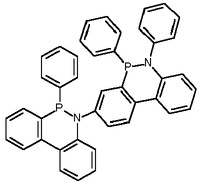 | 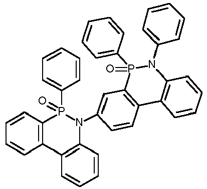 | **94%** |
| **4d** | 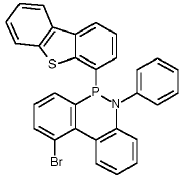 | 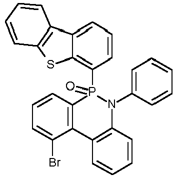 | **90%** |
| **5d** | 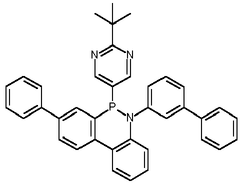 | 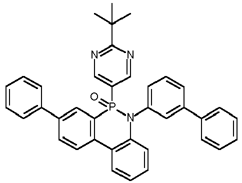 | **89%** |
| **6d** | 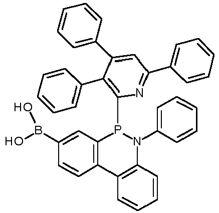 | 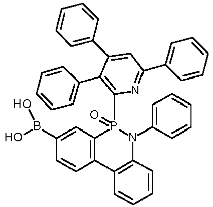 | **74%** |
| **7d** | 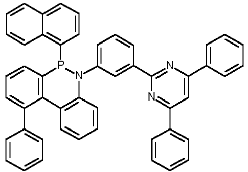 | 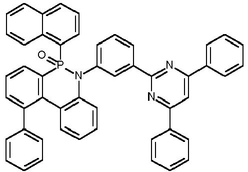 | **90%** |
| **8d** | 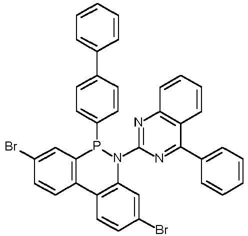 | 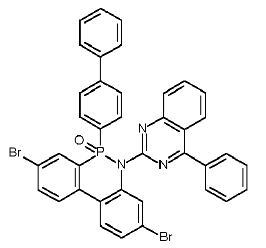 | **89%** |
| **9d** | 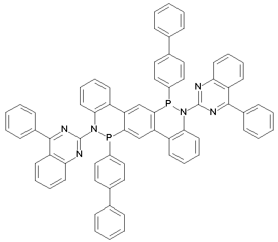 | 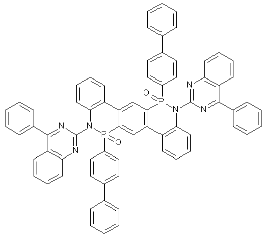 | **92%** |
| **10c** | 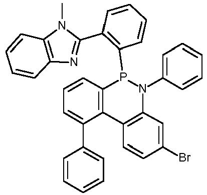 | 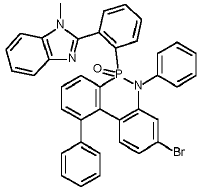 | **93%** |
| **11d** | 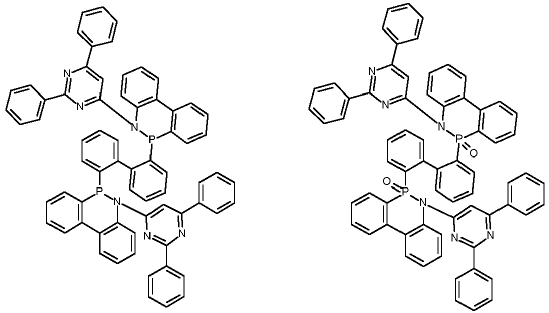 | 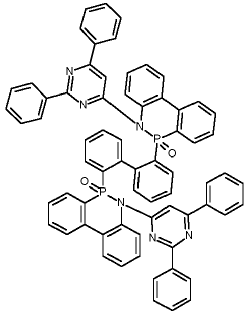 | **91%** |

Die Verbindungen **1d-3d, 5d, 7d, 9d** und **10d** werden aus Toluol umkristallisiert und abschließend zweimal fraktioniert sublimiert (p ca. 10⁻⁶ mbar, T = 330-450 °C).

### e) 2-Brom-5,6-diphenyl-benzo[c][1,2]benzazaphosphinin-6-oxid

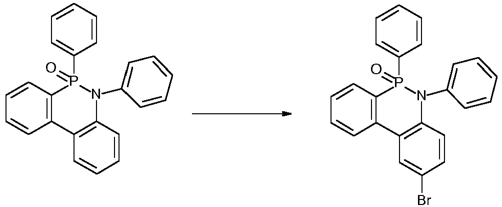

Eine Lösung von 56 g (154 mmol) 5,6-Diphenylbenzo[c][2,1]benzazaphosphinin-6-oxid in Chloroform (1000 mL) wird bei 0 °C unter Lichtausschluss portionsweise mit *N*-Bromsuccinimid (24.7 g, 139 mmol) versetzt und 2 h bei dieser Temperatur gerührt. Die Reaktion wird durch Zugabe von Natriumsulfit-Lösung beendet und weitere 30 min. bei Raumtemperatur gerührt. Nach Phasentrennung wird die organische Phase mit Wasser gewaschen und die wässrige Phase mit Dichlormethan extrahiert. Die vereinigten organischen Phasen werden über Natriumsulfat getrocknet und im Vakuum eingeengt. Der Rückstand wird in Toluol gelöst und über Kieselgel filtriert. Anschließend wird das Rohprodukt aus Toluol/Heptan umkristallisiert. Ausbeute: 44 g (99 mmol); 65 % d. Th. eines farblosen Feststoffes.

Analog können folgende Verbindungen hergestellt werden:

| | **Edukt** | **Produkt** | **Ausbeute** |
|---|---|---|---|
| 1e | 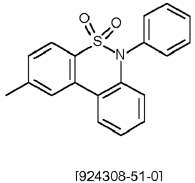 | 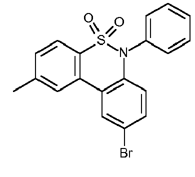 | 67% |
| 2e | 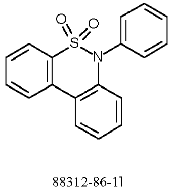 | 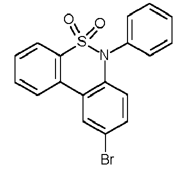 | 68% |
| 3e | 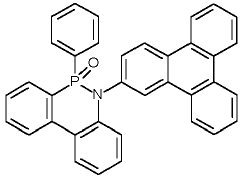 | 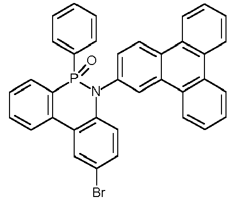 | 80% |
| 4e | 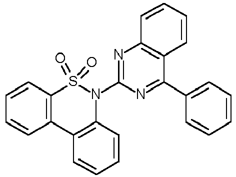 | 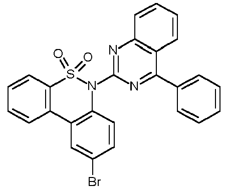 | 79% |
| 5e | 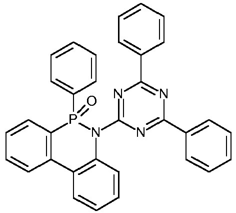 | 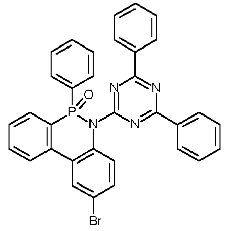 | 77% |

### f) 5,6-Diphenyl-2-(9-phenylcarbazol-3-yl)benzo[c][1,2]benzazaphosphinin-6-oxid

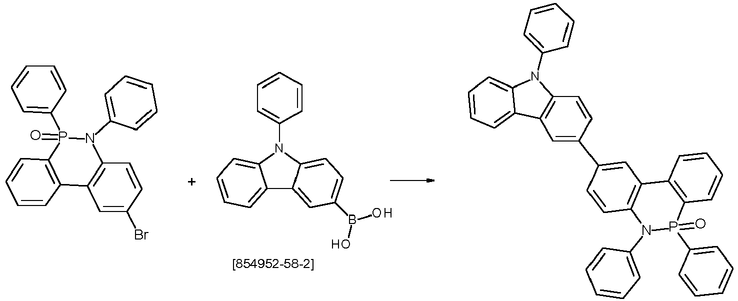

31 (70 mmol) 2-Brom-5,6-diphenyl-benzo[c][1,2]benzazaphosphinin-6-oxid, 20.8 g (75 mmol) Phenylcarbazol-3-boronsäure und 14.7 g (139 mmol) Natriumcarbonat werden in 200 mL Toulol, 52 mL Ethanol und 100 mL Wasser suspendiert. Zu dieser Suspension werden 80 mg (0.69 mmol) Tetrakistriphenylphosphin-palladium(0) gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Heptan / Dichlormethan umkristallisiert. Die Ausbeute beträgt 33 g (55 mmol); 79 % d. Th..

Analog können die folgenden Verbindungen erhalten werden:

| | **Edukt 1** | **Edukt 2** | **Produkt** | **Ausbeute** |
|---|---|---|---|---|
| 1f | 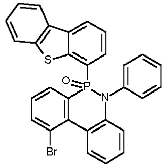 | 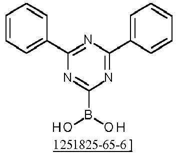 | 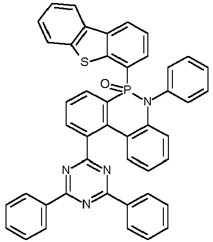 | 75% |
| 2f | 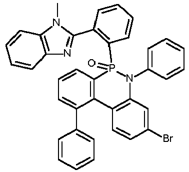 | 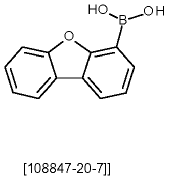 | 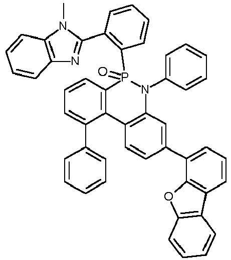 | 64% |
| 3f | 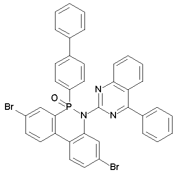 | 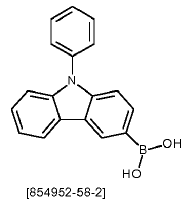 | 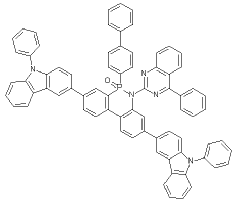 | 76% |
| 4f | 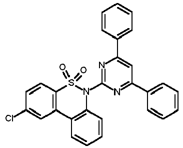 | 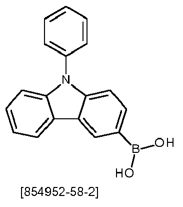 | 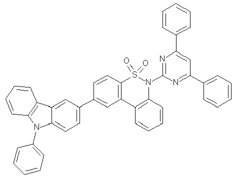 | 81% |
| 5f | 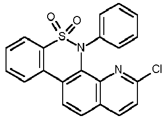 | 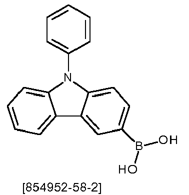 | 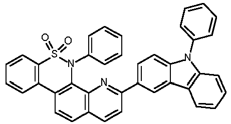 | 63% |
| 6f | 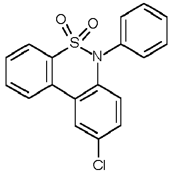 | 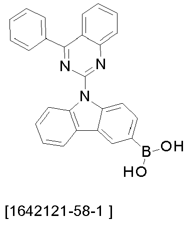 | 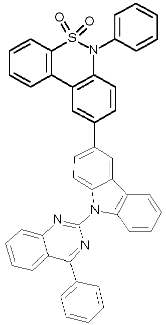 | 76% |
| 7f | 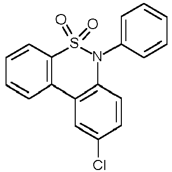 | 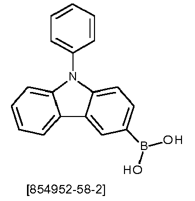 | 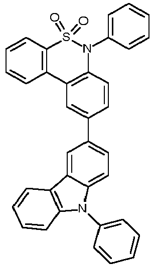 | 80% |
| 8f | 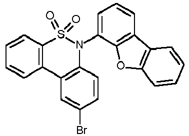 | 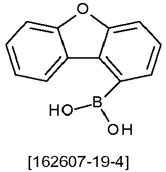 | 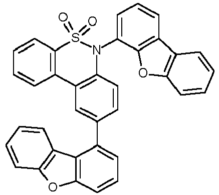 | 74% |
| 9f | 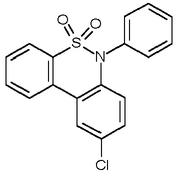 | 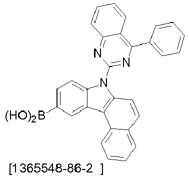 | 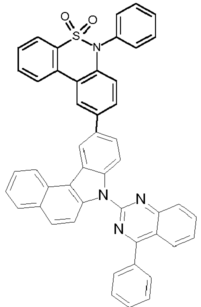 | 77% |
| 10f | 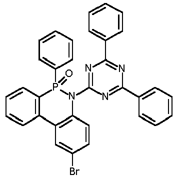 | 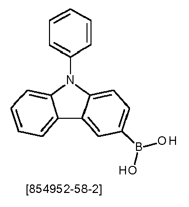 | 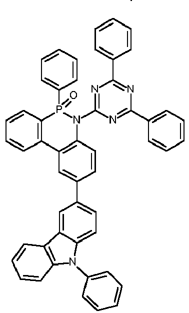 | 54% |
| 11f | 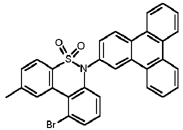 | 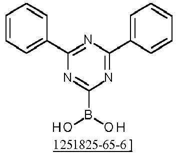 | 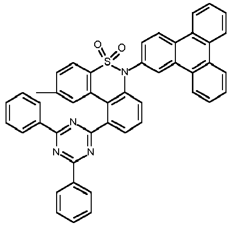 | 76% |
| 12f | 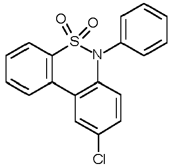 | 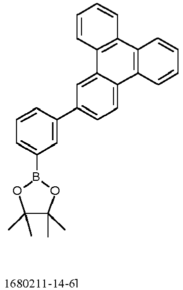 | 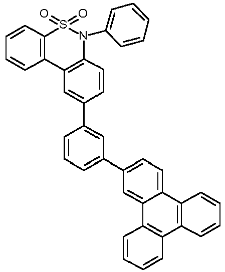 | 70% |
| 13f | 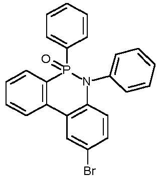 | 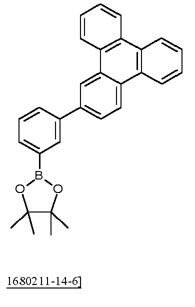 | 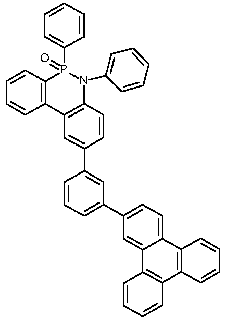 | 68% |
| 14f | 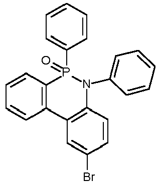 | 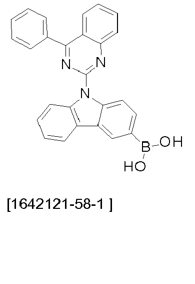 | 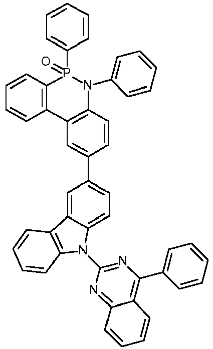 | 67% |
| 15f | 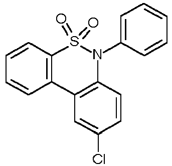 | 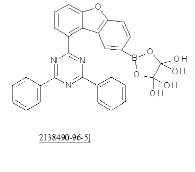 | 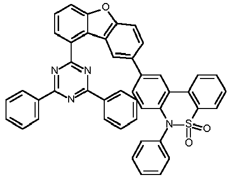 | 66% |
| 16f | 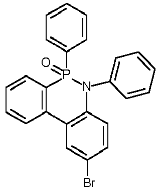 | 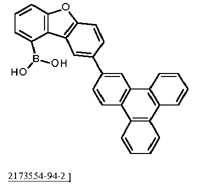 | 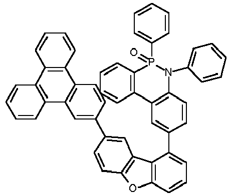 | 73% |
| 17f | 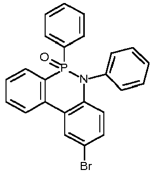 | 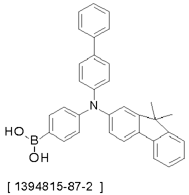 | 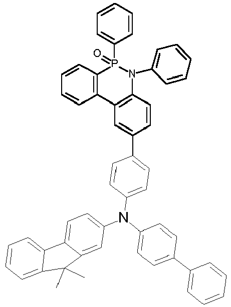 | 71% |
| 18f | 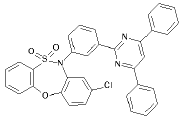 | 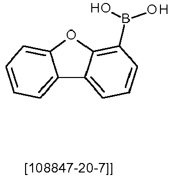 | 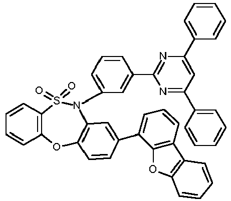 | 65% |
| 19f | 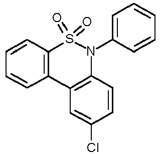 | 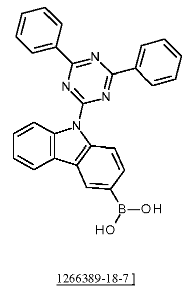 | 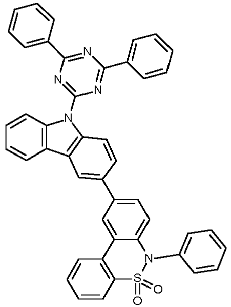 | 68% |
| 20f | 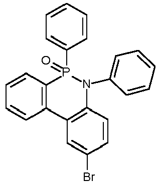 | 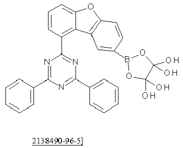 | 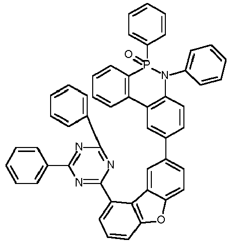 | 71% |

### Herstellung der OLEDs

In den folgenden Beispielen E1 bis E9 (siehe Tabelle 1) wird der Einsatz der erfindungsgemäßen Materialien in OLEDs vorgestellt.

**Vorbehandlung für die Beispiele E1-E9:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell den folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC2:TEG1 (55%:35%:10%) bedeutet hierbei, dass das Material IC1 in einem Volumenanteil von 55%, IC2 in einem Volumenanteil von 35% und TEG1 in einem Volumenanteil von 10% in der Schicht vorliegt. Analog kann auch die Elektronentransportschicht aus einer Mischung von zwei Materialien bestehen.

Die OLEDs werden standardmäßig charakterisiert. Die Elektrolumineszenzspektren werden bei einer Leuchtdichte von 1000 cd/m² bestimmt und daraus die CIE 1931 x und y Farbkoordinaten berechnet.

### Verwendung von erfindungsgemäßen Mischungen in OLEDs

Die erfindungsgemäßen Materialien können als Matrixmaterial in der Emissionsschicht von phosphoreszierenden grünen OLEDs eingesetzt werden. Die Ergebnisse sind in Tabelle 3 zusammengefasst.

**Tabelle 1: Aufbau der OLEDs**

| Bsp | HIL Dicke | HTL Dicke | EBL Dicke | EML Dicke | HBL Dicke | ETL Dicke | EIL Dicke |
|---|---|---|---|---|---|---|---|
| E1 | HATCN 5nm | SpMA1 230nm | SpMA2 20nm | 18c:IC2:TEG1 (64%:29%:7%) 40nm | ST2 5nm | ST2:LiQ (50%:50%) 30nm | LiQ 1nm |
| E2 | HATCN 5nm | SpMA1 230nm | SpMA2 20nm | IC1:f:TEG1 (47%:46%:7%) 40nm | ST2 5nm | ST2:LiQ (50%:50%) 30nm | LiQ 1nm |
| E3 | HATCN 5nm | SpMA1 230nm | SpMA2 20nm | CbzT4:8f:TEG1 (66%:22%:12%) 40nm | ST2 5nm | ST2:LiQ (50%:50%) 30nm | LiQ 1nm |
| E4 | HATCN 5nm | SpMA1 230nm | SpMA2 20nm | IC1:10f:TEG1 (66%:22%:12%) 40nm | ST2 5nm | ST2:LiQ (50%:50%) 30nm | LiQ 1nm |
| E5 | HATCN 5nm | SpMA1 230nm | SpMA2 20nm | CbzT4:13f:TEG1 (47%:46%:7%) 40nm | ST2 5nm | ST2:LiQ (50%:50%) 30nm | LiQ 1nm |
| E6 | HATCN 5nm | SpMA1 230nm | SpMA2 20nm | 15f:IC2:TEG1 (47%:46%:7%) 40nm | ST2 5nm | ST2:LiQ (50%:50%) 30nm | LiQ 1nm |
| E7 | HATCN 5nm | SpMA1 230nm | SpMA2 20nm | IC1:17f:TEG1 (44%:44%:12%) 40nm | ST2 5nm | ST2:LiQ (50%:50%) 30nm | LiQ 1nm |
| E8 | HATCN 5nm | SpMA1 230nm | SpMA2 20nm | CbzT4:18f:TEG1 (47%:46%:7%) 40nm | ST2 5nm | ST2:LiQ (50%:50%) 30nm | LiQ 1nm |
| E9 | HATCN 5nm | SpMA1 230nm | SpMA2 20nm | 19f:IC2:TEG1 (34%:59%:7%) 40nm | ST2 5nm | ST2:LiQ (50%:50%) 30nm | LiQ 1nm |

**Tabelle 2: Strukturformeln der Materialien für die OLEDs**

| | |
|---|---|
| 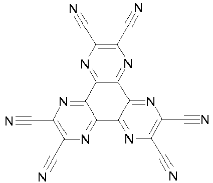 | 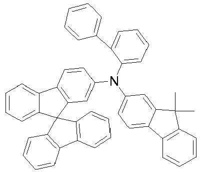 |
| HATCN | SpMA1 |
| 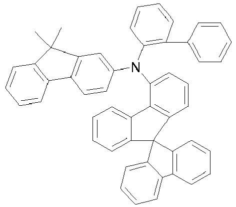 | 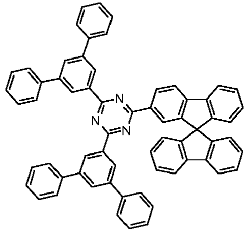 |
| SpMA3 | ST2 |
| 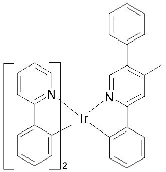 | 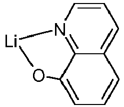 |
| TEG1 | LiQ |
| 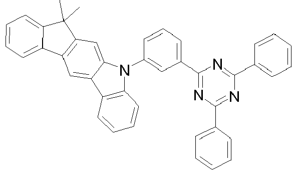 | 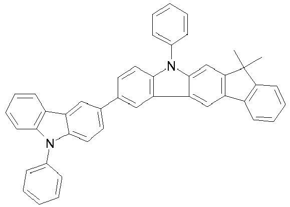 |
| IC1 | IC2 |
| 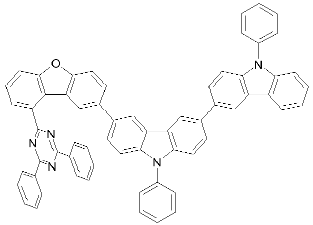 | 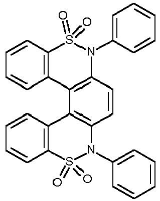 |
| CbzT4 | 18c |
| 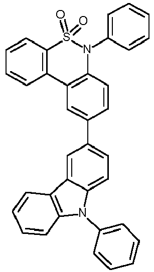 | 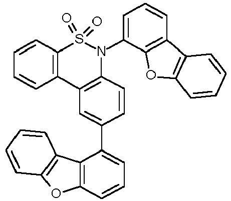 |
| f | 8f |
| 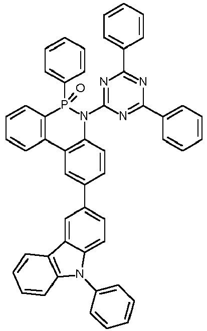 | 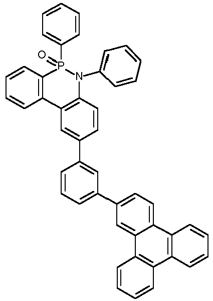 |
| 10f | 13f |
| 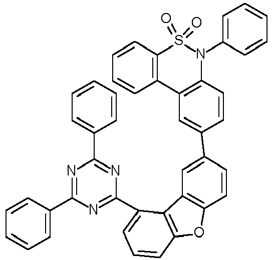 | 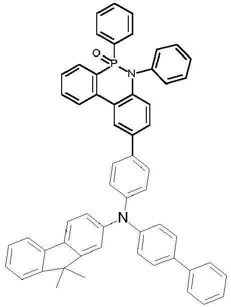 |
| 15f | 17f |
| 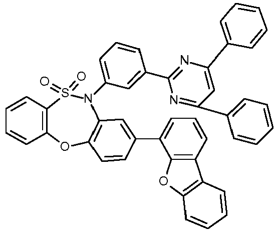 | 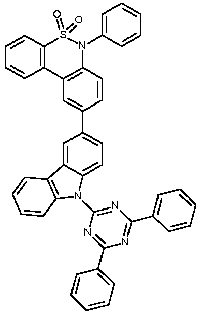 |
| 18f | 19f |

**Tabelle 3: Ergebnisse der Elektrolumineszenzvorrichtungen**

| Bsp. | U1000 (V) | SE1000 (cd/A) | EQE 1000 (%) | CIE x/y bei 1000 cd/m² |
|---|---|---|---|---|
| E1 | 3.3 | 68 | 19 | 0.36/0.61 |
| E2 | 3.5 | 73 | 18 | 0.35/0.62 |
| E3 | 3.4 | 72 | 17 | 0.34/0.62 |
| E4 | 3.5 | 69 | 18 | 0.35/0.61 |
| E5 | 3.2 | 73 | 17 | 0.35/0.62 |
| E6 | 3.4 | 73 | 17 | 0.35/0.62 |
| E7 | 3.7 | 65 | 16 | 0.32/0.63 |
| E8 | 3.5 | 63 | 16 | 0.33/0.63 |
| E9 | 3.1 | 60 | 19 | 0.33/0.63 |

## Patentansprüche

1. Verbindung gemäß Formel (1), 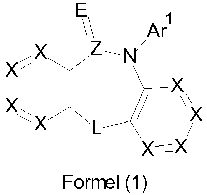 wobei für die verwendeten Symbole gilt:
Z ist bei jedem Auftreten gleich oder verschieden PAr² oder S(=O);
E ist bei jedem Auftreten gleich oder verschieden O oder S, wenn das Symbol Z, an das dieses E bindet, für PAr² steht, und O, wenn das Symbol Z, an das dieses E bindet, für S(=O) steht;
L ist ausgewählt aus der Gruppe bestehend aus einer Einfachbindung, NAr², O, S, S(=O)₂, P(=O)Ar², -X=X- oder -C(=O)-NAr²-;
X steht bei jedem Auftreten gleich oder verschieden für CR oder N,
wobei maximal zwei Gruppen X pro Cyclus für N stehen, oder zwei benachbarte X stehen für eine Gruppe der folgenden Formel (2), (3) oder (4), 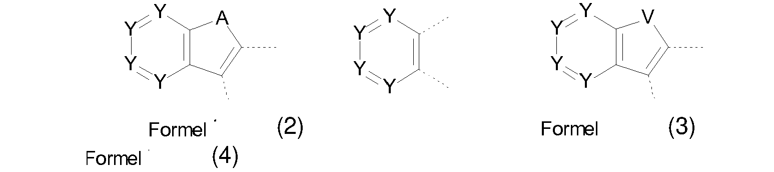
wobei die gestrichelten Bindungen die Verknüpfung dieser Gruppe in der Formel (1) kennzeichnen;
Y ist gleich oder verschieden bei jedem Auftreten CR oder N, wobei maximal zwei Gruppen Y pro Cyclus für N stehen;
Ar¹, Ar² ist bei jedem Auftreten gleich oder verschieden ein aromatisches oder heteroaromatisches Ringsystem mit 5 bis 40 aromatischen Ringatomen, welches mit einem oder mehreren Resten R substituiert sein kann, wobei Ar¹ und Ar² nicht miteinander verknüpft sind, mit der Maßgabe, dass die Substituenten R, die an Ar¹ binden, nicht für Fluor stehen;
A ist bei jedem Auftreten gleich oder verschieden NAr², O, S oder CR₂;
V ist bei jedem Auftreten gleich oder verschieden -NAr²-Z(=E)-;
R ist bei jedem Auftreten gleich oder verschieden H, D, F, Cl, Br, I, N(Ar³)₂, OAr³, SAr³, CN, NO₂, NAr³R¹, N(R¹)₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 40 aromatischen Ringatomen, das jeweils durch einen oder mehrere Reste R¹ substituiert sein kann; dabei können zwei Reste R auch miteinander ein aliphatisches oder heteroaliphatisches Ringsystem bilden;
Ar³ ist bei jedem Auftreten gleich oder verschieden ein aromatisches oder heteroaromatisches Ringsystem mit 5 bis 30 aromatischen Ringatomen, das durch einen oder mehrere Reste R¹ substituiert sein kann;
R¹ ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein aliphatisches Ringsystem bilden;
R² ist bei jedem Auftreten gleich oder verschieden H, D, F, CN oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
dabei sind die folgenden Verbindungen von der Erfindung ausgeschlossen:

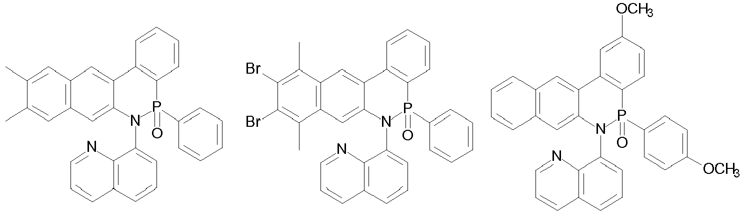

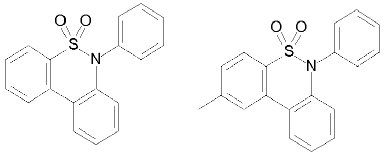

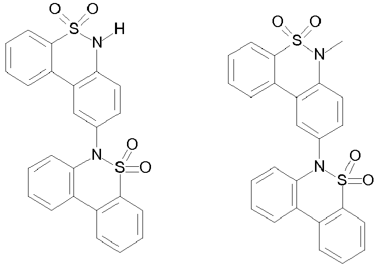

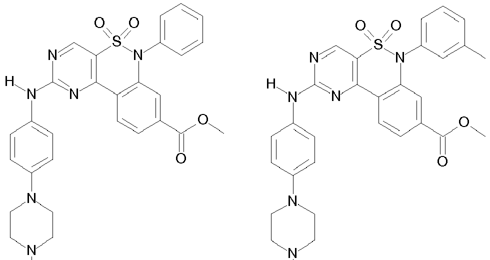

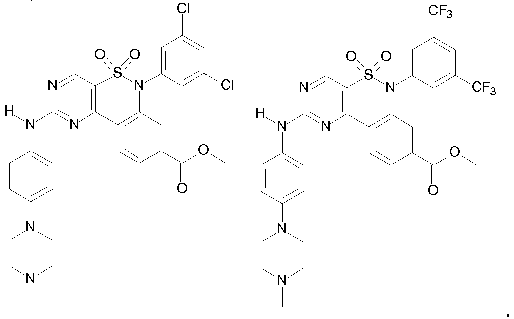

2. Verbindung nach Anspruch 1, ausgewählt aus den Verbindungen der Formeln (1-1) und (5) bis (16), 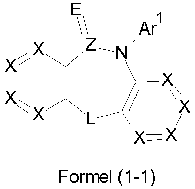 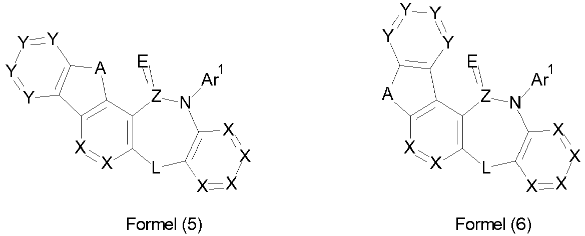 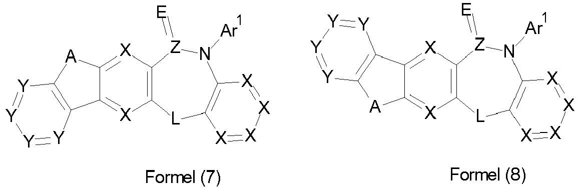 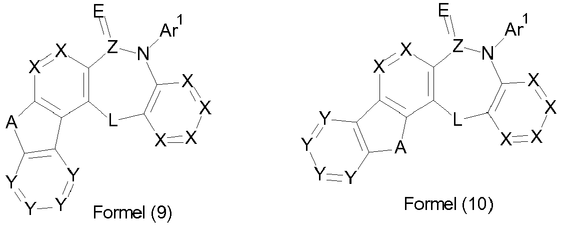 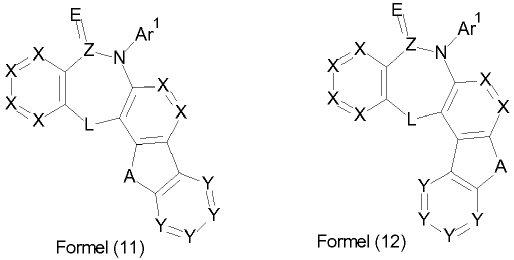 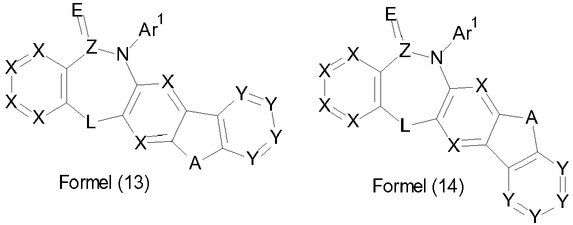 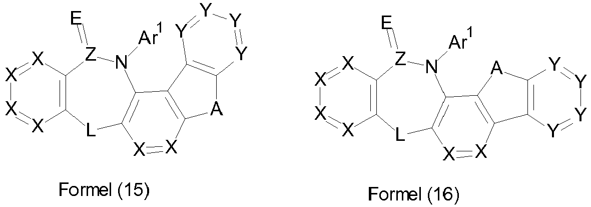 wobei X bei jedem Auftreten gleich oder verschieden für CR oder N steht, wobei maximal zwei Gruppen X pro Cyclus für N stehen, und die weiteren verwendeten Symbole die in Anspruch 1 genannten Bedeutungen aufweisen.

3. Verbindung nach Anspruch 1 oder 2, **dadurch gekennzeichnet, dass** die Gruppe der Formel (2), (3) bzw. (4) ausgewählt ist aus den Gruppen der Formel (2a), (3a) bzw. (4a), 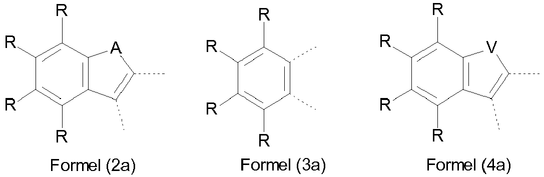 wobei die verwendeten Symbole die oben genannten Bedeutungen aufweisen.

4. Verbindung nach einem oder mehreren der Ansprüche 1 bis 3, **dadurch gekennzeichnet, dass** die Symbole X, die nicht für eine Gruppe der Formel (2), (3) oder (4) stehen, gleich oder verschieden bei jedem Auftreten für CR stehen und dass die Symbole Y gleich oder verschieden bei jedem Auftreten für CR stehen.

5. Verbindung nach einem oder mehreren der Ansprüche 1 bis 4, ausgewählt aus den Strukturen der Formeln (1a) und (5a) bis (16a), 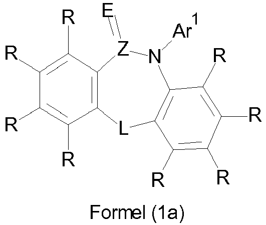 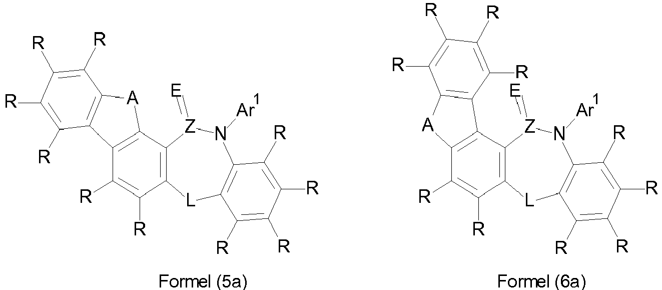 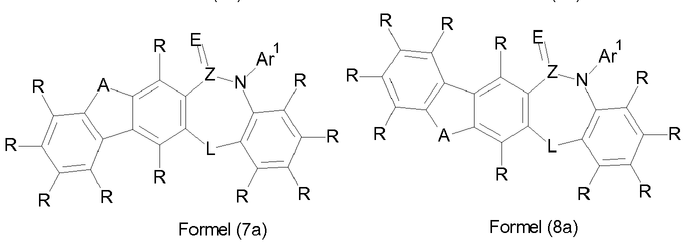 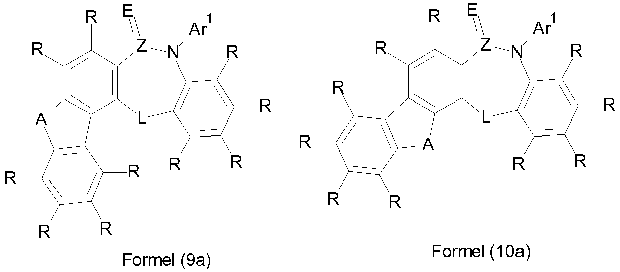 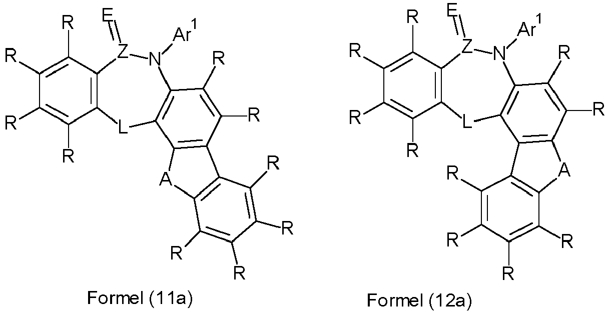 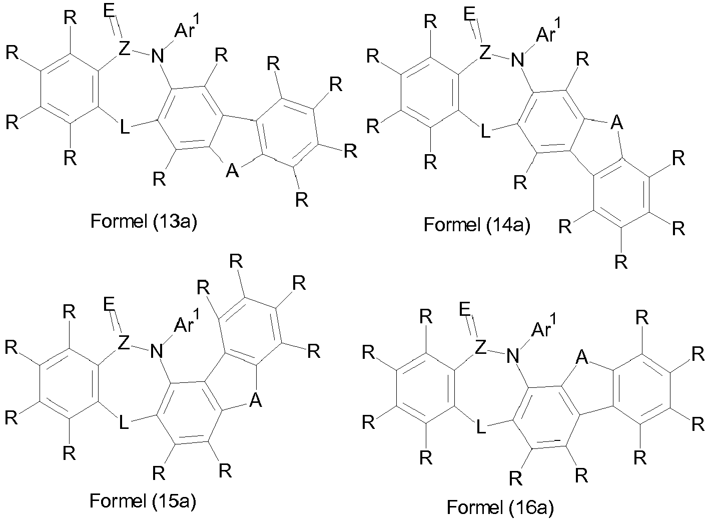 wobei die Symbole die in Anspruch 1 genannten Bedeutungen aufweisen.

6. Verbindung nach einem oder mehreren der Ansprüche 1 bis 5, ausgewählt aus den Strukturen der Formeln (17) und (18), 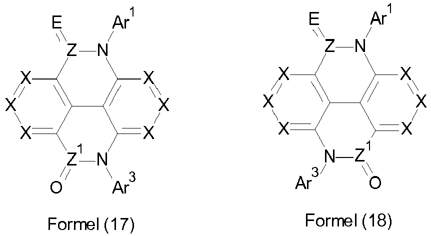 wobei die verwendeten Symbole die in Anspruch 1 genannten Bedeutungen aufweisen und Z¹ für PR¹ oder S(=O) steht.

7. Verbindung nach einem oder mehreren der Ansprüche 1 bis 6, ausgewählt aus den Strukturen der Formeln (1b) bzw. (5b) bis (16b), 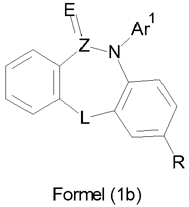 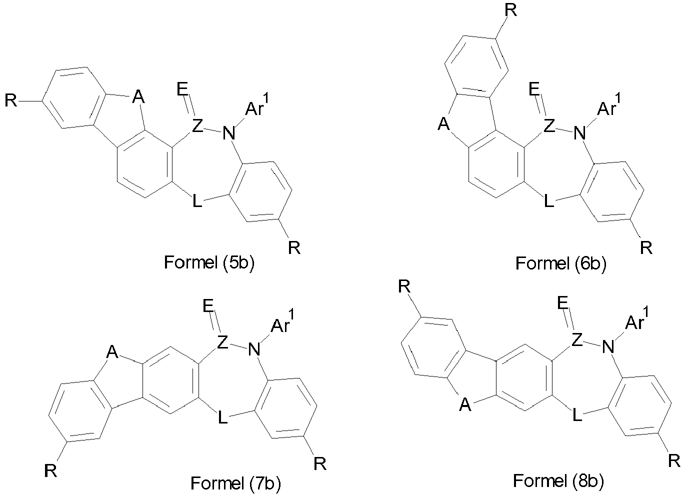 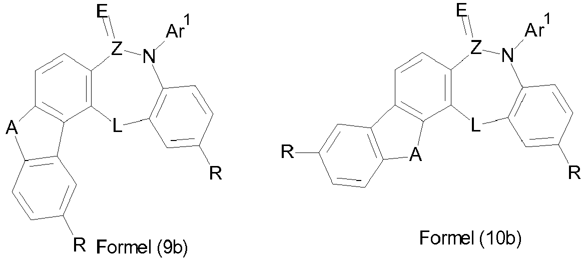 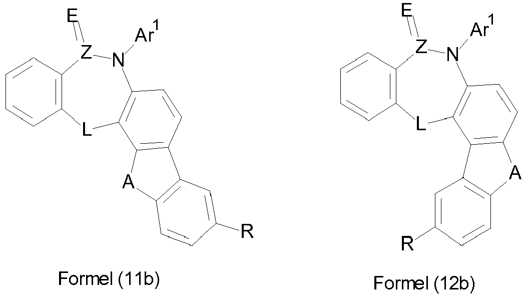 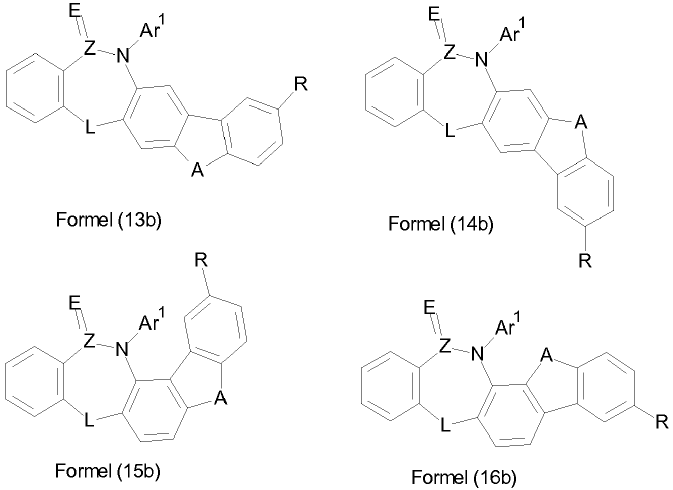 wobei die verwendeten Symbole die in Anspruch 1 genannten Bedeutungen aufweisen.

8. Verbindung nach einem oder mehreren der Ansprüche 1 bis 7, **dadurch gekennzeichnet, dass** Z für PAr² steht und E für O steht und L für eine Einfachbindung steht.

9. Verbindung nach einem oder mehreren der Ansprüche 1 bis 8, **dadurch gekennzeichnet, dass** Ar¹ und Ar² bei jedem Auftreten gleich oder verschieden ein aromatisches oder heteroaromatisches Ringsystem mit 6 bis 24 aromatischen Ringatomen darstellen, das durch einen oder mehrere Reste R substituiert sein kann, wobei für Ar¹ die Reste R nicht für Fluor stehen.

10. Verbindung nach einem oder mehreren der Ansprüche 1 bis 9, **dadurch gekennzeichnet, dass** R bei jedem Auftreten gleich oder verschieden ausgewählt ist aus der Gruppe bestehend aus H, D, F, N(Ar³)₂, CN, OR¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R¹ substituiert sein kann, wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R¹ substituiert sein kann; dabei können zwei Reste R auch miteinander ein aliphatisches Ringsystem bilden.

11. Formulierung, enthaltend mindestens eine Verbindung nach einem oder mehreren der Ansprüche 1 bis 10 und mindestens eine weitere Verbindung und/oder ein Lösemittel.

12. Verwendung einer Verbindung nach einem oder mehreren der Ansprüche 1 bis 10 in einer elektronischen Vorrichtung.

13. Elektronische Vorrichtung enthaltend mindestens eine Verbindung nach einem oder mehreren der Ansprüche 1 bis 10.

14. Elektronische Vorrichtung nach Anspruch 13, wobei es es um eine organische Elektrolumineszenzvorrichtung handelt, **dadurch gekennzeichnet, dass** die Verbindung nach einem oder mehreren der Ansprüche 1 bis 10 in einer emittierenden Schicht, insbesondere als Matrixmaterial, und/oder in einer Elektronentransportschicht und/oder in einer Lochblockierschicht und/oder in einer Lochtransportschicht und/oder in einer Exzitonenblockierschicht enthalten.

## Claims

1. Compound of formula (1) 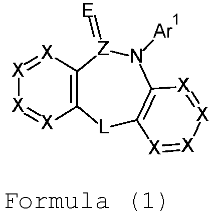 where the symbols used are as follows:
Z is the same or different at each instance and is PAr² or S(=O);
E is the same or different at each instance and is O or S when the symbol Z to which this E binds is PAr², and
O when the symbol Z to which this E binds is S(=O);
L is selected from the group consisting of a single bond, NAr², O, S, S(=O)₂, P(=O)Ar², -X=X- and -C (=O) -NAr²-;
X is the same or different at each instance and is CR or N,
where not more than two X groups per cycle are N, or two adjacent X are a group of the following formula (2), (3) or (4) : 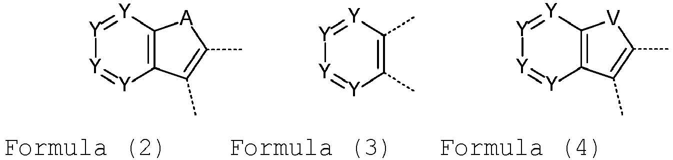
where the dotted bonds indicate the linkage of this group in the formula (1);
Y is the same or different at each instance and is CR or N, where not more than two Y groups per cycle are N;
Ar¹, Ar² is the same or different at each instance and is an aromatic or heteroaromatic ring system which has 5 to 40 aromatic ring atoms and may be substituted by one or more R radicals, where Ar¹ and Ar² are not joined to one another, with the proviso that the substituents R that bind to Ar¹ are not fluorine;
A is the same or different at each instance and is NAr², O, S or CR₂;
V is the same or different at each instance and is -NAr²-Z (=E) -;
R is the same or different at each instance and is H, D, F, Cl, Br, I, N(Ar³)₂, OAr³, SAr³, CN, NO₂, NAr³R¹, N(R¹)₂, OR¹, SR¹, COOR¹, C(=O)N(R¹)₂, Si(R¹)₃, B(OR¹)₂, C(=O)R¹, P(=O) (R¹)₂, S(=O)R¹, S(=O)₂R¹, OSO₂R¹, a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more R¹ radicals, where one or more nonadjacent CH₂ groups may be replaced by Si(R¹)₂, C=O, NR¹, O, S or CONR¹, or an aromatic or heteroaromatic ring system which has 5 to 40 aromatic ring atoms and may be substituted in each case by one or more R¹ radicals; at the same time, two R radicals together may also form an aliphatic or heteroaliphatic ring system;
Ar³ is the same or different at each instance and is an aromatic or heteroaromatic ring system which has 5 to 30 aromatic ring atoms and may be substituted by one or more R¹ radicals;
R¹ is the same or different at each instance and is H, D, F, Cl, Br, I, N(R²)₂, CN, NO₂, OR², SR², Si(R²)₃, B(OR²)₂, C(=O)R², P(=O) (R²)₂, S(=O)R², OSO₂R², a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more R² radicals, where one or more nonadjacent CH₂ groups may be replaced by Si(R²)₂, C=O, NR², O, S or CONR², or an aromatic or heteroaromatic ring system which has 5 to 40 aromatic ring atoms and may be substituted in each case by one or more R² radicals; at the same time, two or more R¹ radicals together may form an aliphatic ring system;
R² is the same or different at each instance and is H, D, F, CN or an aliphatic, aromatic or heteroaromatic organic radical, especially a hydrocarbyl radical, having 1 to 20 carbon atoms, in which one or more hydrogen atoms may also be replaced by F;
where the following compounds are excluded from the invention:

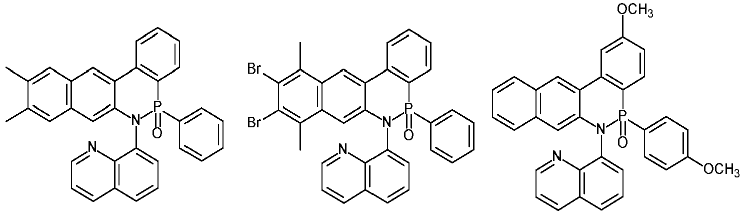

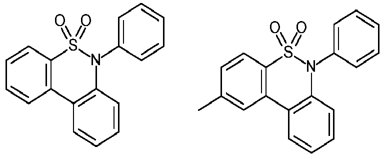

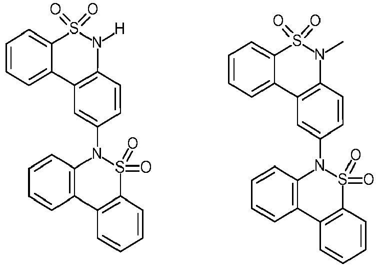

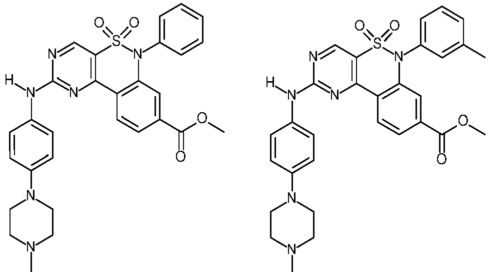

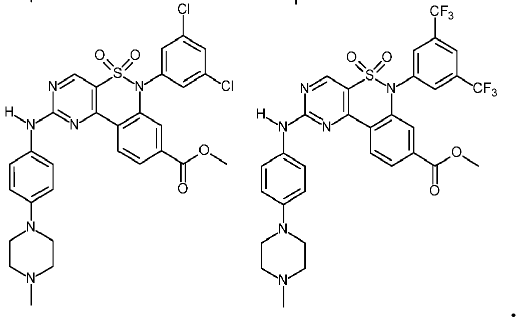

2. Compound according to Claim 1, selected from the compounds of the formulae (1-1) and (5) to (16): 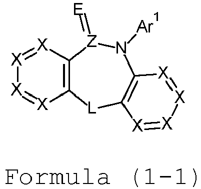 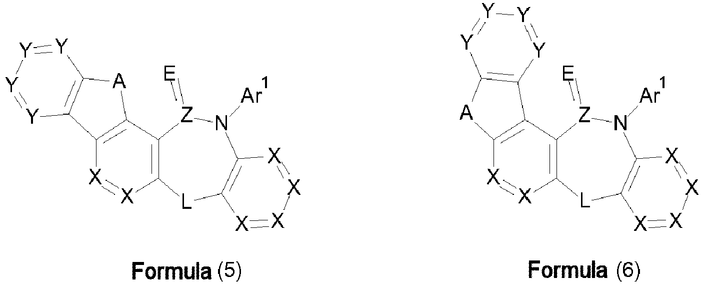 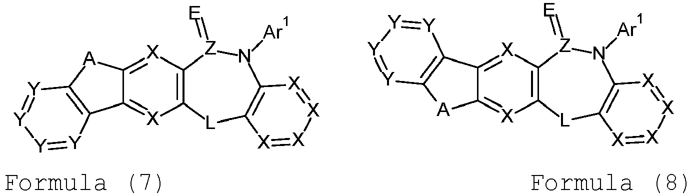 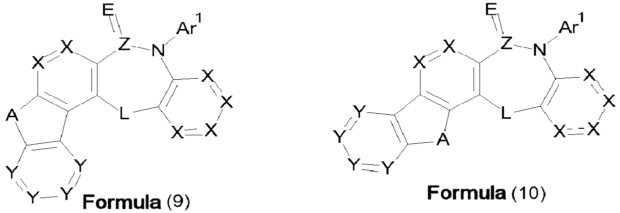 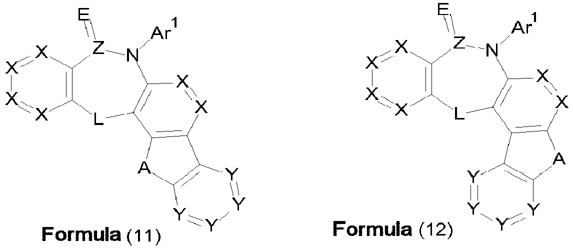 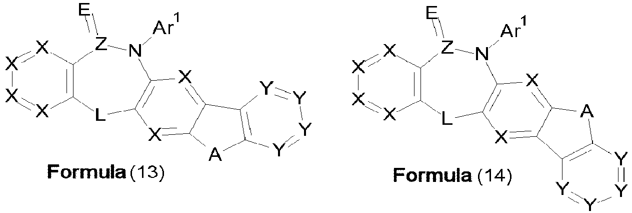 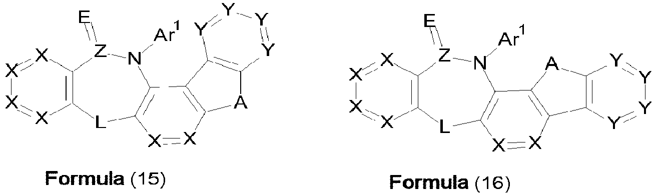 where X is the same or different at each instance and is CR or N, where not more than two X groups per cycle are N, and the further symbols used have the definitions given in Claim 1.

3. Compound according to Claim 1 or 2, **characterized in that** the group of the formula (2), (3) or (4) is respectively selected from the groups of the formula (2a), (3a) or (4a): 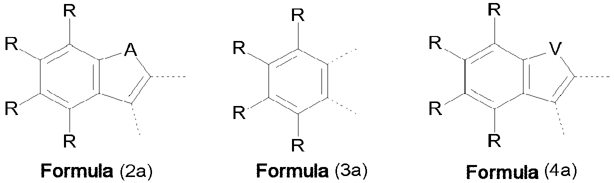 where the symbols used have the definitions given above.

4. Compound according to one or more of Claims 1 to 3, **characterized in that** the symbols X that are not a group of the formula (2), (3) or (4) are the same or different at each instance and are CR, and **in that** the symbols Y are the same or different at each instance and are CR.

5. Compound according to one or more of Claims 1 to 4, selected from the structures of the formulae (1a) and (5a) to (16a): 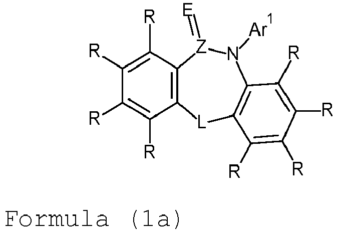 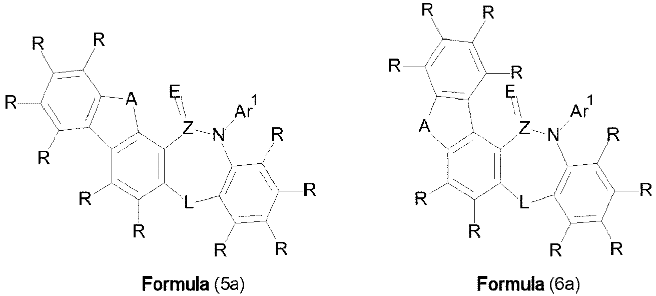 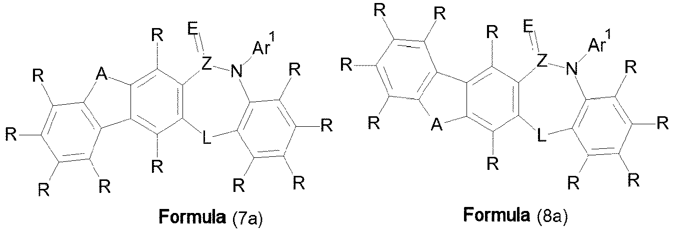 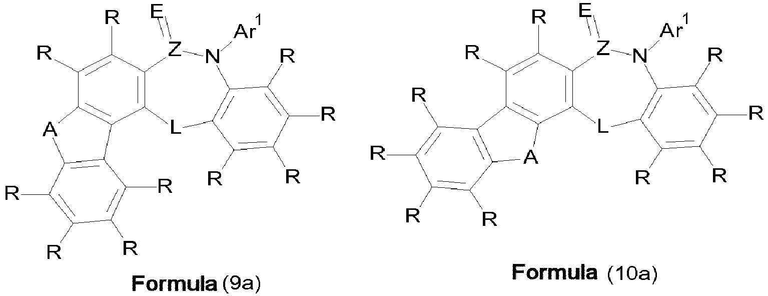 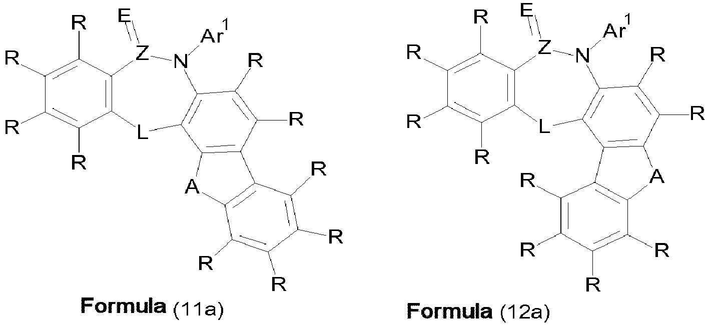 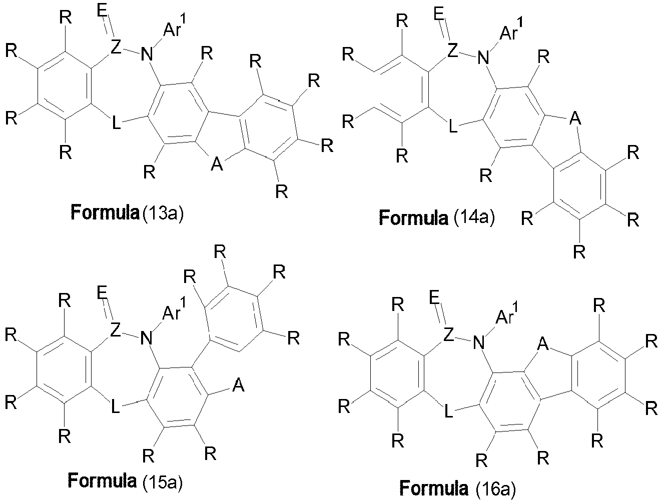 where the symbols have the definitions given in Claim 1.

6. Compound according to one or more of Claims 1 to 5, selected from the structures of the formulae (17) and (18) : 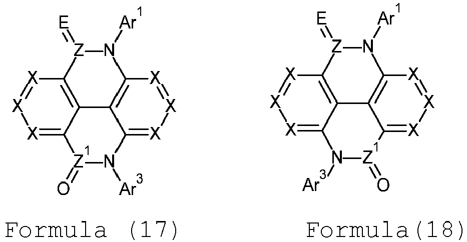 where the symbols used have the definitions given in Claim 1 and Z¹ is PR¹ or S(=O).

7. Compound according to one or more of Claims 1 to 6, selected from the structures of the formulae (1b) or (5b) to (16b): 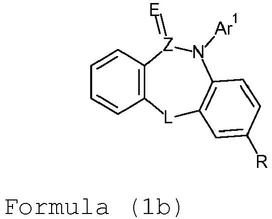 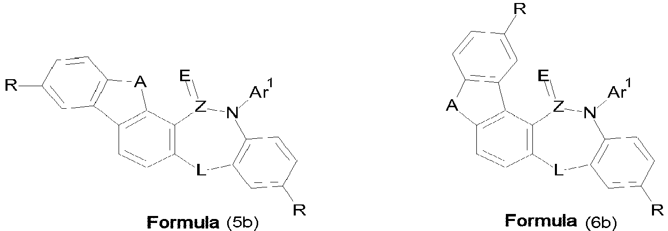 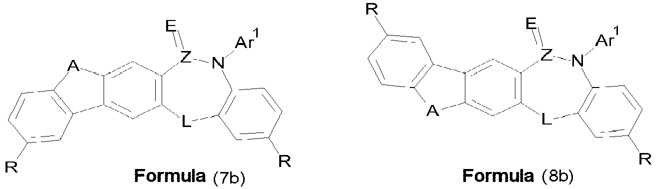 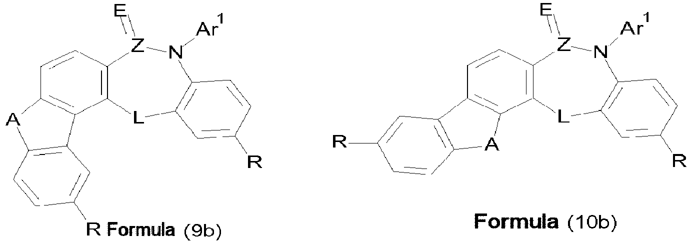 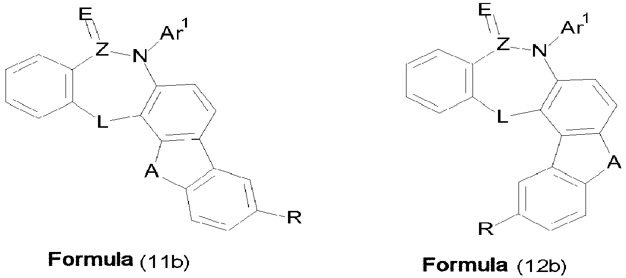 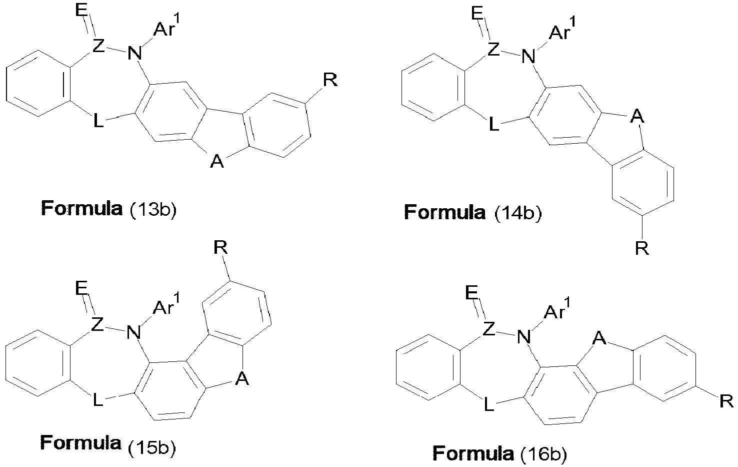 where the symbols used have the definitions given in Claim 1.

8. Compound according to one or more of Claims 1 to 7, **characterized in that** Z is PAr² and E is O and L is a single bond.

9. Compound according to one or more of Claims 1 to 8, **characterized in that** Ar¹ and Ar² are the same or different at each instance and are an aromatic or heteroaromatic ring system which has 6 to 24 aromatic ring atoms and may be substituted by one or more R radicals, where the R radicals in the case of Ar¹ are not fluorine.

10. Compound according to one or more of Claims 1 to 9, **characterized in that** R is the same or different at each instance and is selected from the group consisting of H, D, F, N(Ar³)₂, CN, OR¹, a straight-chain alkyl group having 1 to 10 carbon atoms or an alkenyl group having 2 to 10 carbon atoms or a branched or cyclic alkyl group having 3 to 10 carbon atoms, where the alkyl or alkenyl group may in each case be substituted by one or more R¹ radicals, where one or more nonadjacent CH₂ groups may be replaced by O, or an aromatic or heteroaromatic ring system which has 6 to 30 aromatic ring atoms and may be substituted in each case by one or more R¹ radicals; at the same time, two R radicals together may also form an aliphatic ring system.

11. Formulation comprising at least one compound according to one or more of Claims 1 to 10 and at least one further compound and/or a solvent.

12. Use of a compound according to one or more of Claims 1 to 10 in an electronic device.

13. Electronic device comprising at least one compound according to one or more of Claims 1 to 10.

14. Electronic device according to Claim 13 which is an organic electroluminescent device, **characterized in that** the compound according to one or more of Claims 1 to 10 is present in an emitting layer, especially as matrix material, and/or in an electron transport layer and/or in a hole blocker layer and/or in a hole transport layer and/or in an exciton blocker layer.

## Revendications

1. Composé selon la formule (1) 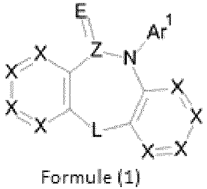 où, pour les symboles utilisés, ce qui suit est d'application :
Z représente, en chaque occurrence, de manière identique ou différente, PAr² ou S(=O) ;
E représente, en chaque occurrence, de manière identique ou différente, O ou S, lorsque le symbole Z, auquel ce radical E est lié, représente PAr², et O, lorsque le symbole Z, auquel ce radical E est lié, représente S(=O) ;
L est choisi dans le groupe constitué par une simple liaison, , NAr², O, S, S (=O)₂, P(=O)Ar², -X=X-ou -C(=O)-NAr²- ;
X représente, en chaque occurrence, de manière identique ou différente, CR ou N,
au maximum deux groupes X par cycle représentant N ou deux X adjacents représentant un groupe des formules suivantes (2), (3) ou (4), 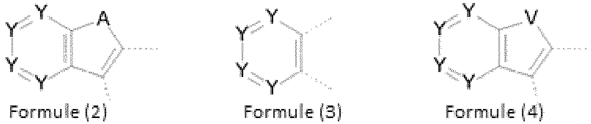
les liaisons en pointillés caractérisant la liaison de ce groupe dans la formule (1) ;
Y représente, de manière identique ou différente, en chaque occurrence, CR ou N, au maximum deux groupes Y par cycle représentant N ;
Ar¹, Ar² représentent, en chaque occurrence, de manière identique ou différente, un système cyclique aromatique ou hétéroaromatique comprenant 5 à 40 atomes de cycle aromatique, qui peut être substitué par un ou plusieurs radicaux R, Ar¹ et Ar² n'étant pas liés à l'un à l'autre, à condition que les substituants R liés à Ar¹ ne représentent pas fluor ;
A représente, en chaque occurrence, de manière identique ou différente, NAr², O, S ou CR₂ ;
V représente, en chaque occurrence, de manière identique ou différente, -NAr²-Z(=E)- ;
R représente, en chaque occurrence, de manière identique ou différente, H, D, F, Cl, Br, I, N(Ar³)₂, OAr³, SAr³, CN, NO₂, NAr³R¹, N(R¹)₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, un groupe alkyle linéaire comprenant 1 à 20 atomes de carbone ou un groupe alcényle ou alcynyle comprenant 2 à 20 atomes de carbone ou un groupe alkyle ramifié ou cyclique comprenant 3 à 20 atomes de carbone, le groupe alkyle, alcényle ou alcynyle pouvant à chaque fois être substitué par un ou plusieurs radicaux R¹ et un ou plusieurs groupes CH₂ non adjacents pouvant être remplacés par Si(R¹)₂, C=O, NR¹, O, S ou CONR¹, ou un système cyclique aromatique ou hétéroaromatique comprenant 5 à 40 atomes de cycle aromatique, qui peut à chaque fois être substitué par un ou plusieurs radicaux R¹ ; deux radicaux R pouvant également former ensemble un système cyclique aliphatique ou hétéroaliphatique ;
Ar³ représente, en chaque occurrence, de manière identique ou différente, un système cyclique aromatique ou hétéroaromatique comprenant 5 à 30 atomes de cycle aromatique, qui peut être substitué par un ou plusieurs radicaux R¹ ;
R¹ représente, en chaque occurrence, de manière identique ou différente, H, D, F, Cl, Br, I, N(R²)₂, CN, NO₂, OR², SR², Si(R²)₃, B(OR²)₂, C(=O)R², P(=O) (R²)₂, S(=O)R², OSO₂R², un groupe alkyle linéaire comprenant 1 à 20 atomes de carbone ou un groupe alcényle ou alcynyle comprenant 2 à 20 atomes de carbone ou un groupe alkyle ramifié ou cyclique comprenant 3 à 20 atomes de carbone, le groupe alkyle, alcényle ou alcynyle pouvant à chaque fois être substitué par un ou plusieurs radicaux R², un ou plusieurs groupes CH₂ non adjacents pouvant être remplacés par Si(R²)₂, C=O, NR², O, S ou CONR², ou un système cyclique aromatique ou hétéroaromatique comprenant 5 à 40 atomes de cycle aromatique, qui peut à chaque fois être substitué par un ou plusieurs radicaux R² ; deux radicaux R¹ ou plus pouvant former ensemble un système cyclique aliphatique ;
R² représente, en chaque occurrence, de manière identique ou différente, H, D, F, CN ou un radical organique, en particulier un radical hydrocarboné, aliphatique, aromatique ou hétéroaromatique, comprenant 1 à 20 atomes de carbone, dans lequel un ou plusieurs atomes H peuvent également être remplacés par F ;
les composés suivants étant exclus de l'invention :

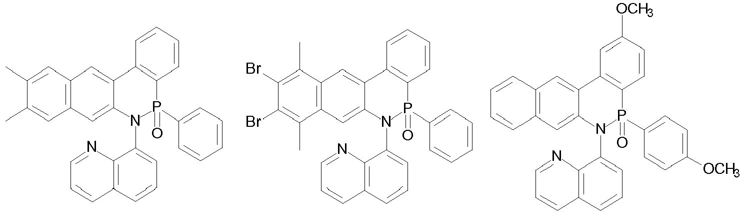

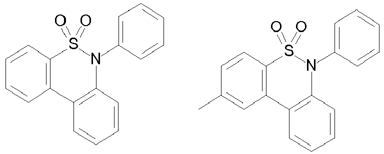

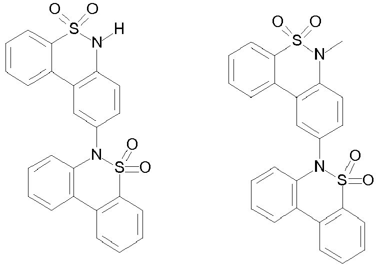

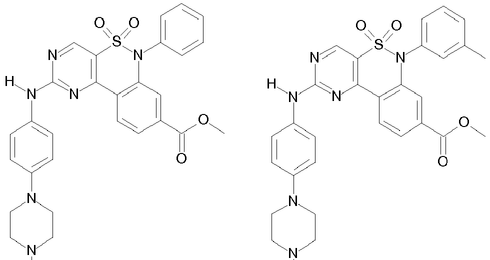

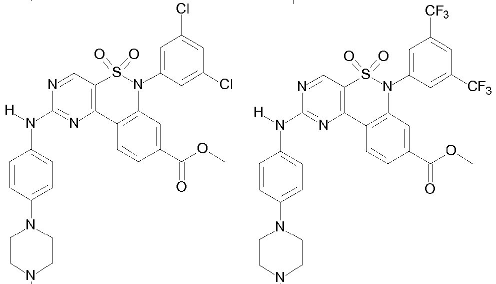

2. Composé selon la revendication 1, choisi parmi les composés des formules (1-1) et (5) à (16), 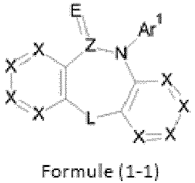 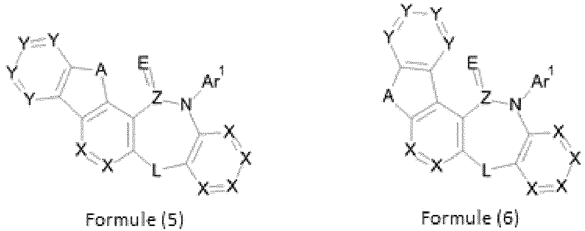 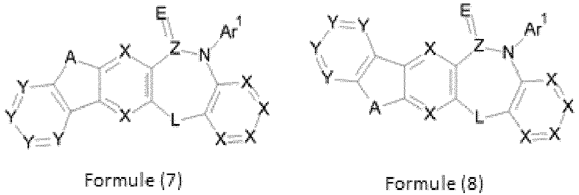 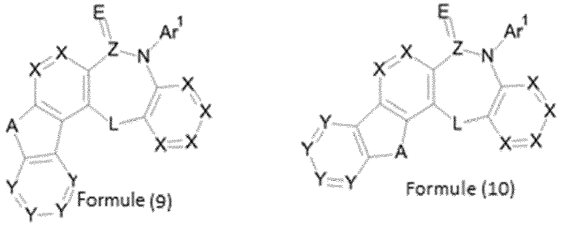 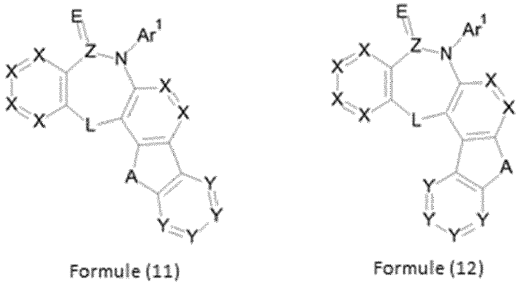 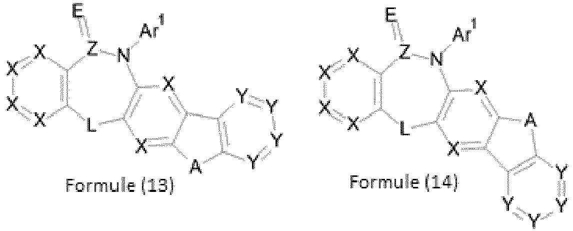 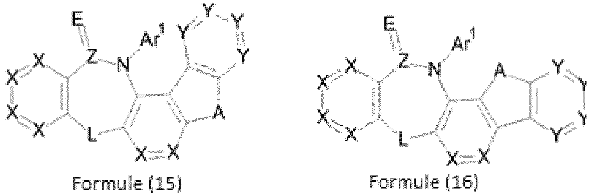 dans lesquelles X représente, en chaque occurrence, de manière identique ou différente, CR ou N, au maximum deux groupes X par cycle représentant N et les autres symboles utilisés présentant les significations mentionnées dans la revendication 1.

3. Composé selon la revendication 1 ou 2, **caractérisé en ce que** le groupe de formule (2), (3) ou (4) est choisi parmi les groupes de formule (2a), (3a) ou (4a), 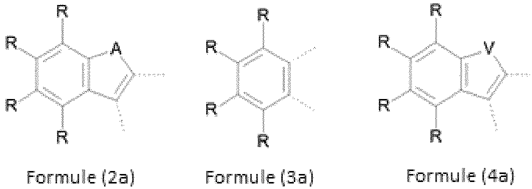 les symboles utilisés présentant les significations susmentionnées.

4. Composé selon l'une ou plusieurs des revendications 1 à 3, **caractérisé en ce que** les symboles X, qui ne représentent pas un groupe de formule (2), (3) ou (4) représentent, de manière identique ou différente, en chaque occurrence, CR et **en ce que** les symboles Y représentent, de manière identique ou différente, en chaque occurrence, CR.

5. Composé selon l'une ou plusieurs des revendications 1 à 4, choisi parmi les structures des formules (la) et (5a) à (16a), 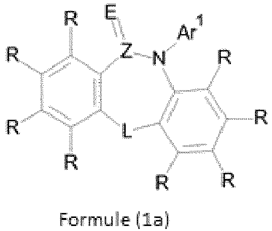 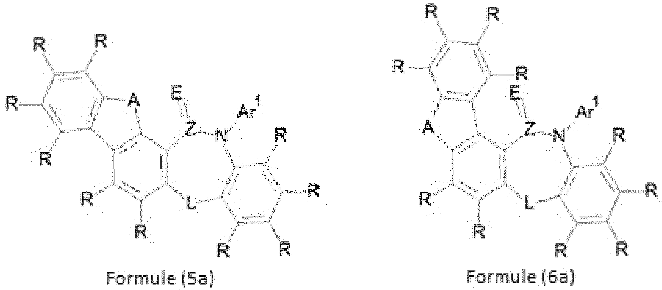 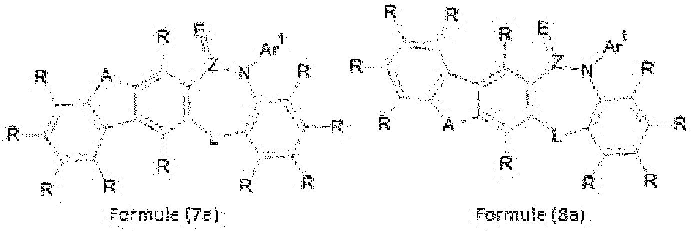 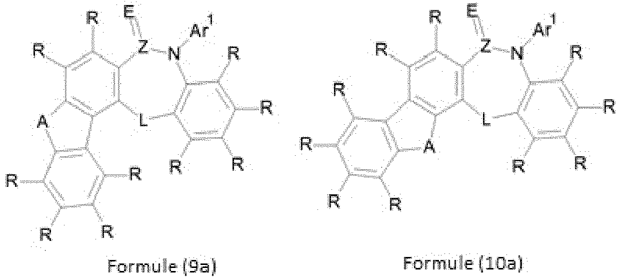 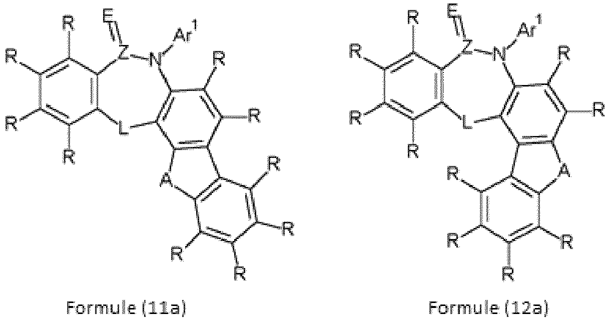 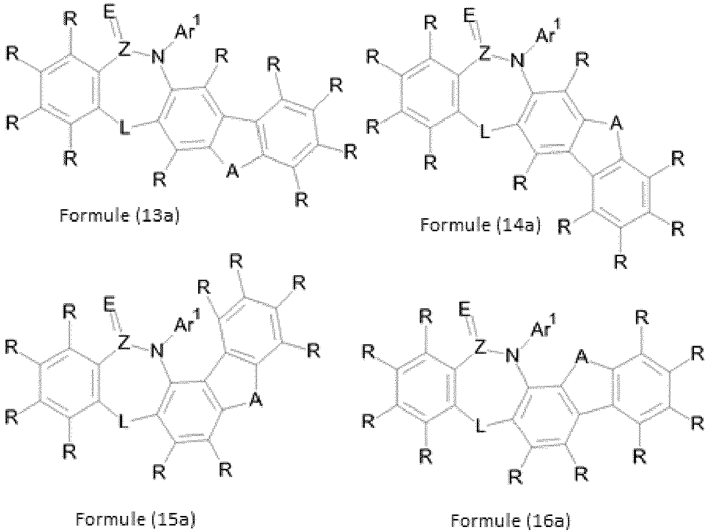 les symboles présentant les significations mentionnées dans la revendication 1.

6. Composé selon l'une ou plusieurs des revendications 1 à 5, choisi parmi les structures des formules (17) et (18), 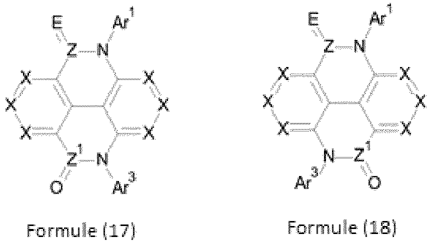 les symboles utilisés présentant les significations mentionnées dans la revendication 1 et Z¹ représentant PR¹ ou S(=O).

7. Composé selon l'une ou plusieurs des revendications 1 à 6, choisi parmi les structures des formules (1b) ou (5b) à (16b), 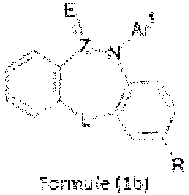 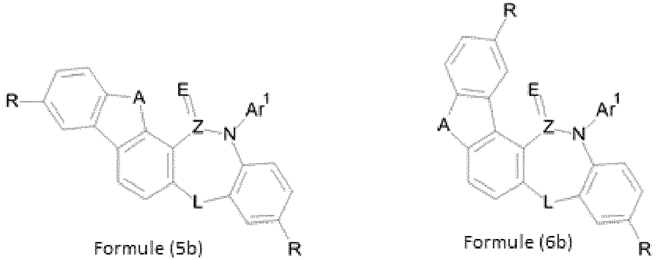 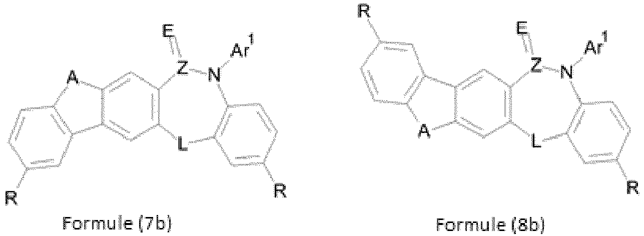 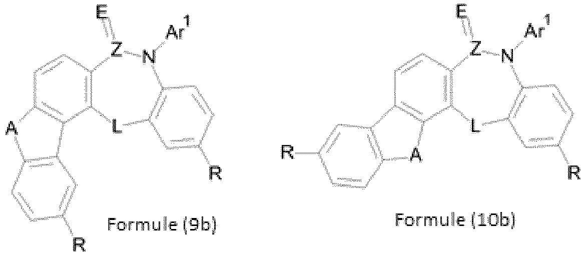 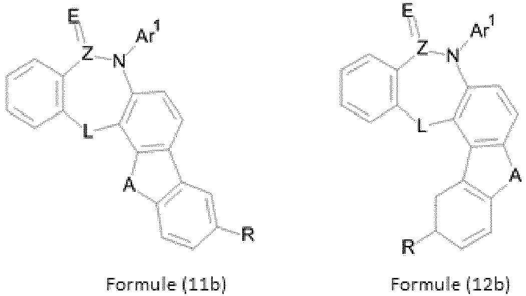 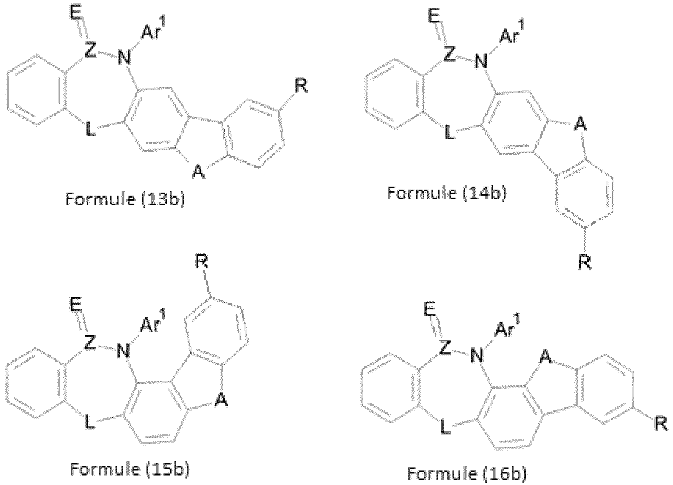 les symboles utilisés présentant les significations mentionnées dans la revendication 1.

8. Composé selon l'une ou plusieurs des revendications 1 à 7, **caractérisé en ce que** Z représente PAr² et E représente O et L représente une simple liaison.

9. Composé selon l'une ou plusieurs des revendications 1 à 8, **caractérisé en ce que** Ar¹ et Ar² représentent, en chaque occurrence, de manière identique ou différente, un système cyclique aromatique ou hétéroaromatique comprenant 6 à 24 atomes de cycle aromatique, qui peut être substitué par un ou plusieurs radicaux R, où, pour Ar¹, les radicaux R ne représentent pas fluor.

10. Composé selon l'une ou plusieurs des revendications 1 à 9, **caractérisé en ce que** R est choisi, en chaque occurrence, de manière identique ou différente, dans le groupe constitué par H, D, F, N(Ar³)₂, CN, OR¹, un groupe alkyle linéaire comprenant 1 à 10 atomes de carbone ou un groupe alcényle comprenant 2 à 10 atomes de carbone ou un groupe alkyle ramifié ou cyclique comprenant 3 à 10 atomes de carbone, le groupe alkyle ou alcényle pouvant à chaque fois être substitué par un ou plusieurs radicaux R¹, un ou plusieurs groupes CH₂ non adjacents pouvant être remplacés par O, ou un système cyclique aromatique ou hétéroaromatique comprenant 6 à 30 atomes de cycle aromatique, qui peut à chaque fois être substitué par un ou plusieurs radicaux R¹ ; deux radicaux R pouvant également former ensemble un système cyclique.

11. Formulation, contenant au moins un composé selon l'une ou plusieurs des revendications 1 à 10 et au moins un autre composé et/ou un solvant.

12. Utilisation d'un composé selon l'une ou plusieurs des revendications 1 à 10 dans un dispositif électronique.

13. Dispositif électronique contenant au moins un composé selon l'une ou plusieurs des revendications 1 à 10.

14. Dispositif électronique selon la revendication 13, le dispositif étant un dispositif organique électroluminescent, **caractérisé en ce que** le composé selon l'une ou plusieurs des revendications 1 à 10 est contenu dans une couche émettrice, en particulier comme matériau de matrice, et/ou dans une couche de transport d'électrons et/ou dans une couche de blocage de trous et/ou dans une couche de transport de trous et/ou dans une couche de blocage d'excitons.
